(19) Europäisches Patentamt
European Patent Office
Office européen des brevets

(11) EP 4 450 589 B1

(12) EUROPEAN PATENT SPECIFICATION

(45) Date of publication and mention of the grant of the patent:
**19.11.2025 Bulletin 2025/47**

(21) Application number: **24170807.2**

(22) Date of filing: **17.04.2024**

(51) International Patent Classification (IPC):
***C09K 11/06*** *(2006.01)* ***H10K 85/60*** *(2023.01)*
*H10K 50/11* *(2023.01)*

(52) Cooperative Patent Classification (CPC):
**C09K 11/06; H10K 85/654; H10K 85/6572;**
H10K 50/11

# (54) BLUE LUMINESCENT COMPOUND AND ORGANIC LIGHT-EMITTING DEVICE

BLAU LUMINESZIERENDE VERBINDUNG UND ORGANISCHE LICHTEMITTIERENDE VORRICHTUNG

COMPOSÉ LUMINESCENT BLEU ET DISPOSITIF ÉLECTROLUMINESCENT ORGANIQUE

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **18.04.2023 JP 2023067913**
**05.10.2023 KR 20230132512**

(43) Date of publication of application:
**23.10.2024 Bulletin 2024/43**

(73) Proprietor: **Samsung Display Co., Ltd.**
**Yongin-si, Gyeonggi-do 17113 (KR)**

(72) Inventors:
- **HIROSE, Tomoya**
  **Yokohama-city, 230-0027 (JP)**
- **MIYAZAKI, Eigo**
  **Yokohama-city, 230-0027 (JP)**
- **IMAMURA, Atsushi**
  **Yokohama-city, 230-0027 (JP)**
- **ITO, Mitsunori**
  **Yokohama-city, 230-0027 (JP)**

(74) Representative: **Elkington and Fife LLP**
**Prospect House**
**8 Pembroke Road**
**Sevenoaks, Kent TN13 1XR (GB)**

(56) References cited:
**WO-A1-2023/008865 CN-A- 114 685 506**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 450 589 B1

## Description

FIELD OF THE INVENTION

The disclosure relates to a blue luminescent compound and an organic light-emitting device.

BACKGROUND OF THE INVENTION

Luminescent materials of organic light-emitting devices may be classified into fluorescent materials, phosphorescent materials, and thermally activated delayed fluorescence (TADF) materials, according to a radiation deactivation process thereof. Generally, fluorescent devices have luminescence efficiency of about 5 % based on a luminescence mechanism that uses fluorescence from a singlet state. Meanwhile, devices using phosphorescent materials or TADF materials may use triplet energy for luminescence, and thus may have increased luminescence efficiency. Accordingly, some red and green phosphorescent materials have been put to practical use. However, fluorescent materials have been used for blue luminescent materials in view of device lifespan, and increasing device efficiency has become an issue.
However, there remains a need for a compound to provide improved color purity and luminescence efficiency.
WO 2023/008865 discloses light-emitting organic molecules and their use in organic light-emitting diodes (OLEDs) and in other optoelectronic devices.

SUMMARY OF THE INVENTION

According to the invention, a blue luminescent compound is provided in accordance with claim 1In an embodiment, an organic light-emitting device includes an emission layer including the blue luminescent compound.
Additional aspects will be set forth in part in the description which follows and, in part, will be apparent from the description, or may be learned by practice of the presented embodiments of the disclosure.

BRIEF DESCRIPTION OF THE DRAWINGS

The above and other aspects, features, and advantages of certain embodiments of the disclosure will be more apparent from the following description taken in conjunction with the accompanying drawings, in which:

FIGS. 1A to 1C are schematic diagrams illustrating a process of calculating $\beta_1$;
FIG. 2 is a schematic cross-sectional view illustrating an organic light-emitting device according to an embodiment;
FIG. 3 is a schematic cross-sectional view illustrating an organic light-emitting device according to another embodiment;
FIG. 4 is a schematic cross-sectional view illustrating an organic light-emitting device according to another embodiment;
FIG. 5 is an energy level diagram illustrating the energy relationship of the ground state ($S_0$) and the excited state ($S_1$) of a molecule;
FIG. 6 is a graph illustrating full width at half maximum [FWHM, (nm)] of emission in photoluminescence (PL) versus reorganization energy [electronvolts (eV)) in accordance with an example;
FIG. 7 is a graph illustrating photoluminescence (PL) intensity (arbitrary units) versus wavelength (nm) in accordance with an example; and
FIG. 8 is a graph illustrating photoluminescence (PL) intensity (arbitrary units) versus wavelength (nm) in accordance with an example.

DETAILED DESCRIPTION OF THE EMBODIMENTS

Reference will now be made in detail to embodiments, examples of which are illustrated in the accompanying drawings, wherein like reference numerals refer to like elements throughout. In this regard, the present embodiments may have different forms and should not be construed as being limited to the descriptions set forth herein. Accordingly, the embodiments are merely described below, by referring to the figures, to explain aspects. As used herein, the term "and/or" includes any and all combinations of one or more of the associated listed items. Expressions such as "at least one of," when preceding a list of elements, modify the entire list of elements and do not modify the individual elements of the list.
It will be understood that, although the terms "first," "second," "third" etc. may be used herein to describe various elements, components, regions, layers and/or sections, these elements, components, regions, layers and/or sections should not be limited by these terms. These terms are only used to distinguish one element, component, region, layer or section from another element, component, region, layer or section. Thus, "a first element," "component," "region," "layer"

or "section" discussed below could be termed a second element, component, region, layer or section without departing from the teachings herein.

The terminology used herein is for the purpose of describing particular embodiments only and is not intended to be limiting. As used herein, "a", "an," "the," and "at least one" do not denote a limitation of quantity, and are intended to include both the singular and plural, unless the context clearly indicates otherwise. For example, "an element" has the same meaning as "at least one element," unless the context clearly indicates otherwise. "At least one" is not to be construed as limiting "a" or "an." "Or" means "and/or." As used herein, the term "and/or" includes any and all combinations of one or more of the associated listed items. It will be further understood that the terms "comprises" and/or "comprising," or "includes" and/or "including" when used in this specification, specify the presence of stated features, regions, integers, steps, operations, elements, and/or components, but do not preclude the presence or addition of one or more other features, regions, integers, steps, operations, elements, components, and/or groups thereof.

Furthermore, relative terms, such as "lower" or "bottom" and "upper" or "top," may be used herein to describe one element's relationship to another element as illustrated in the Figures. It will be understood that relative terms are intended to encompass different orientations of the device in addition to the orientation depicted in the Figures. For example, if the device in one of the figures is turned over, elements described as being on the "lower" side of other elements would then be oriented on "upper" sides of the other elements. The term "lower," can therefore, encompass both an orientation of "lower" and "upper," depending on the particular orientation of the figure. Similarly, if the device in one of the figures is turned over, elements described as "below" or "beneath" other elements would then be oriented "above" the other elements. The terms "below" or "beneath" can, therefore, encompass both an orientation of above and below.

"About" or "approximately" as used herein is inclusive of the stated value and means within an acceptable range of deviation for the particular value as determined by one of ordinary skill in the art, considering the measurement in question and the error associated with measurement of the particular quantity (i.e., the limitations of the measurement system). For example, "about" can mean within one or more standard deviations, or within $\pm$ 30%, 20%, 10% or 5% of the stated value.

Unless otherwise defined, all terms (including technical and scientific terms) used herein have the same meaning as commonly understood by one of ordinary skill in the art to which this disclosure belongs. It will be further understood that terms, such as those defined in commonly used dictionaries, should be interpreted as having a meaning that is consistent with their meaning in the context of the relevant art and the present disclosure, and will not be interpreted in an idealized or overly formal sense unless expressly so defined herein.

Embodiments are described herein with reference to cross section illustrations that are schematic illustrations of idealized embodiments. As such, variations from the shapes of the illustrations as a result, for example, of manufacturing techniques and/or tolerances, are to be expected. Thus, embodiments described herein should not be construed as limited to the particular shapes of regions as illustrated herein but are to include deviations in shapes that result, for example, from manufacturing. For example, a region illustrated or described as flat may, typically, have rough and/or nonlinear features. Moreover, sharp angles that are illustrated may be rounded. Thus, the regions illustrated in the figures are schematic in nature and their shapes are not intended to illustrate the precise shape of a region and are not intended to limit the scope of the present claims.

Hereinafter, embodiments of the disclosure will be described. However, the disclosure is not limited to the following embodiments. In addition, unless otherwise specified, measurements of operation and physical properties are performed at room temperature (about 20 °C to about 25 °C) and at relative humidity (RH) of about 40 % RH to about 50 % RH.

As used herein, the expression "X and Y are each independently" refers to X and Y being identical to or different from each other.

As used herein, the term "group derived from a ring" refers to a group having free valence by removing hydrogen atoms directly bonded to ring-forming atoms in a ring structure as much as a valence number.

Recently, a method has been developed to realize high luminescence efficiency while maintaining high color purity of a fluorescent material by combining an auxiliary dopant having TADF properties with the fluorescent material in an emission layer (as disclosed in Hajime Nakanotani et al., "High-efficiency organic light-emitting diodes with fluorescent emitters," Nature Communications, 2014, 5, 4016). Consequently, renewed attention has been focused on the development of fluorescent materials.

A new international standard for television broadcasting, BT.2100, has been announced, and to comply with the color gamut of this standard, light-emitting devices are required to exhibit high color purity for the three colors of red, blue, and green. Improvement in color purity has been achieved by adopting a microcavity structure that uses the resonance effect of light between electrodes. However, since light deviating from a specific wavelength is not extracted, using a luminescent material with a wide full width at half maximum (FWHM) of a spectrum leads to a decrease in the luminescence efficiency of a device. Accordingly, there is remains a need for a luminescent material with a small FWHM of an emission spectrum. Researchers have disclosed a nitrogen-containing compound (Formula 1, reproduced below) with a condensed ring reported to be a blue luminescent material with a small FWHM of an emission spectrum (Morgane Rivoal et al.,

"Substituted dibenzo[2,3:5,6]-pyrrolizino[1,7-bc]indolo[1,2,3-lm]carbazoles: a series of new electron donors," Tetrahedron, 69, (2013), 3302-3307 and Claude Niebel et al., "Dibenzo[2,3:5,6]pyrrolizino[1,7-bc]indolo[1,2,3-lm]carbazole: a new electron donor," New Journal of Chemistry, 2010, 34, 1243-1246). The material emits blue light with a very small FWHM of an emission spectrum for a solution, and is considered promising as a basic core structure for luminescent materials in organic light-emitting devices.

Formula 1

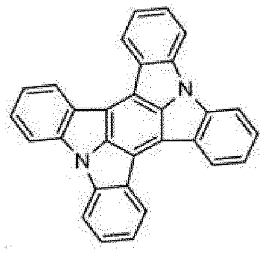

Examples of materials having the structural core as represented by Formula 1 and devices using said materials have been reported. For example, a derivative of the above compound has been used as an active layer of an organic transistor (WO 2013/084805). Japanese Patent Nos. JP 2020-107742 and JP 2022-103061 disclose examples of the use of a derivative of the above compound as a luminescent material for an organic light-emitting device.

Provided is a compound with high color purity due to a small full width at half maximum (FWHM) of an emission spectrum, with a peak wavelength within the blue light wavelength region [e.g., a wavelength of 445 nanometers (nm) to 470 nm), and capable of improving the luminescence efficiency of an organic light-emitting device.

Also provided is an organic light-emitting device including the above-described compound.

Blue luminescent compound

A blue luminescent compound according to the disclosure includes a nitrogen-containing aromatic heterocyclic skeleton and at least two aromatic substituents bonded to the nitrogen-containing aromatic heterocyclic skeleton, wherein the nitrogen-containing aromatic heterocyclic skeleton includes four branched or cyclic alkyl groups each having 3 to 20 carbon atoms, and the nitrogen-containing aromatic heterocyclic skeleton and the at least two aromatic substituents are not located on the same plane. The blue luminescent compound of the disclosure having the above-described structure may have high color purity due to a small full width at half maximum (FWHM) of an emission spectrum, may have a peak wavelength within the blue wavelength region (e.g., a wavelength of 445 nm to 470 nm), and may improve the luminescence efficiency of an organic light-emitting device.

The at least two aromatic substituents may include both an aromatic hydrocarbon group and an aromatic heterocyclic group.

The nitrogen-containing aromatic heterocyclic skeleton of the blue luminescent compound according to the disclosure includes four branched or cyclic alkyl groups each having 3 to 20 carbon atoms, which are bulky substituents. While not wishing to be bound by theory, it is understood that the bulky substituents may suppress intermolecular aggregation. In general, when a compound aggregates, luminescence due to the aggregation state occurs, and the luminescence tends to have a broadened spectrum. Accordingly, color purity deteriorates, making it difficult to achieve luminescence with high color purity. In addition, when a luminescent material aggregates, concentration quenching occurs and luminescence efficiency decreases. In this regard, it is important to suppress aggregation of a luminescent material to obtain a luminescent material with high color purity and high luminescence efficiency, and ultimately, to obtain an organic light-emitting device with high color purity and high luminescence efficiency.

As described above, the blue luminescent compound according to the disclosure includes a bulky substituent, thereby suppressing intermolecular aggregation. Accordingly, the blue luminescent compound according to the disclosure may have high color purity due to a small FWHM of an emission spectrum, may have a peak wavelength within the blue wavelength region (e.g., a wavelength of 445 nm to 470 nm), and may improve the luminescence efficiency of an organic light-emitting device.

The presumed mechanism to suppress intermolecular aggregation is based on assumption, and does not affect the technical scope of the disclosure. In addition, regarding other assumptions made herein, the accuracy of such assumptions does not affect the technical scope of the disclosure.

As used herein, the expression "a nitrogen-containing aromatic heterocyclic skeleton and an aromatic substituent are not located on the same plane" is defined as below. Regarding the blue luminescent compound, the most stable structure is calculated according to (I) $S_0$ calculation method: structural optimization calculation by density

functional theory (DFT) including functional B3LYP, basis function 6-31G(d,p), and toluene solvent effect (PCM) in the "Calculation by DFT" section. From the most stable structure, using GaussView (Gaussian Inc.), a dihedral angle between a nitrogen-containing aromatic heterocyclic skeleton (core portion) and an aromatic substituent bonded to the nitrogen-containing aromatic heterocyclic skeleton (core portion) via a single bond is calculated, and a case where the dihedral angle is about 5° to about 175° is defined as "the nitrogen-containing aromatic heterocyclic skeleton and the aromatic substituent are not located on the same plane."

The dihedral angle between the nitrogen-containing aromatic heterocyclic skeleton (core portion) and the aromatic substituent bonded to the nitrogen-containing aromatic heterocyclic skeleton (core portion) via a single bond is defined as below. When A1 is an atom of the nitrogen-containing aromatic heterocyclic skeleton bonded to the aromatic substituent, A2 is an atom closest to A1 in the nitrogen-containing aromatic heterocyclic skeleton, B1 is an atom of the aromatic substituent bonded to the nitrogen-containing aromatic heterocyclic skeleton, and B2 is an atom closest to B1 in the aromatic substituent, a dihedral angle formed by A2A1B1B2 is defined as an angle formed by triangle △A2A1B1 having vertices A2, A1, and B1 and triangle △B2B1A1 having vertices B2, B1, and A1.

As described above, the dihedral angle may be calculated with GaussView (Gaussian Inc.) from the most stable structure calculated by DFT, with Gaussian 16 (Gaussian Inc.) as the calculation software, according to the calculation method of (I).

As described above, the dihedral angle of the blue luminescent compound according to the disclosure may be in a range of about 5° to about 175°, for example, about 10° to about 170°, or for example, about 30° to about 150°.

The blue luminescent compound according to the invention is represented by Formula (1), wherein $\beta_{sum}$ as defined below is greater than 13 and less than 28.

Formula (1)

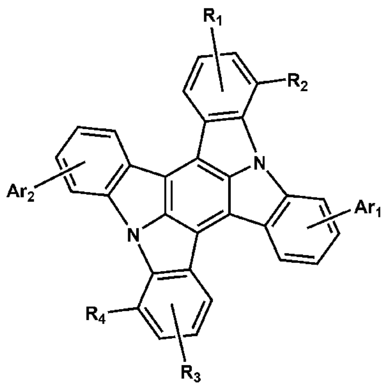

In Formula (1),

$R_1$ to $R_4$ are each independently a substituted or unsubstituted branched or cyclic alkyl group having 3 to 20 carbon atoms or a substituted or unsubstituted trialkylsilyl group having 3 to 60 carbon atoms, and
$Ar_1$ and $Ar_2$ are each independently a substituted or unsubstituted aromatic hydrocarbon cyclic group having 6 to 14 ring atoms or a substituted or unsubstituted aromatic heterocyclic group having 5 to 14 ring atoms:
$\beta_{sum}$
wherein, when $\beta_1$ is a size of substituent $Ar_1$ in Formula (1) in a direction perpendicular to a core represented by Formula (1a),
$\beta_2$ is a size of substituent $Ar_2$ in Formula (1) in the direction perpendicular to the core represented by Formula (1a),
$\beta_3$ is a size of substituent $R_1$ in Formula (1) in the direction perpendicular to the core represented by Formula (1a),
$\beta_4$ is a size of substituent $R_2$ in Formula (1) in the direction perpendicular to the core represented by Formula (1a),
$\beta_5$ is a size of substituent $R_3$ in Formula (1) in the direction perpendicular to the core represented by Formula (1a), and
$\beta_6$ is a size of substituent $R_4$ in Formula (1) in the direction perpendicular to the core represented by Formula (1a),
$\beta_{sum}=\beta_1+\beta_2+\beta_3+\beta_4+\beta_5+\beta_6$.

In this regard, the direction perpendicular to the core is defined as a direction perpendicular to triangle △A2A1B1 described above.

Formula (1a)

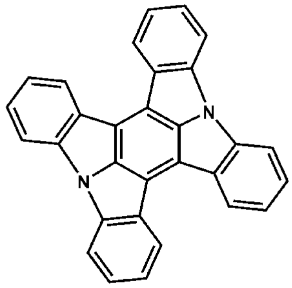

$\beta_{sum}$ may be an index indicating the total length of substituents extending in a direction perpendicular to the plane of the core represented by the Formula (1a) of the blue luminescent compound. When $\beta_{sum}$ is greater than 13 and less than 28, the blue luminescent compound may have more suppressed aggregation, higher color purity, and a peak wavelength within the blue wavelength region (e.g., a wavelength of 445 nm to 470 nm), and may improve the luminescence efficiency when used in an organic light-emitting device. When $\beta_{sum}$ is 13 or less, an emission wavelength may shift toward a longer wavelength. In addition, when $\beta_{sum}$ is 28 or more, an emission wavelength may shift toward a longer wavelength and color purity may deteriorate.

$\beta_{sum}$ may be about 15 to about 25, for example, about 20 to about 23.

Herein, $\beta_1$ to $\beta_6$ may be calculated with GaussView (Gaussian Inc.) from the most stable structure calculated according to (I) $S_0$ calculation method: structural optimization calculation by DFT including functional B3LYP, basis function 6-31G(d, p), and toluene solvent effect (PCM) in the "Calculation by DFT" section. Hereinafter, a method of calculating $\beta_1$, which is the size of substituent $Ar_1$ in the direction perpendicular to the core represented by Formula (1a), will be described as an example. $\alpha$, $\theta$, and $\varphi$ described below may be obtained by using GaussView.

FIGS. 1A to 1C are schematic diagrams illustrating a process of calculating $\beta_1$. A1 is an atom of the core represented by Formula (1a), which is bonded to substituent $Ar_1$ including n atoms excluding hydrogen atoms, A2 is an atom closest to A1, B1 is an atom of substituent $Ar_1$ bonded to the core, and Bn is an atom in substituent $Ar_1$. In addition, molecules are located such that A1 and B1 overlap the x-axis and the core overlaps the xy-plane (FIG. 1A).

$\alpha$ is a length between B1 and Bn, and $\theta$ is an angle formed by A1B1Bn. In projection $Ar_1$' of substituent $Ar_1$ onto the yz-plane, Bn corresponds to Bn'. Accordingly, a length between B1 and Bn' is represented by $\alpha\cdot|\sin\theta|$ (FIGS. 1A and 1B).

When Bn' is projected onto the z-axis, Bn" is a point corresponding to Bn'. When $\varphi$ is a dihedral angle formed by A2A1B1Bn, an angle formed by the y-axis and Bn' may be represented by $\varphi$. Accordingly, a length between B1 and Bn" is represented by $\alpha\cdot|(\sin\theta)|\cdot|(\sin\varphi)|$ (FIG. 1C).

The same calculation is performed for all atoms (B1, B2, ..., Bn) of substituent $Ar_1$, wherein $\beta_1$ is a distance between two points that are largest among all combinations of B1 to Bn. For substituents $Ar_2$, $R_1$, $R_2$, $R_3$, and $R_4$, $\beta_2$, $\beta_3$, $\beta_4$, $\beta_5$, and $\beta_6$ are calculated in the same manner as described above, wherein the total sum ($\beta_1+\beta_2+\beta_3+\beta_4+\beta_5+\beta_6$) is defined as $\beta_{sum}$. The detailed calculation method will be described in the Examples.

Examples of the branched alkyl group having 3 to 20 carbon atoms, which is used as $R_1$ to $R_4$ in Formula (1), may include an isopropyl group, an isobutyl group, a sec-butyl group, a tert-butyl group, an isopentyl group, a neopentyl group, a tert-pentyl group, a sec-isoamyl group, an isohexyl group, a neohexyl group, a 4-methylhexyl group, a 5-methylhexyl group, a 1-ethylhexyl group, a 2-ethylhexyl group, a 3-ethylhexyl group, a 4-ethylhexyl group, a 2-ethylpentyl group, a heptan-3-yl group, a heptan-4-yl group, a 4-methylhexan-2-yl group, a 3-methylhexan-3-yl group, a 2,3-dimethylpentan-2-yl group, a 2,4-dimethylpentan-2-yl group, a 4,4-dimethylpentan-2-yl group, a 6-methylheptyl group, a 2-ethylhexyl group, an octan-2-yl group, a 6-methylheptan-2-yl group, a 6-methyloctyl group, a 3,5,5-trimethylhexyl group, a nonan-4-yl group, a 2,6-dimethylheptan-3-yl group, a 3,6-dimethylheptan-3-yl group, a 3-ethylheptan-3-yl group, a 3,7-dimethyloctyl group, a 8-methylnonyl group, a 3-methylnonan-3-yl group, a 4-ethyloctan-4-yl group, a 9-methyldecyl group, an undecan-5-yl group, a 3-ethylnonan-3-yl group, a 5-ethylnonan-5-yl group, a 2,2,4,5,5-pentamethylhexan-4-yl group, a 10-methylundecyl group, a 11-methyldodecyl group, a tridecan-6-yl group, a tridecan-7-yl group, a 7-ethylundecan-2-yl group, a 3-ethylundecan-3-yl group, a 5-ethylundecan-5-yl group, a 12-methyltridecyl group, a 13-methyltetradecyl group, a pentadecan-7-yl group, a pentadecan-8-yl group, a 14-methylpentadecyl group, a 15-methylhexadecyl group, a heptadecan-8-yl group, a heptadecan-9-yl group, a 3,13-dimethylpentadecan-7-yl group, a 2,2,4,8,10,10-hexamethylundecan-5-yl group, a 16-methylheptadecyl group, a 17-methyloctadecyl group, a nonadecan-9-yl group, a nonadecan-10-yl group, a 2,6,10,14-tetramethylpentadecan-7-yl group, a 18-methylnonadecyl group, and the like.

In addition, examples of the cyclic alkyl group having 3 to 20 carbon atoms, which is used as $R_1$ to $R_4$ in Formula (1), may include a cyclopropyl group, a cyclobutyl group, a cyclopentyl group, a cyclohexyl group, a cycloheptyl group, a cyclooctyl group, a cyclononyl group, a cyclodecyl group, a cycloundecyl group, a cyclododecyl group, a cyclotridecyl group, a cyclotetradecyl group, a cyclopentadecyl group, a cyclohexadecyl group, a cycloheptadecyl group, a cyclooctadecyl group, a cyclononadecyl group, a cycloeicosyl group, and the like.

Examples of the trialkylsilyl group having 3 to 60 carbon atoms, which is used as $R_1$ to $R_4$ in Formula (1), may include, but are not particularly limited to, a trimethylsilyl group, a triethylsilyl group, a tri-iso-propylsilyl group, a tri-tert-butylsilyl group, a di-tert-butyl(methyl)silyl group, a tert-butyldimethylsilyl group, and the like. The number of carbon atoms per alkyl group in the trialkylsilyl group may be 1 to 20. The alkyl group having 1 to 20 carbon atoms may be linear, branched, or cyclic.

The branched or cyclic alkyl group having 3 to 20 carbon atoms, which is used as $R_1$ to $R_4$ in Formula (1), may be an isopropyl group, a tert-butyl group, or a cyclohexyl group.

The aromatic hydrocarbon group used as $Ar_1$ and $Ar_2$ in Formula (1) refers to a group derived from a hydrocarbon ring that is partially or entirely aromatic. When the aromatic hydrocarbon group includes two or more partially or entirely aromatic hydrocarbon rings, the rings may be bonded via a single bond or condensed to each other. The number of ring-forming atoms in the aromatic hydrocarbon group may be 6 to 14, for example, 6 to 10, or for example, 6. Examples of the aromatic hydrocarbon group (also called an "aromatic hydrocarbon cyclic group") having 6 to 14 ring-forming atoms may include, but are not particularly limited to, a phenyl group, a biphenyl group, a naphthyl group, a fluorenyl group, an anthracenyl group, a phenanthrenyl group, and the like. In particular, the aromatic hydrocarbon group may be a phenyl group, a naphthyl group, or an anthracenyl group, for example, a phenyl group.

The aromatic heterocyclic group used as $Ar_1$ and $Ar_2$ in Formula (1) refers to a group derived from a hetero ring that is partially or entirely aromatic. When the aromatic heterocyclic group includes two or more partially or entirely aromatic hetero rings, the rings may be partially or entirely bonded to each other via a single bond. When the aromatic heterocyclic group includes two or more partially or entirely aromatic hetero rings or other rings, the rings may be condensed with each other. In addition, when the aromatic heterocyclic group includes two or more partially or entirely aromatic hetero rings or other rings, one atom may serve as a ring-forming atom of the rings. Examples of a hetero atom in the aromatic heterocyclic group may include, but are not particularly limited to, a nitrogen atom (N), an oxygen atom (O), a phosphorus atom (P), a sulfur atom (S), a silicon atom (Si), and the like. The number of ring-forming atoms in the aromatic heterocyclic group may be 5 to 14, for example, 5 to 13. The number of hetero atoms in the aromatic heterocyclic group may be, but is not particularly limited to, 1 to 3. In addition, the number of hetero atoms in the aromatic heterocyclic group may be 1 or 2, for example, 1. Examples of the aromatic heterocyclic group may include, but are not particularly limited to, a thiophenyl group, a furanyl group, a pyranyl group, an imidazonyl group, a thiazonyl group, an oxazonyl group, an oxadiazonyl group, a triazonyl group, a pyridyl group, a bipyridyl group, a pyrimidyl group, a triazinyl group, a triazolyl group, a pyridazinyl group, a pyridinyl group, a quinolinyl group, a quinazolinyl group, a quinoxalinyl group, a phenoxadinyl group, a phthalazinyl group, a pyridpyrimidinyl group, a pyridpyrazinyl group, a pyrazinopyrazinyl group, an isoquinolinyl group, an indolyl group, a carbazolyl group, a benzoxazolyl group, a benzimidazolyl group, a benzothiazolyl group, a benzothiophenyl group, a dibenzothiophenyl group, a thienothiophenyl group, a benzofuranyl group, a phenanthrolinyl group, a thiazolyl group, an isoxazolyl group, an oxadiazolyl group, a thiadiazolyl group, a phenothiazinyl group, a dibenzosilolyl group, a dibenzofuranyl group, a xanthonyl group, and the like. In particular, the aromatic heterocyclic group may be a pyridyl group (a 2-pyridyl group, a 3-pyridyl group, or a 4-pyridyl group), a dibenzothiophenyl group, or a dibenzofuranyl group.

The branched alkyl group, the cyclic alkyl group, the aromatic hydrocarbon group, and the aromatic heterocyclic group used as $R_1$ to $R_4$ and $Ar_1$ to $Ar_2$ in Formula (1) may each be a substituted group. Examples of a substituent may include, but are not particularly limited to, an alkyl group, an alkenyl group, an alkynyl group, a halogen atom, a cyano group, a trialkylsilyl group having 3 to 60 carbon atoms, and the like. In this regard, the number of carbon atoms per alkyl group in the trialkylsilyl group as a substituent may be 1 to 20. The alkyl group having 1 to 20 carbon atoms may be linear, branched, or cyclic.

The alkyl group, the alkenyl group, and the alkynyl group as substituents may each be linear, branched, or cyclic. The number of carbon atoms in the alkyl group may be, but is not particularly limited to, 1 or more. The number of carbon atoms in each of the alkenyl group and the alkynyl group may be, but is not particularly limited to, 2 or more. In addition, the number of carbon atoms in each of the alkyl group, the alkenyl group, and the alkynyl group may be 20 or less, for example, 10 or less, or for example, 4 or less. Examples of the linear alkyl group may include, but are not particularly limited to, a methyl group, an ethyl group, an n-propyl group, an n-butyl group, an n-pentyl group, an n-hexyl group, an n-heptyl group, an n-octyl group, an n-nonyl group, an n-decyl group, n-undecyl group, an n-tridecyl group, an n-tetradecyl group, an n-pentadecyl group, an n-hexadecyl group, an n-heptadecyl group, an n-octadecyl group, an n-nonadecyl group, an n-eicosyl group, and the like. Examples of the branched alkyl group and the cyclic alkyl group may be the same as described above.

Examples of the alkenyl group may include, but are not particularly limited to, a vinyl group, a 2-prophenyl group, a 2-butenyl group, a 3-butenyl group, a 1-methyl-2-prophenyl group, a 2-methyl-2-prophenyl group, a 2-pentenyl group, a 3-pentenyl group, a 4-pentenyl group, a 1-methyl-2-butenyl group, a 2-methyl-2-butenyl group, a 3-methyl-2-butenyl group, a 1-methyl-3-butenyl group, a 2-methyl-3-butenyl group, a 3-methyl-3-butenyl group, a 1,1-dimethyl-2-prophenyl group, a 1,2-dimethyl-2-prophenyl group, a 1-ethyl-2-prophenyl group, and the like. Examples of the alkynyl group may include, but

are not particularly limited to, a 2-butynyl group, a 3-pentynyl group, a hexynyl group, a heptynyl group, an octynyl group, a decynyl group, and the like.

A peak wavelength in photoluminescence (PL) of the blue luminescent compound according to an embodiment may be, but is not particularly limited to, 400 nm or more. In addition, the peak wavelength may be 420 nm or more, for example, 445 nm or more. In addition, the peak wavelength may be 500 nm or less, for example, 490 nm or less, or for example, 470 nm or less. When the peak wavelength is within the ranges above, the color quality of blue luminescence may be improved.

The blue luminescent compound according to an embodiment may have a small (narrow) FWHM value of an emission intensity of a peak having the PL peak wavelength above. In detail, the FWHM may be 30 nm or less, for example, 25 nm or less, or for example, 20 nm or less. When the FWHM is within the ranges above, the color purity of luminescence may be improved. A lower limit of the FWHM may be 1 nm or more.

The peak wavelength in PL and the FWHM in PL may each be measured/calculated using a spectrofluorophotometer. The measurement/calculation method will be described in detail in the Examples.

Hereinafter, Blue Luminescent Compounds 1 to 48 according to an embodiment will be described in detail. However, the disclosure is not limited to such examples.

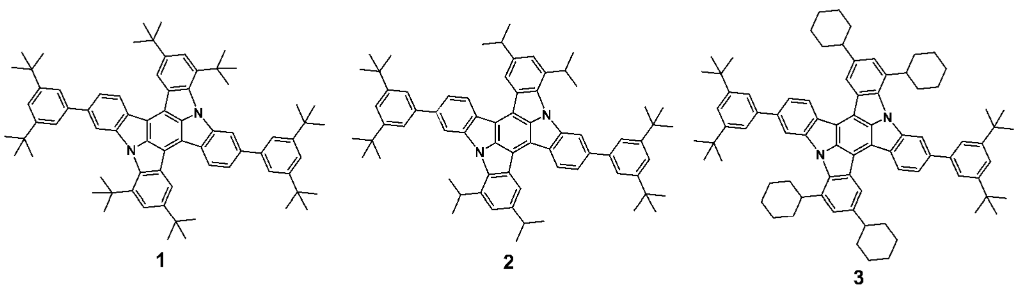

1 2 3

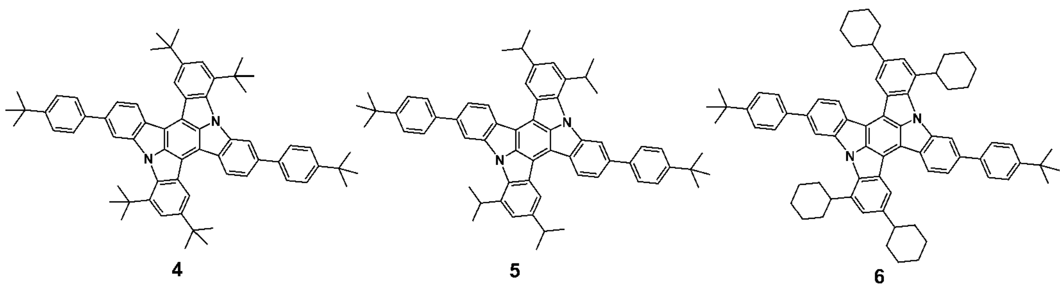

4 5 6

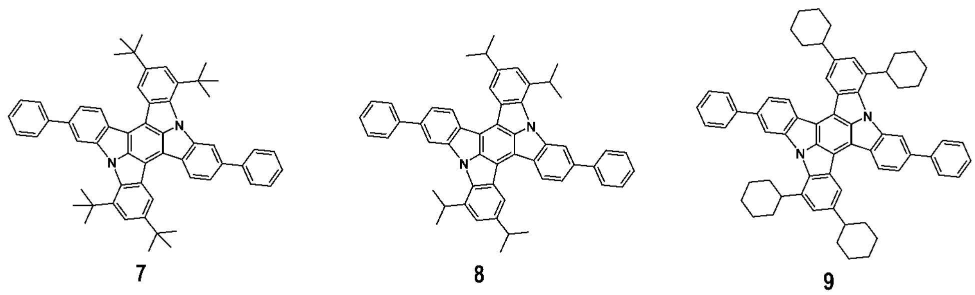

7 8 9

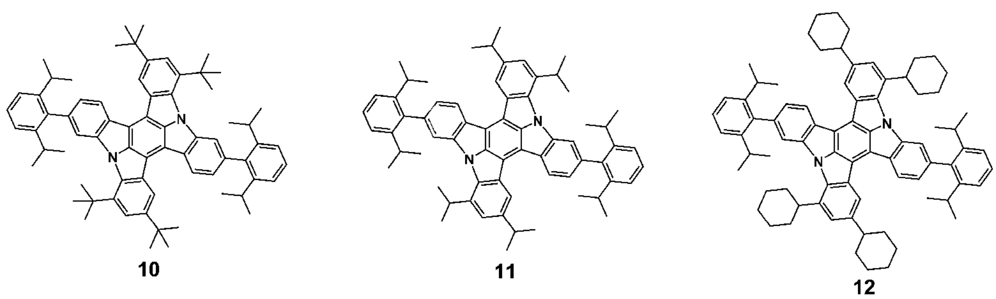

10 11 12

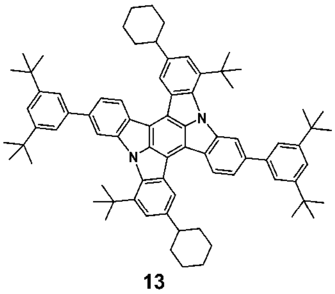

13

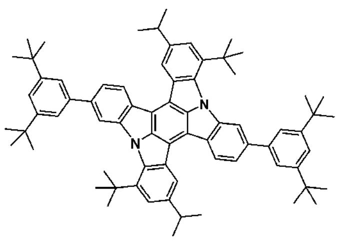

14

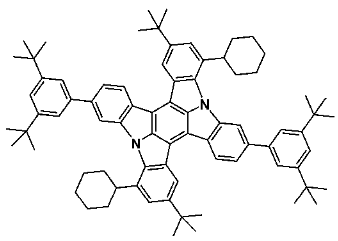

15

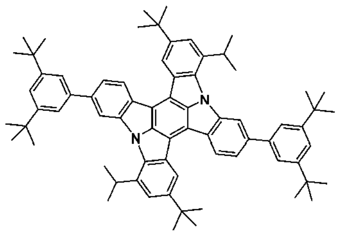

16

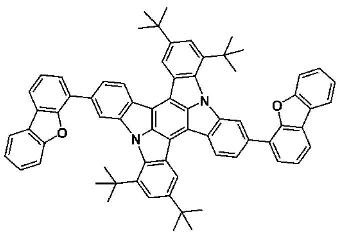

17

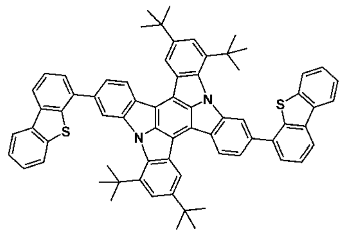

18

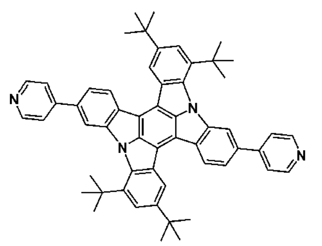

19

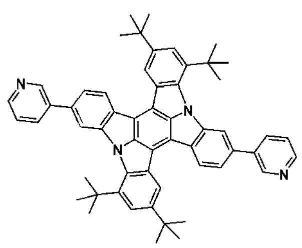

20

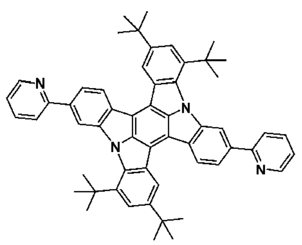

21

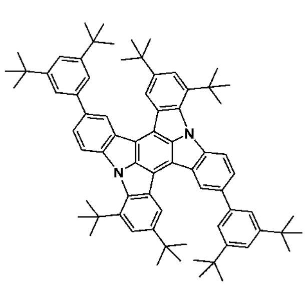

22

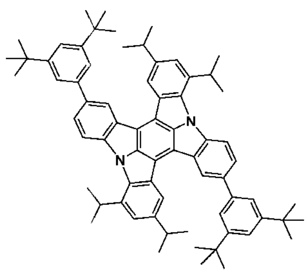

23

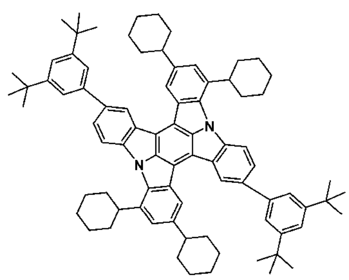

24

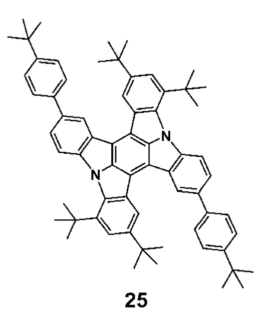

25

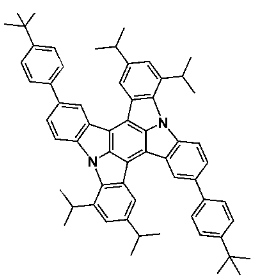

26

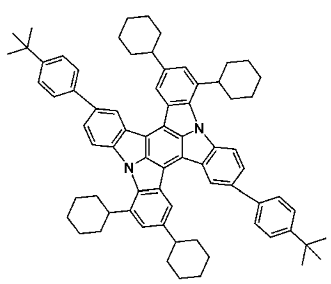

27

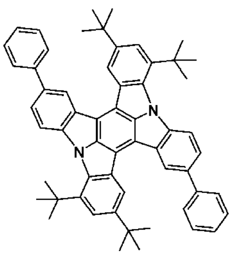

28

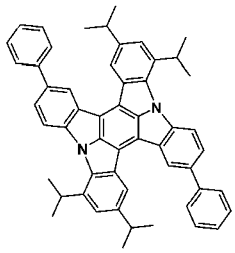

29

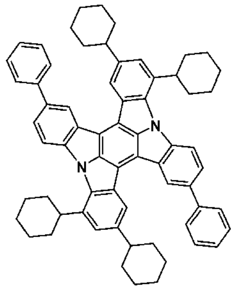

30

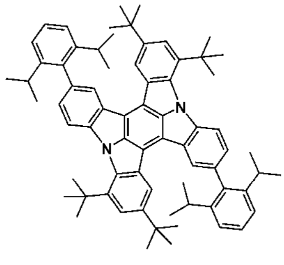

31

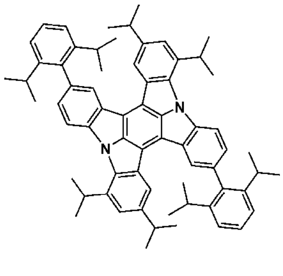

32

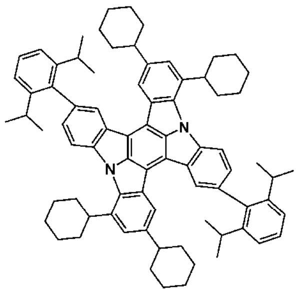

33

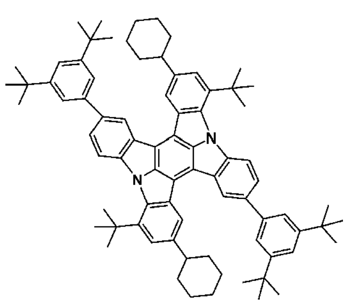

34

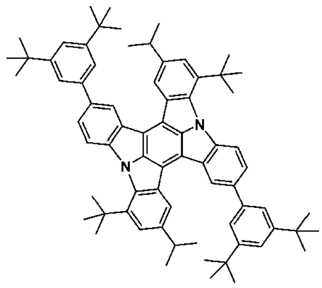

35

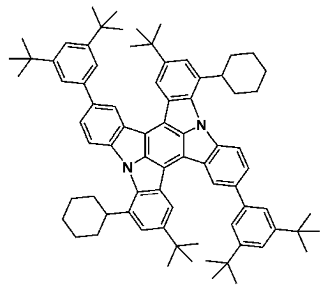

36

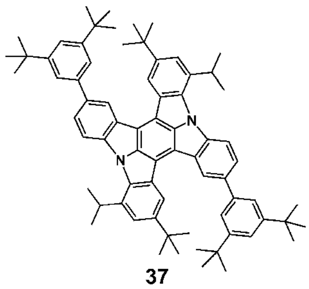

37

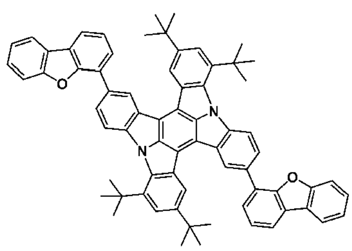

38

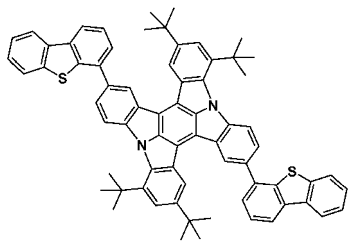

39

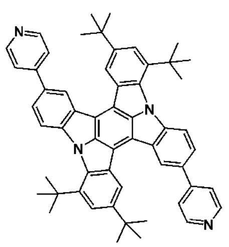

40

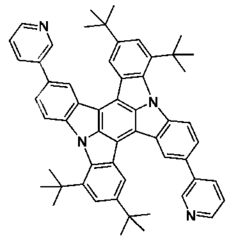

41

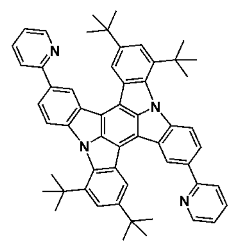

42

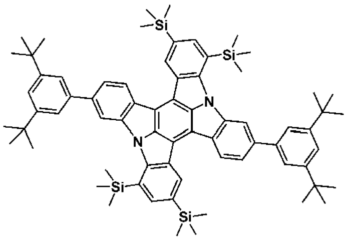

43

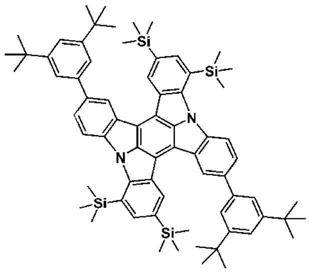

44

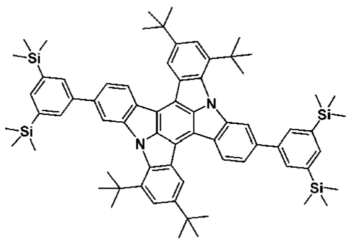

45

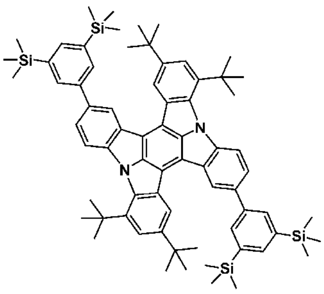

46

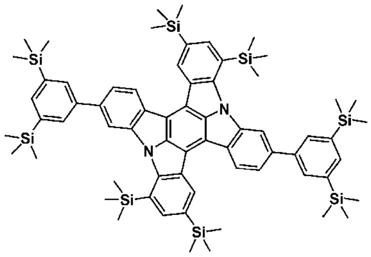

47

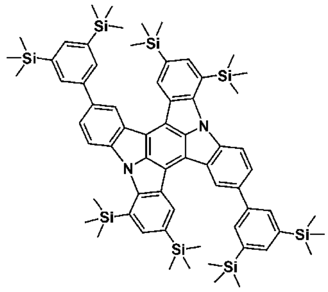

48

Among the blue luminescent compounds above, a compound represented by Formula (2) and a compound represented by Formula (3) may be more suitable in view of exhibiting effects of the disclosure to a greater extent.

Formula (2)

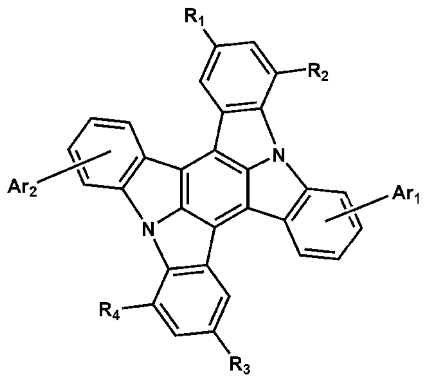

In Formula (2),

$R_1$ to $R_4$ may each independently be a substituted or unsubstituted branched or cyclic alkyl group having 3 to 20 carbon atoms or a substituted or unsubstituted trialkylsilyl group having 3 to 60 carbon atoms, and
$Ar_1$ and $Ar_2$ may each independently be a substituted or unsubstituted aromatic hydrocarbon cyclic group having 6 to 14 ring atoms or a substituted or unsubstituted aromatic heterocyclic group having 5 to 14 ring atoms.

Formula (3)

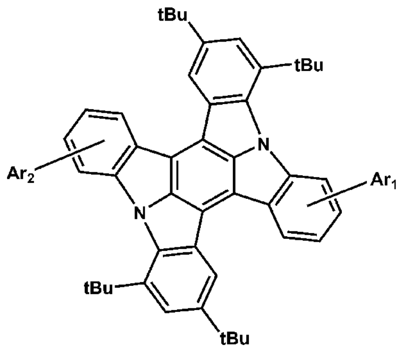

In Formula (3),

$Ar_1$ and $Ar_2$ may each independently be a substituted or unsubstituted aromatic hydrocarbon cyclic group having 6 to 14 ring atoms or a substituted or unsubstituted aromatic heterocyclic group having 5 to 14 ring atoms, and
tBu represents a tert-butyl group.

Among Blue Luminescent Compounds 1 to 48, Compounds 1, 2, 4, 5, 7, 10, 14, 17, 18, 19, 20, 21, 22, 25, 26, 28, 31, 38, 39, 40, 41, and 42 may be more suitable.

A method of synthesizing the blue luminescent compound according to an embodiment is not particularly limited, and the blue luminescent compound may be synthesized based on known synthesis methods. In particular, the blue luminescent compound may be synthesized according to or in view of a method described in the Examples. For example, the blue luminescent compound may be synthesized by changing raw materials or reaction conditions in the method described in the Examples, adding or excluding some processes to or from the method described in the Examples, or appropriately combining the method described in the Examples with a known synthesis method.

A method of identifying the structure of the blue luminescent compound according to an embodiment is not particularly limited. The structure of the blue luminescent compound according to the disclosure may be identified by, for example, a known method (e.g., nuclear magnetic resonance spectroscopy, liquid chromatography-mass spectrometry, etc.).

Fluorescence emitter

Another aspect of the disclosure relates to a fluorescence emitter which includes the luminescent compound and is used with a phosphorescent complex to be described below. By using the fluorescence emitter with the phosphorescent complex, luminescence efficiency and device lifespan may be significantly improved. It is assumed that the improvements in device performance are due to energy transfer from the phosphorescent complex to the blue luminescent compound by a fluorescence resonance energy transfer (FRET) mechanism. As a result, energy may be transferred with high efficiency from the phosphorescent complex to the blue luminescent compound.

The fluorescence emitter according to the disclosure may have a peak wavelength of an emission spectrum within the blue wavelength range, thereby realizing blue luminescence.

Details of the phosphorescent complex may be the same as described below. In addition, the fluorescence emitter may be used with a host material to be described below. Details of the host material may be the same as described below.

Material for organic light-emitting device

Another aspect of the disclosure relates to a material for an organic light-emitting device which includes the blue luminescent compound. The material may be a material for an emission layer.

The material for an organic light-emitting device according to an embodiment may include the blue luminescent compound and other materials used in an organic light-emitting device. The other materials used in an organic light-emitting device may be, but are not particularly limited to, phosphorescent compounds or host materials. In addition, the other materials may be a phosphorescent complex and a host material. In this regard, the blue luminescent compound may be used as a dopant material, and the phosphorescent complex may be used as an auxiliary dopant. By using the blue luminescent compound with the phosphorescent complex or the host material (the phosphorescent complex and the host material), luminescence efficiency and device lifespan may be significantly improved. When the material for an organic

light-emitting device contains the host material, the phosphorescent complex is assumed to receive energy from the host material. In addition, the phosphorescent complex presumably transfers energy to the blue luminescent compound by a FRET mechanism. As a result, energy may be transferred with high efficiency from the phosphorescent complex to the blue luminescent compound. In addition, the other materials used in an organic light-emitting device may include other materials known in the art.

When the material for an organic light-emitting device is a material for an emission layer, an amount of the blue luminescent compound may be, but is not particularly limited to, 0.05 weight percent (wt%) or more based on the total weight of the material for an emission layer. In addition, the amount may be 0.1 wt% or more, for example, 0.2 wt% or more. When the amount is within the ranges above, an organic light-emitting device with high luminescence color purity and high luminescence efficiency may be obtained. In addition, an amount of the blue luminescent compound may be, but is not particularly limited to, 50 wt% or less based on the total weight of the material for an organic light-emitting device (in particular, the material for an emission layer). In addition, the amount may be 30 wt% or less, for example, 25 wt% or less, for example, 10 wt% or less, for example, 5 wt% or less, or for example, 2 wt% or less. When the amount is within the ranges above, an organic light-emitting device with high luminescence color purity and high luminescence efficiency may be obtained.

Phosphorescent complex

The material for an organic light-emitting device according to an embodiment may further include a phosphorescent complex in addition to the blue luminescent compound. By including the phosphorescent complex, luminescence efficiency and device lifespan may be significantly improved. The reason for such improvement may be because, as described above regarding the fluorescence emitter, energy may be transferred with high efficiency from the phosphorescent complex to the blue luminescent compound.

The phosphorescent complex may be, but is not particularly limited to, a metal complex in view of luminescence efficiency. The phosphorescent complex may be a platinum complex or a palladium complex, for example, a platinum complex. Accordingly, in the material for an organic light-emitting device according to an embodiment, the phosphorescent complex may be, for example, a platinum complex.

The phosphorescent complex may be, but is not particularly limited to, a compound having a structure of Formula (4), in view of luminescence color purity and luminescence efficiency.

Formula (4)

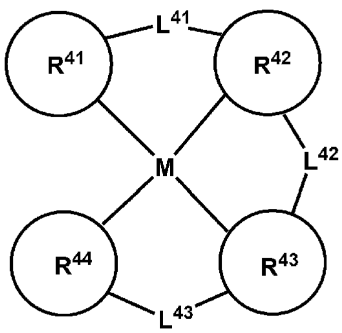

In Formula (4), M may be a metal ion having a coordination number of 4,

$R^{41}$, $R^{42}$, $R^{43}$, and $R^{44}$ may each independently be a substituted or unsubstituted hydrocarbon cyclic group or a substituted or unsubstituted heterocyclic group,
$L^{41}$ may be a linking group linking $R^{41}$ and $R^{42}$,
$L^{42}$ may be a linking group linking $R^{42}$ and $R^{43}$, and
$L^{43}$ may be a linking group linking $R^{43}$ and $R^{44}$.

In Formula (4), the hydrocarbon cyclic group refers to a group derived from at least one hydrocarbon ring. When the hydrocarbon cyclic group includes two or more hydrocarbon rings, the rings may be partially or entirely bonded via a single bond or condensed to each other. In addition, when the hydrocarbon cyclic group includes two or more hydrocarbon rings, one atom may serve as a ring-forming atom of any of the rings.

In Formula (4), the heterocyclic group refers to a group derived from at least one hetero ring. The heterocyclic group may be, but is not particularly limited to, an aromatic heterocyclic group or a non-aromatic heterocyclic group. In particular, the heterocyclic group may be an aromatic heterocyclic group in view of luminescence color purity.

Examples of a substituent substituting the hydrocarbon cyclic group or the heterocyclic group in Formula (4) may include, but are not particularly limited to, the substituents described above.

In Formula (4), M may be a platinum (Pt) ion or a palladium (Pd) ion, for example, a platinum (Pt) ion.

As the phosphorescent complex, a known compound may be used. For example, the platinum complex described in Tyler Fleetham et al., "Efficient "Pure" Blue OLEDs Employing Tetradentate Pt Complexes with a Narrow Spectral Bandwidth," Advanced Materials, 2014, 26, 7116-7121, the platinum complex described in European Patent Publication No. 3670520, the platinum complex and palladium complex described in Japanese Patent Publication No. 2019-029500, or the platinum complex described in U.S. Patent Publication No. 2015/0162552 may be used.

Hereinafter, the phosphorescent complex according to an embodiment will be described in detail. However, the disclosure is not limited to such examples.

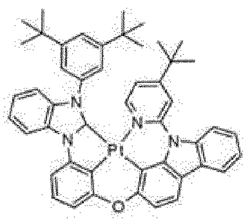
P 1

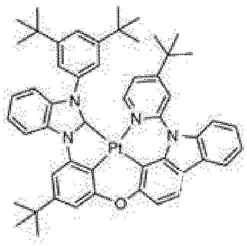
P 2

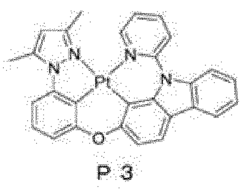
P 3

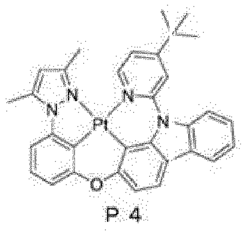
P 4

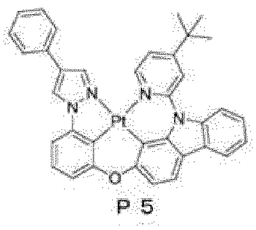
P 5

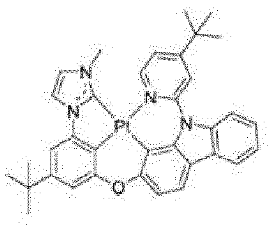
P 6

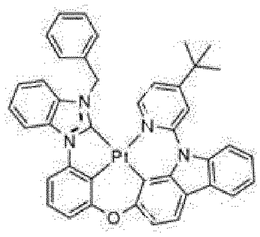
P 7

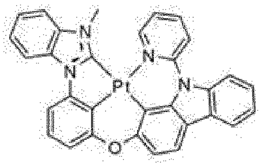
P 8

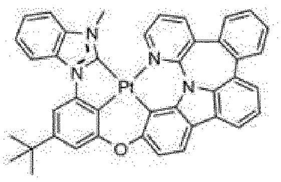
P 9

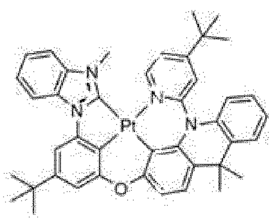
P 1 0

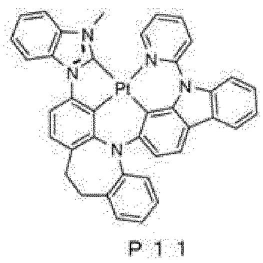
P 1 1

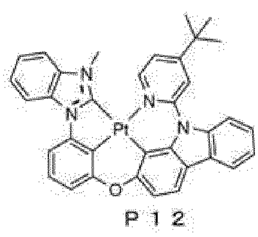

P12

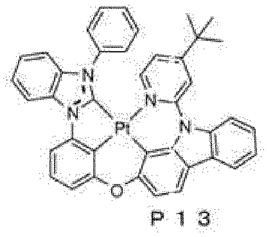

P13

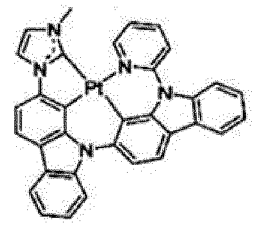

P14

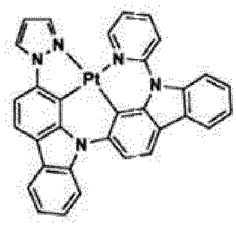

P15

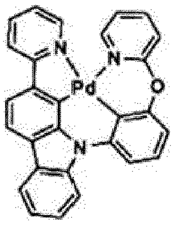

P16

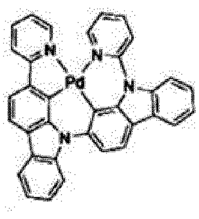

P17

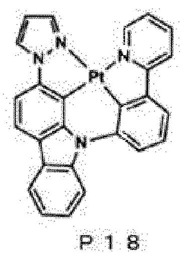

P18

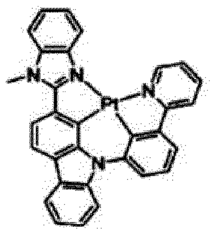

P19

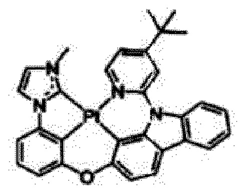

P20

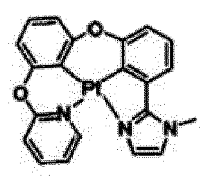

P21

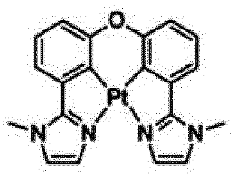

P22

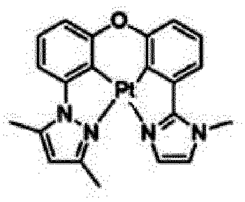

P23

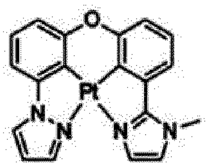

P24

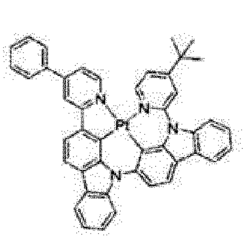

P25

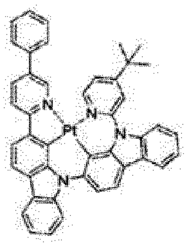

P26

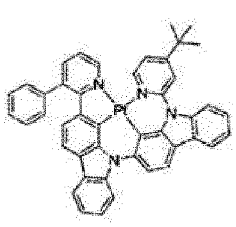

P27

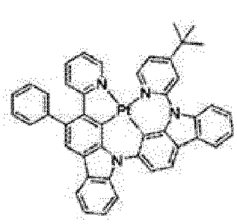

P28

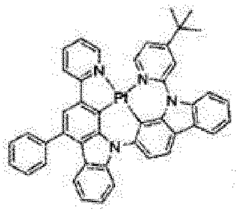

P29

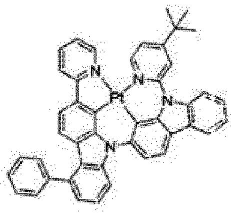

P30

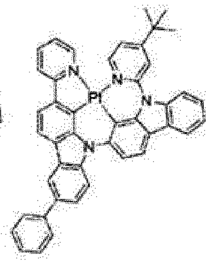

P31

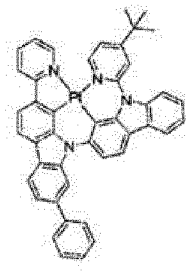

P32

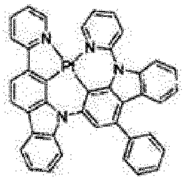

P33

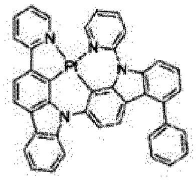

P34

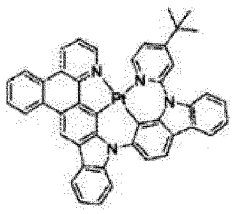

P35

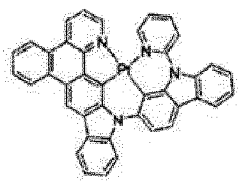

P36

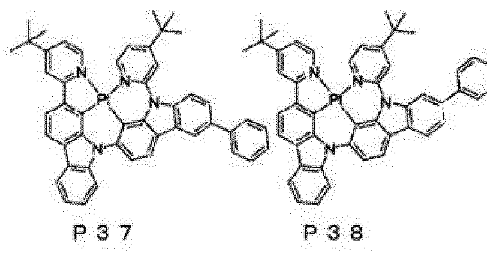

P37

P38

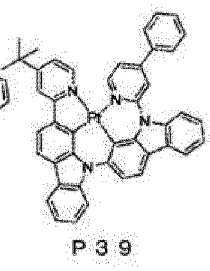

P39

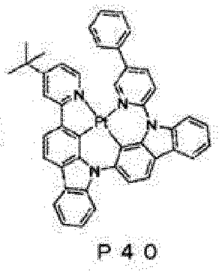

P40

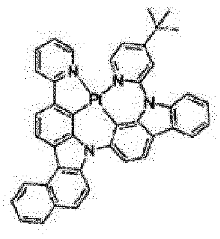

P41

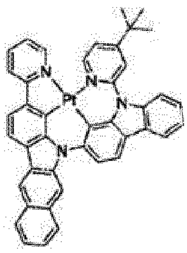

P42

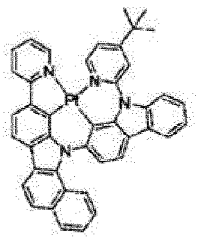

P43

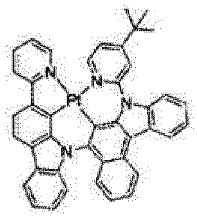

P44

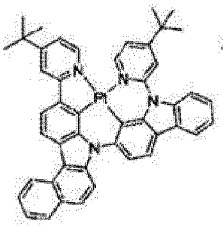

P45

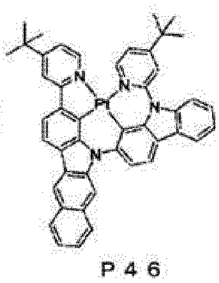

P46

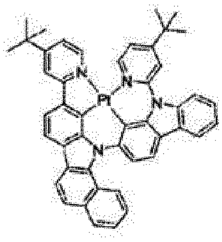

P47

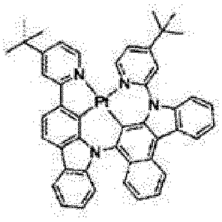

P48

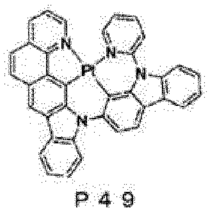
P49

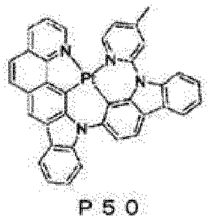
P50

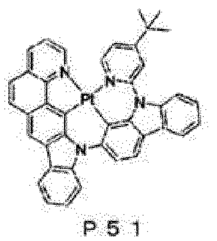
P51

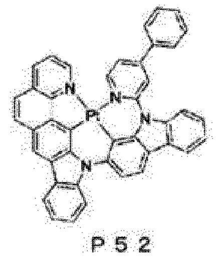
P52

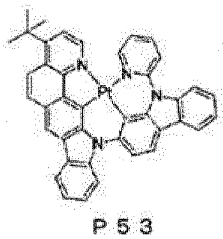
P53

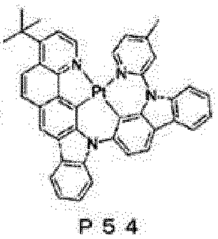
P54

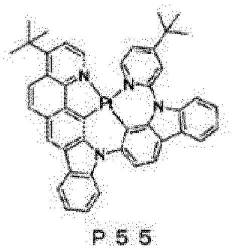
P55

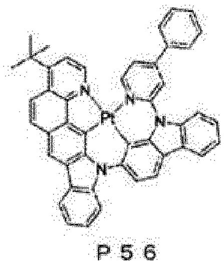
P56

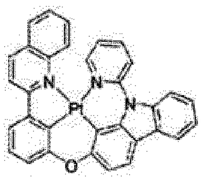
P57

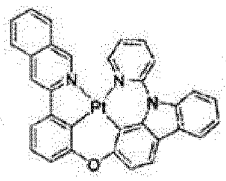
P58

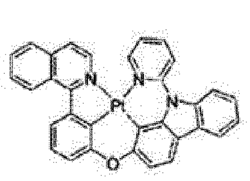
P58

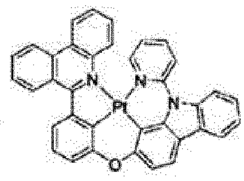
P59

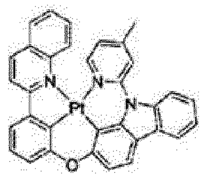
P60

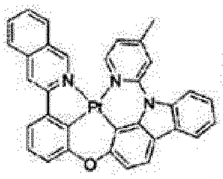
P61

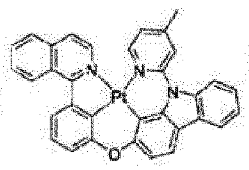
P62

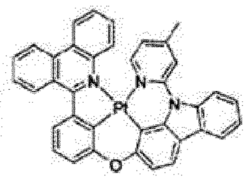
P63

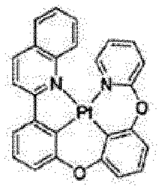
P64

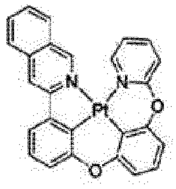
P65

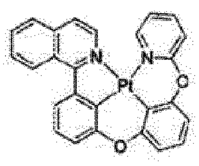
P66

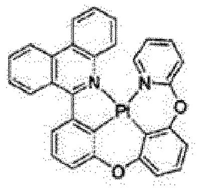
P67

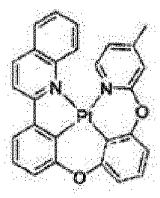
P68

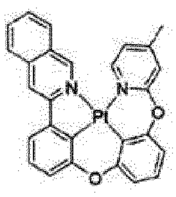
P69

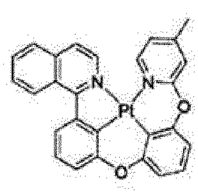
P70

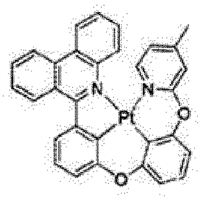
P71

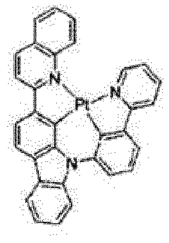
P 7 2

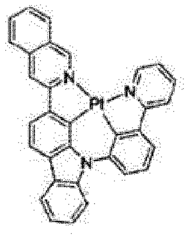
P 7 3

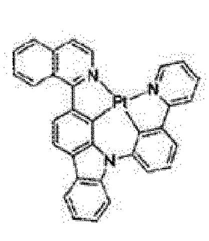
P 7 4

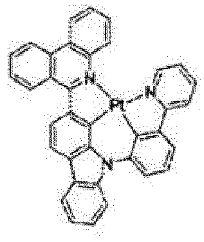
P 7 5

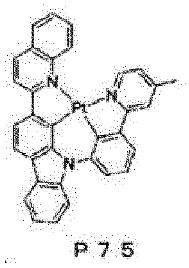
P 7 5

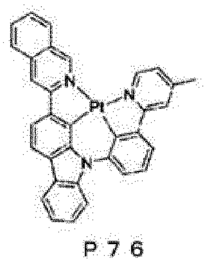
P 7 6

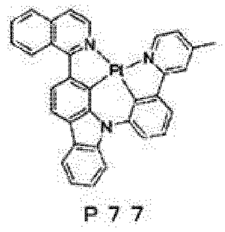
P 7 7

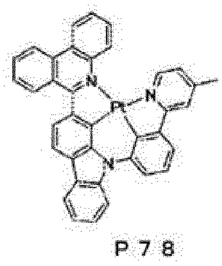
P 7 8

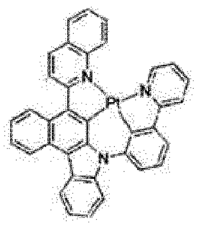
P 7 9

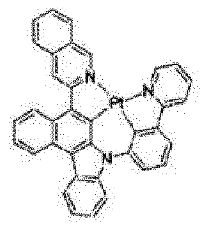
P 8 0

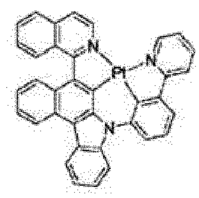
P 8 1

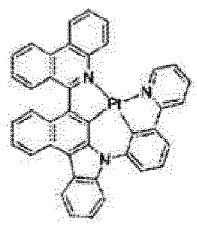
P 8 2

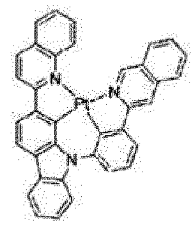
P 8 3

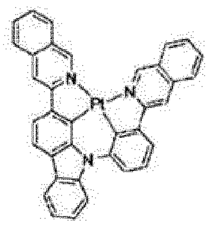
P 8 4

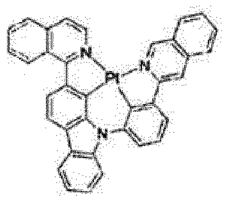
P 8 5

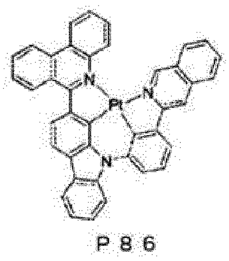
P 8 6

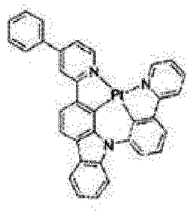
P 8 7

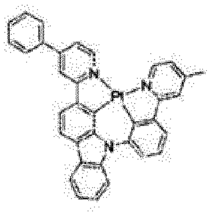
P 8 8

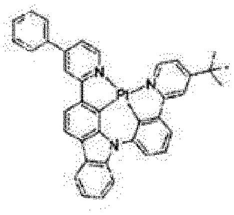
P 8 9

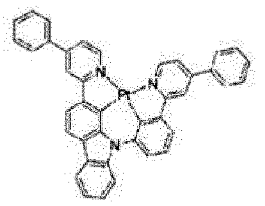
P 9 0

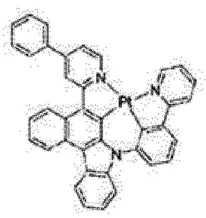
P 9 1

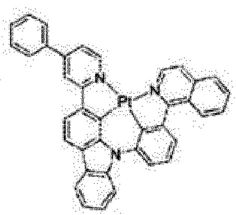
P 9 2

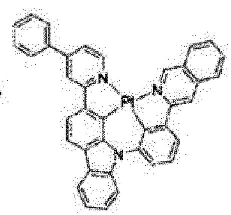
P 9 3

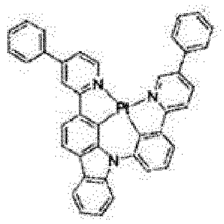
P 9 4

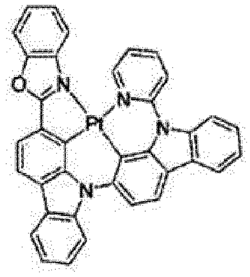
P 9 5

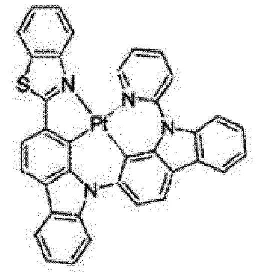
P 9 6

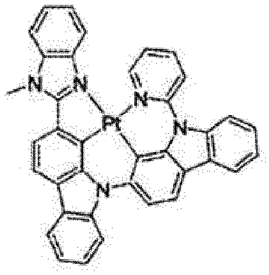
P 9 7

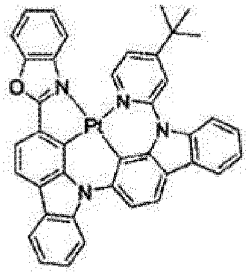
P 9 8

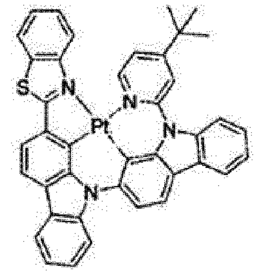
P 9 9

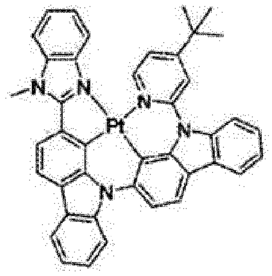
P 1 0 0

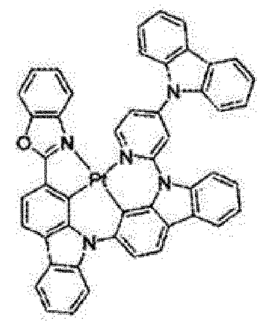
P 1 0 1

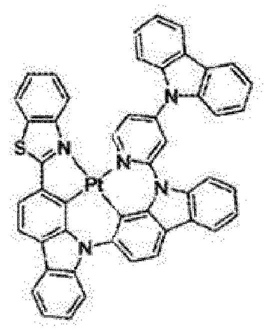
P 1 0 2

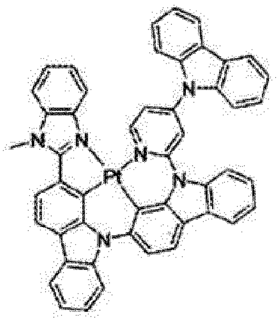
P 1 0 3

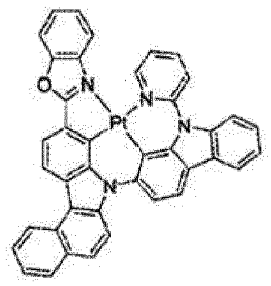
P 1 0 4

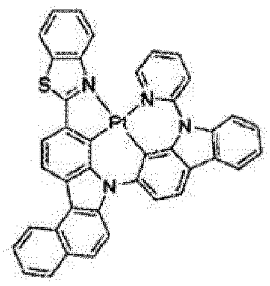
P 1 0 5

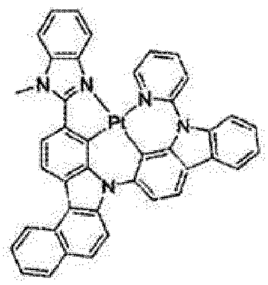
P 1 0 6

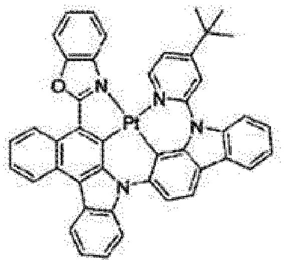
P 1 0 7

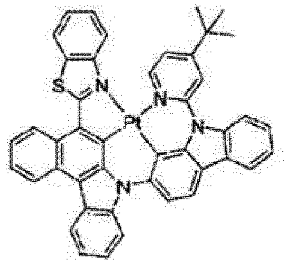
P 1 0 8

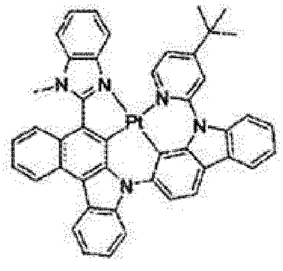
P 1 0 9

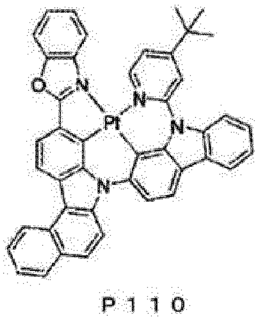
P 1 1 0

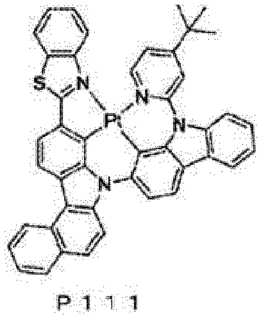
P 1 1 1

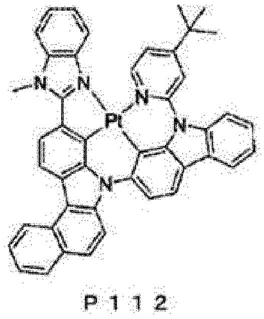
P 1 1 2

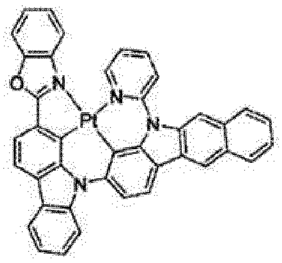
P 1 1 3

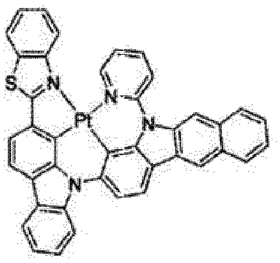
P 1 1 4

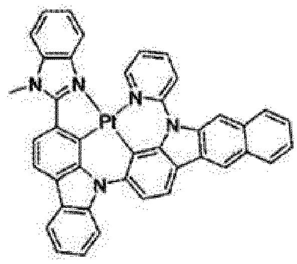
P 1 1 5

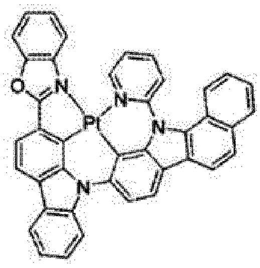
P 1 1 6

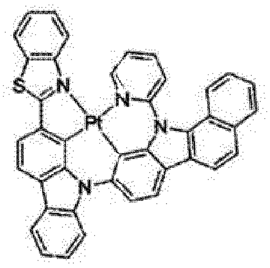
P 1 1 7

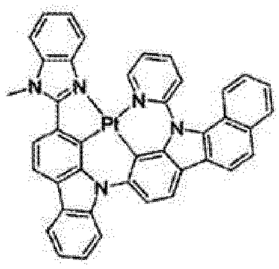
P 1 1 8

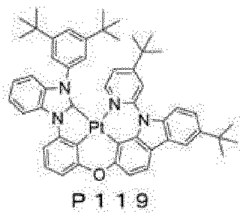
P 1 1 9

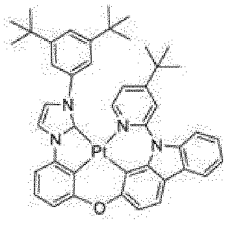
P 1 2 0

An amount of the phosphorescent complex may be, but is not particularly limited to, 0.1 wt% or more, for example, 0.2 wt% or more, based on the total weight of the material for an organic light-emitting device (in particular, the material for an emission layer). In addition, the amount may be 0.5 wt% or more, for example, 1 wt% or more. In addition, the amount may be 3 wt% or more, for example, 5 wt% or more. When the amount is within the ranges above, an organic light-emitting device with high luminescence color purity and high luminescence efficiency may be obtained. In addition, an amount of the phosphorescent complex may be, but is not particularly limited to, 50 wt% or less based on the total weight of the

material for an organic light-emitting device (in particular, the material for an emission layer). In addition, the amount may be 40 wt% or less, for example, 30 wt% or less. When the amount is within the ranges above, an organic light-emitting device with high luminescence color purity and high luminescence efficiency may be obtained. An amount of the phosphorescent complex based on the total weight of an emission layer of an organic light-emitting device to be described below may be the same as described above.

When the material for an organic light-emitting device (in particular, the material for an emission layer) includes the phosphorescent complex, an amount of the phosphorescent complex may be 100 parts by weight or more based on 100 parts by weight of the blue luminescent compound. In addition, the amount may be 150 parts by weight or more, for example, 200 parts by weight or more, based on 100 parts by weight of the blue luminescent compound. When the amount is within the ranges above, an organic light-emitting device with high luminescence color purity and high luminescence efficiency may be obtained. In addition, an amount of the phosphorescent complex may be, but is not particularly limited to, 10,000 parts by weight or less based on 100 parts by weight of the blue luminescent compound. In addition, the amount may be 7,500 parts by weight or less, for example, 5,000 parts by weight or less, based on 100 parts by weight of the blue luminescent compound. When the amount is within the ranges above, an organic light-emitting device with high luminescence color purity and high luminescence efficiency may be obtained. An amount (parts by weight) of the phosphorescent complex based on 100 parts by weight of the blue luminescent compound in an emission layer of an organic light-emitting device to be described below may be the same as described above.

Host material

The material for an organic light-emitting device according to an embodiment may further include a host material in addition to the blue luminescent compound. By using the blue luminescent compound as a dopant material in combination with the host material, high luminescence efficiency and device lifespan may be realized in an organic light-emitting device.

The host material may be, but is not particularly limited to, a known host material. Examples of the host material may include compounds other than the compound represented by Formula (1), such as a compound having a carbazole ring structure, a compound having a ring structure in which at least one ring-forming carbon atom of a carbazole ring is substituted with a nitrogen atom, a compound having a triazine ring structure, or a compound having an anthracene ring structure. By using such compounds as host materials, efficient energy transfer in an emission layer may be promoted. In addition, the balance of carrier mobility between electrons and holes may be improved. In the carbazole ring structure, a ring structure in which at least one ring-forming carbon atom of a carbazole ring is substituted with a nitrogen atom, the triazine ring structure, or the anthracene ring structure of the compounds above, hydrogen atoms bonded to ring-forming atoms constituting such ring structures may be substituted with other atoms or substituents. In addition, two or more of such substituents may constitute a ring structure.

The host material may include a compound having a triphenylsilyl group in view of improving the luminescence efficiency and lifespan of a device.

The compound having the carbazole ring structure or the compound having the ring structure in which at least one ring-forming carbon atom of a carbazole ring is substituted with a nitrogen atom may be, but is not particularly limited to, a compound having a structure represented by Formula (5).

Formula (5)

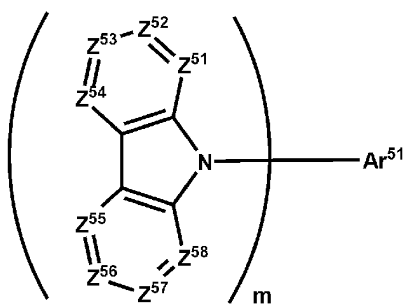

In Formula (5),

$Z^{51}$ may be CH, $CR^{51}$, or N,
$Z^{52}$ may be CH, $CR^{52}$, or N,
$Z^{53}$ may be CH, $CR^{53}$, or N,
$Z^{54}$ may be CH, $CR^{54}$, or N,

$Z^{55}$ may be CH, $CR^{55}$, or N,
$Z^{56}$ may be CH, $CR^{56}$, or N,
$Z^{57}$ may be CH, $CR^{57}$, or N,
$Z^{58}$ may be CH, $CR^{58}$, or N,
$R^{51}$ to $R^{58}$ may each independently be a group from (5a) to (5h):

(5a) a cyano group;
(5b) a substituted or unsubstituted alkyl group having 1 to 20 carbon atoms;
(5c) a substituted or unsubstituted alkoxy group having 1 to 20 carbon atoms;
(5d) a substituted or unsubstituted arylamino group having 6 to 20 carbon atoms;
(5e) a substituted or unsubstituted phosphoryl group (a $-POH_2$ group);
(5f) a substituted or unsubstituted silyl group (a $-SiQ^1Q^2Q^3$ group);
(5g) a substituted or unsubstituted aromatic hydrocarbon group; and
(5h) a substituted or unsubstituted heterocyclic group,

$Ar^{51}$ may be a group including at least one of the aromatic hydrocarbon group and the heterocyclic group, and
m may be 1, 2, 3, 4, 5, or 6,
wherein $R^{51}$ and $R^{52}$, $R^{52}$ and $R^{53}$, $R^{53}$ and $R^{54}$, $R^{55}$ and $R^{56}$, $R^{56}$ and $R^{57}$, or $R^{57}$ and $R^{58}$ may optionally form an aliphatic hydrocarbon ring, an aromatic hydrocarbon ring, or a hetero ring, each including bonded carbon atoms, and

wherein $Q^1$, $Q^2$, and $Q^3$ are independently hydrogen, a substituted or unsubstituted alkyl group having 1 to 20 carbon atoms, a substituted or unsubstituted aromatic hydrocarbon group, or a substituted or unsubstituted heterocyclic group.

In (5b), the alkyl group having 1 to 20 carbon atoms is not particularly limited, and may be linear, branched, or cyclic. In particular, the alkyl group may be branched in view of device lifespan and luminescence color purity. The number of carbon atoms in the alkyl group may be 2 or more, for example, 3 or more, or for example, 4 or more, in view of solubility and luminescence color purity. In addition, the number of carbon atoms in the alkyl group may be 10 or less, for example, 8 or less, or for example, 6 or less, in view of device lifespan. The number of carbon atoms in the alkyl group may be 4. Examples of the alkyl group may include, but are not particularly limited to, a methyl group, an ethyl group, an n-propyl group, an isopropyl group, an n-butyl group, an s-butyl group (a sec-butyl group), a t-butyl group (a tert-butyl group), an i-butyl group, a 2-ethyl butyl group, a 3,3-dimethylbutyl group, an n-pentyl group, an i-pentyl group, a neopentyl group, a t-pentyl group, a cyclopentyl group, a 1-methylpentyl group, a 3-methylpentyl group, a 2-ethylpentyl group, a 4-methyl-2-pentyl group, an n-hexyl group, a 1-methylhexyl group, a 2-ethylhexyl group, a 2-butylhexyl group, a cyclohexyl group, a 4-methylcyclohexyl group, a 4-t-butylcyclohexyl group, an n-heptyl group, a 1-methylheptyl group, a 2,2-dimethylheptyl group, a 2-ethylheptyl group, a 2-butylheptyl group, an n-octyl group, a t-octyl group, a 2-ethyloctyl group, a 2-butyloctyl group, a 2-hexyloctyl group, a 3,7-dimethyloctyl group, a cyclooctyl group, an n-nonyl group, an n-decyl group, an adamantyl group, a 2-ethyldecyl group, a 2-butyldecyl group, a 2-hexyldecyl group, a 2-octyldecyl group, an n-undecyl group, an n-dodecyl group, a 2-ethyldodecyl group, a 2-butyldodecyl group, a 2-hexyldodecyl group, a 2-octyldecyl group, an n-tridecyl group, an n-tetradecyl group, an n-pentadecyl group, an n-hexadecyl group, a 2-ethylhexadecyl group, a 2-butylhexadecyl group, an n-heptadecyl group, an n-octadecyl group, an n-nonadecyl group, an n-eicosyl group, and the like. In particular, the alkyl group may be a branched alkyl group, an isopropyl group, or a tert-butyl group, for example, a tert-butyl group.

The term "substituted alkyl group having 1 to 20 carbon atoms" refers to a group formed by substituting an unsubstituted alkyl group having 1 to 20 carbon atoms with a substituent. Accordingly, the number of carbon atoms in the substituted alkyl group may be greater than 20.

In (5c), the alkoxy group having 1 to 20 carbon atoms is not particularly limited, and the alkoxy group may be linear, branched, or cyclic. In particular, the alkoxy group may be linear in view of device lifespan. The number of carbon atoms in the alkoxy group may be 1 to 10 in view of device lifespan. The number of carbon atoms in the alkoxy group may be 1 to 8, for example, 1 to 6, or for example, 1. Examples of an alkyl group constituting the alkoxy group may include, but are not particularly limited to, those mentioned in the above description of the alkyl group. Examples of the alkoxy group may include, but are not particularly limited to, a methoxy group, an ethoxy group, an n-propoxy group, an isopropoxy group, a butoxy group, a pentyloxy group, a hexyloxy group, an octyloxy group, a nonyloxy group, a decyloxy group, and the like. In particular, the alkoxy group may be a methoxy group.

The term "substituted alkoxy group having 1 to 20 carbon atoms" refers to a group formed by substituting an unsubstituted alkoxy group having 1 to 20 carbon atoms with a substituent. Accordingly, the number of carbon atoms in the substituted alkoxy group may be greater than 20.

In (5d), a nitrogen atom of the arylamino group having 6 to 20 carbon atoms may be bonded to a ring-forming carbon atom in Formula (5) via a single bond. Herein, even in a case where a group includes a nitrogen atom, when the nitrogen atom is a ring-forming atom of a hetero ring, the group is handled as a heterocyclic group to be described below,

instead of an arylamino group. Examples of an aryl group constituting the arylamino group may include, but are not particularly limited to, a group having 6 to 20 carbon atoms among aromatic hydrocarbon groups to be described below. The arylamino group may be, but is not particularly limited to, a monoaryl amino group or a diarylamino group. Examples of the arylamino group may include, but are not particularly limited to, a N-phenylamino group, a N-biphenylamino group, a N-terphenylamino group, a N,N-diphenylamino group, a N-biphenyl-N-phenylamino group, and the like.

The term "substituted arylamino group having 6 to 20 carbon atoms" refers to a group formed by substituting an unsubstituted arylamino group having 6 to 20 carbon atoms with a substituent. Accordingly, the number of carbon atoms in the substituted arylamino group may be greater than 20.

In (5g) and $Ar^{51}$, the aromatic hydrocarbon group refers to a group derived from at least one aromatic hydrocarbon ring. As used herein, the term "aromatic hydrocarbon ring" refers to a hydrocarbon ring that is partially or entirely aromatic.

When the aromatic hydrocarbon group includes two or more aromatic hydrocarbon rings, the rings may be bonded via a single bond or condensed to each other. In addition, when the aromatic hydrocarbon group includes two or more aromatic hydrocarbon rings, one atom may serve as a ring-forming atom of any of the rings.

The number of carbon atoms in the aromatic hydrocarbon group may be, but is not particularly limited to, 6 to 30 in view of luminescence color purity. The number of carbon atoms in the aromatic hydrocarbon group may be 6 to 20, for example, 6 to 12, or for example, 6.

Examples of the aromatic hydrocarbon group may include, but are not particularly limited to, a phenyl group, a mesityl group, a t-butylphenyl group, a bis(t-butyl)phenyl group, a biphenyl group, a terphenyl group, a naphthyl group, a fluorenyl group, an anthracenyl group, a terphenyl group, a quarterphenyl group, a quinquephenyl group, a sexiphenyl group, a triphenylenyl group, a pyranyl group, a benzofluorenyl group, a chrysenyl group, and a combination thereof.

The term "substituted aromatic hydrocarbon group" refers to a group formed by substituting an unsubstituted aromatic hydrocarbon group with a substituent. Accordingly, when the number of carbon atoms in the aromatic hydrocarbon group is equal to or less than a certain upper limit of the number of carbon atoms, for example, 30 or less, the number of carbon atoms in the substituted aromatic hydrocarbon group may be greater than the upper limit.

In (5h) and $Ar^{51}$, the heterocyclic group refers to a group derived from at least one hetero ring. The heterocyclic group may be, but is not particularly limited to, an aromatic heterocyclic group or a non-aromatic heterocyclic group. In particular, the heterocyclic group may be an aromatic heterocyclic group in view of luminescence color purity.

The term "aromatic heterocyclic group" refers to a group derived from at least one aromatic hetero ring. As used herein, the term "aromatic hetero ring" refers to a heterocyclic ring that is partially or entirely aromatic. When the aromatic hetero ring is partially aromatic, aromaticity may be derived from a heterocyclic part of the ring or from a hydrocarbon ring part of the ring. The aromatic hetero ring is not particularly limited, and may be, for example, a ring having at least one hetero atom (e.g., a nitrogen atom (N), an oxygen atom (O), a phosphorus atom (P), a sulfur atom (S), or a silicon atom (Si)) as a ring-forming atom, wherein the remaining ring-forming atoms are carbon atoms (C). "Nitrogen-containing aromatic heterocyclic skeleton", as used herein, refers to a core of a compound such that the core includes an aromatic hetero ring having at least one nitrogen atom. An atom constituting a ring structure may be bonded to an exocyclic atom via a double bond. For example, a carbon atom constituting the ring structure may constitute a ketone group (C=O group), a thioketone group (C=S group), or a C=NH group, or a sulfur atom constituting the ring structure may constitute a sulfinyl group (S=O group) or a sulfonyl group (S(=O)=O group). In this case, as used herein, the exocyclic atom forming the double bond with the atom constituting the ring structure may be part of the aromatic hetero ring. In addition, when the exocyclic atom forming the double bond is bonded to a hydrogen atom via a single bond, the hydrogen atom may also be part of the aromatic hetero ring. Examples of the aromatic hetero ring may include, but are not particularly limited to, a pyridine ring, a pyrazine ring, a pyridazine ring, a pyrimidine ring, a triazine ring, a quinoline ring, an isoquinoline ring, a quinoxaline ring, a quinazoline ring, a naphthyridine ring, an acridine ring, a phenazine ring, a benzoquinoline ring, a benzoisoquinoline ring, a phenanthridine ring, a phenanthroline ring, a benzoquinone ring, a coumarin ring, an anthraquinone ring, a fluorenone ring, a furan ring, a thiophene ring, a benzofuran ring, a benzothiophene ring, a dibenzofuran ring, a dibenzothiophene ring, a pyrrole ring, an indole ring, a carbazole ring, an indolecarbazole ring, an imidazole ring, a benzimidazole ring, a pyrazole ring, an indazole ring, an oxazole ring, an isoxazole ring, a benzoxazole ring, a benzoisoxazole ring, a thiazole ring, an isothiazole ring, a benzothiazole ring, a benzoisothiazole ring, an imidazolinone ring, a benzimidazolinone ring, an imidazopyridine ring, an imidazopyrimidine ring, an imidazophenanthridine ring, a benzimidazophenanthridine ring, an azadibenzofuran ring, an azacarbazole ring, an azadibenzothiophene ring, a diazadibenzofuran ring, a diazacarbazole ring, a diazadibenzothiophene ring, a xanthone ring, a thioxanthone ring, and the like.

When the aromatic heterocyclic group includes two or more aromatic hetero rings, the rings may be bonded via a single bond or condensed to each other. In addition, when the aromatic heterocyclic group includes two or more aromatic hetero rings, one atom may serve as a ring-forming atom of any of the rings.

The number of ring-forming atoms in the aromatic heterocyclic group (the sum of the number of ring-forming carbon atoms and the number of ring-forming hetero atoms) may be, but is not particularly limited to, 3 to 30, in view of the peak wavelength of the emission spectrum and luminescence color purity. The number of ring-forming atoms in the

aromatic heterocyclic group may be 5 to 20, for example, 6 to 14. The number of ring-forming hetero atoms in the aromatic heterocyclic group may be, but is not particularly limited to, 1 to 10, in view of the peak wavelength of the emission spectrum and luminescence color purity. The number of ring-forming hetero atoms in the aromatic heterocyclic group may be 1 to 5, for example, 1 to 3. As described above, the term "ring-forming atom" refers to an atom that directly forms a ring structure. In this regard, when there is an exocyclic atom forming a double bond with an atom constituting a ring structure, the exocyclic atom may not be included in the ring-forming atom.

Examples of the aromatic heterocyclic group may include, but are not particularly limited to, a thienyl group, a furanyl group, a pyrrolyl group, an imidazolyl group, a thiazolyl group, an oxazolyl group, an oxadiazolyl group, a triazolyl group, a pyridyl group, a bipyridyl group, a pyrimidyl group, a triazinyl group, an acridinyl group, a pyridazinyl group, a pyrazinyl group, a quinolinyl group, a quinazolinyl group, a quinoxalinyl group, a phenoxazinyl group, a phthalazinyl group, a pyridpyrimidinyl group, a pyridpyrazinyl group, a pyrazinopyrazinyl group, an isoquinolinyl group, an indolyl group, a carbazolyl group, a benzoxazolyl group, a benzimidazolyl group, a benzothiazolyl group, a benzocarbazolyl group, a benzothiophenyl group, a dibenzothiophenyl group, a thienothienyl group, a benzofuranyl group, a phenanthrolinyl group, a thiazolyl group, an isoxazolyl group, an oxadiazolyl group, a thiadiazolyl group, a phenothiazinyl group, a dibenzosilolyl group, a dibenzofuranyl group, a xanthonyl group, and the like.

In addition, the term "non-aromatic heterocyclic group" refers to a group derived from at least one non-aromatic hetero ring. As used herein, the term "non-aromatic hetero ring" refers to a heterocyclic ring that is entirely non-aromatic. The non-aromatic hetero ring is not particularly limited, and may be, for example, a ring having at least one hetero atom (e.g., a nitrogen atom (N), an oxygen atom (O), a phosphorus atom (P), a sulfur atom (S), or a silicon atom (Si)) as a ring-forming atom, wherein the remaining ring-forming atoms are carbon atoms (C). The hetero atom may be a nitrogen atom (N) or an oxygen atom (O), in view of the peak wavelength of the emission spectrum and luminescence color purity. An atom constituting a ring structure may be bonded to an exocyclic atom via a double bond. For example, a carbon atom constituting the ring structure may constitute a ketone group (C=O group), a thioketone group (C=S group), or a C=NH group, or a sulfur atom constituting the ring structure may constitute a sulfinyl group (S=O group) or a sulfonyl group (S(=O)=O group). In this case, as used herein, the exocyclic atom forming the double bond with the atom constituting the ring structure may be part of the non-aromatic hetero ring. In addition, when the exocyclic atom forming the double bond is bonded to a hydrogen atom via a single bond, the hydrogen atom may also be part of the non-aromatic hetero ring. Examples of the non-aromatic hetero ring may include, but are not particularly limited to, a pyrrolidine ring, tetrahydrofuran ring, a tetrahydrothiophene ring, a piperidine ring, a tetrahydropyran ring, a tetrahydrothiopyran ring, a dioxane ring, a morpholine ring, a dioxolane ring, and the like.

When the non-aromatic heterocyclic group includes two or more non-aromatic hetero rings, the rings may be bonded via a single bond or condensed to each other. In addition, when the non-aromatic heterocyclic group includes two or more non-aromatic hetero rings, one atom may serve as a ring-forming atom of any of the rings.

The number of ring-forming atoms in the non-aromatic heterocyclic group (the sum of the number of ring-forming carbon atoms and the number of ring-forming hetero atoms) may be, but is not particularly limited to, 3 to 30, in view of the peak wavelength of the emission spectrum and luminescence color purity. The number of ring-forming atoms in the non-aromatic heterocyclic group may be 5 to 20, for example, 6 to 14. The number of ring-forming hetero atoms in the non-aromatic heterocyclic group may be, but is not particularly limited to, 1 to 10, in view of the peak wavelength of the emission spectrum and luminescence color purity. The number of ring-forming hetero atoms in the non-aromatic heterocyclic group may be 1 to 5, for example, 1 to 3. As described above, the term "ring-forming atom" refers to an atom that directly forms a ring structure. In this regard, when there is an exocyclic atom forming a double bond with an atom constituting a ring structure, the exocyclic atom may not be included in the ring-forming atom.

Examples of the non-aromatic heterocyclic group may include, but are not particularly limited to, a pyrrolidinyl group, a tetrahydrofuranyl group, a tetrahydrothienyl group, a piperidinyl group, a tetrahydropyranyl group, a tetrahydrothiopyranyl group, a dioxanyl group, a morphonyl group, a dioxolanyl group, and the like.

The term "substituted monovalent heterocyclic group" refers to a group formed by substituting an unsubstituted monovalent heterocyclic group with a substituent. Accordingly, when the number of ring-forming atoms in the monovalent heterocyclic group is equal to or less than a certain upper limit of the number of ring-forming atoms, for example, 30 or less, the number of ring-forming atoms in the substituted monovalent heterocyclic group may be greater than the upper limit.

In Formula (5), when the groups of (5b) to (5h) are substituted groups, substituents substituting such groups are not particularly limited. For example, the substituents may be the unsubstituted groups of (5a) to (5h) or an alkenyl group having 2 to 30 carbon atoms.

In Formula (5), $Ar^{51}$ may be, but is not particularly limited to, a group having at least one of the aromatic hydrocarbon group and the heterocyclic group. Examples of $Ar^{51}$ may include, for example, a substituted or unsubstituted aromatic hydrocarbon group, a substituted or unsubstituted heterocyclic group, a group in which at least one substituted or unsubstituted aromatic hydrocarbon group is bonded to at least one substituted or unsubstituted heterocyclic group via a single bond, or a group in which at least two substituted or unsubstituted aromatic hydrocarbon groups or substituted or unsubstituted heterocyclic groups are bonded to each other via a linking group other than the at least two groups.

In this regard, in the group in which at least two substituted or unsubstituted aromatic hydrocarbon groups or substituted or unsubstituted heterocyclic groups are bonded to each other via a linking group other than the at least two groups, the linking group is not particularly limited. Examples of the linking group may include a Si group, a N group, a P=O group, a S(=O)=O group, a C=O group, and the like.

In Formula (5), when groups constituting $Ar^{51}$ are substituted groups, substituents substituting such groups are not particularly limited. For example, the substituents may be the groups of (5a) to (5h). Examples of the substituents substituting such groups may include, but are not particularly limited to, a cyano group, an unsubstituted alkoxy group having 1 to 20 carbon atoms, a monovalent heterocyclic group having 3 to 30 ring-forming atoms substituted with an unsubstituted alkoxy group having 1 to 20 carbon atoms, and the like.

The alkenyl group having 2 to 30 carbon atoms in the substituents of the groups of (5b) to (5h) or the substituents of the groups constituting $Ar^{51}$ is not particularly limited, and may be linear, branched, or cyclic. Examples of the alkenyl group may include, but are not particularly limited to, a vinyl group, a 2-prophenyl group, a 2-butenyl group, a 3-butenyl group, a 1-methyl-2-prophenyl group, a 2-methyl-2-prophenyl group, a 2-pentenyl group, a 3-pentenyl group, a 4-pentenyl group, a 1-methyl-2-butenyl group, a 2-methyl-2-butenyl group, a 3-methyl-2-butenyl group, a 1-methyl-3-butenyl group, a 2-methyl-3-butenyl group, a 3-methyl-3-butenyl group, a 1,1-dimethyl-2-prophenyl group, a 1,2-dimethyl-2-prophenyl group, a 1-ethyl-2-prophenyl group, and the like.

In Formula (5), m may be 1, 2, 3, or 4, for example, 2.

The compound having the carbazole ring structure may be a compound containing a substituted silyl group (a compound having a carbazole ring structure having a substituted silyl group), or may be a compound having a triphenylsilyl group (a compound having a carbazole ring structure having a triphenylsilyl group).

Hereinafter, as the host material according to an embodiment, the compound having the carbazole ring structure and the compound having the ring structure in which at least one ring-forming carbon atom of a carbazole ring is substituted with a nitrogen atom will be described in detail. However, the disclosure is not limited to such examples.

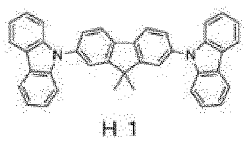
H 1

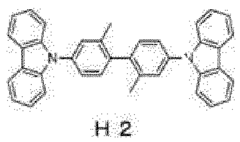
H 2

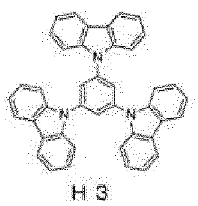
H 3

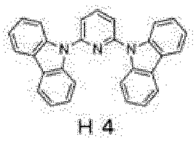
H 4

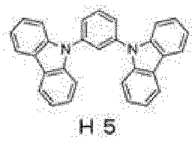
H 5

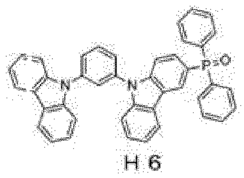
H 6

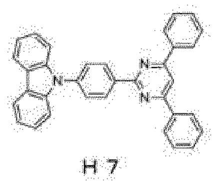
H 7

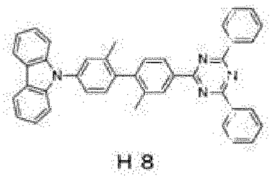
H 8

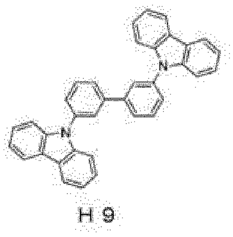
H 9

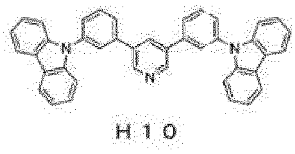
H 1 0

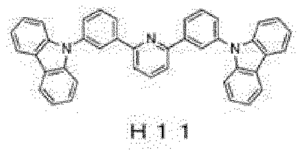
H 1 1

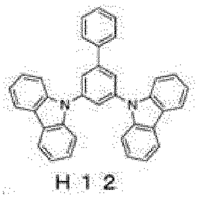
H12

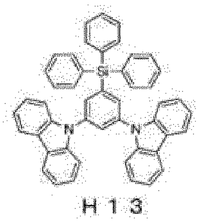
H13

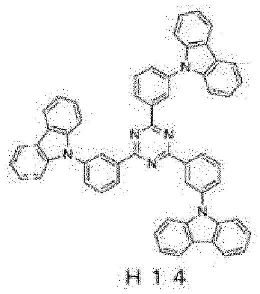
H14

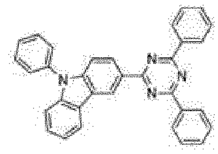
H15

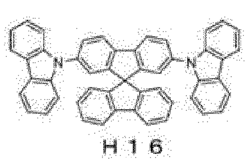
H16

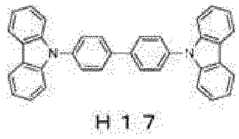
H17

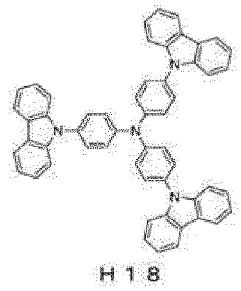
H18

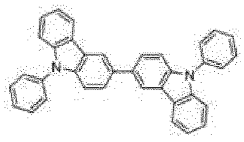
H19

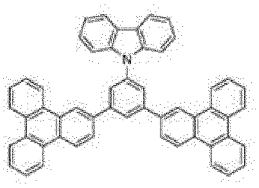
H20

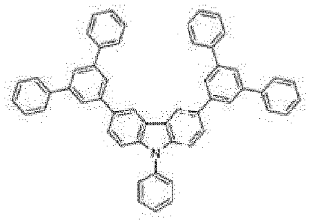
H21

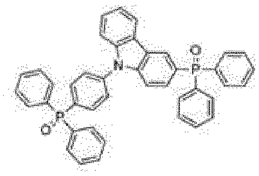
H22

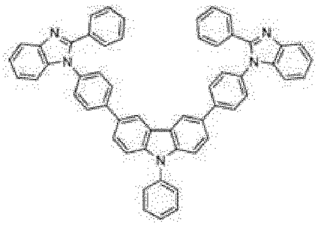
H23

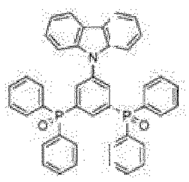
H24

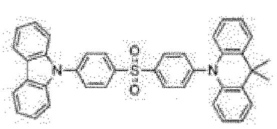
H25

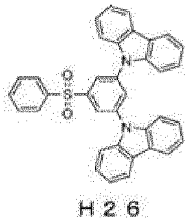

H26

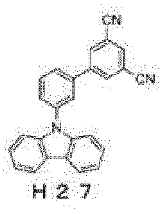

H27

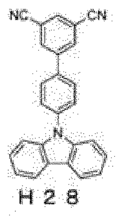

H28

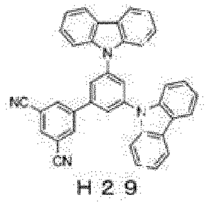

H29

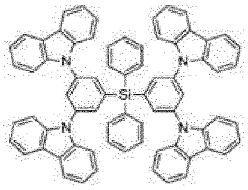

H30

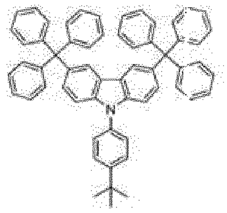

H31

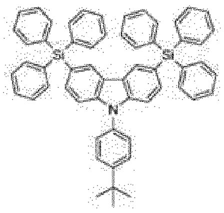

H32

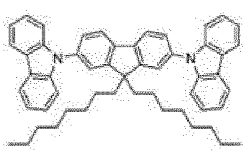

H33

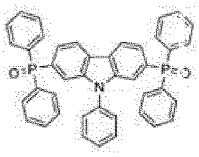

H34

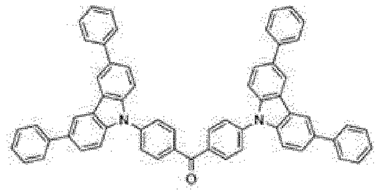

H35

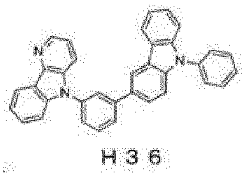

H36

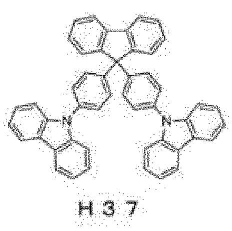

H37

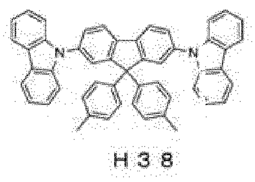

H38

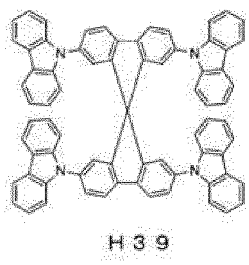

H39

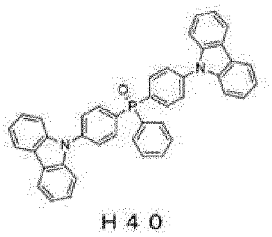

Ｈ４０

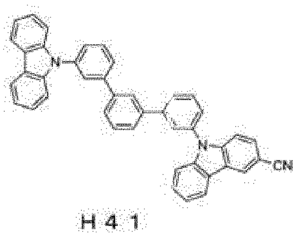

Ｈ４１

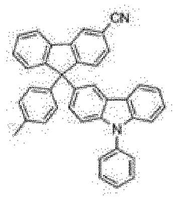

Ｈ４２

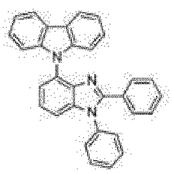

Ｈ４３

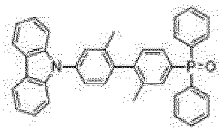

Ｈ４４

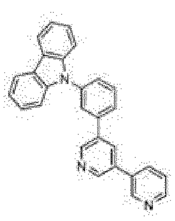

Ｈ４５

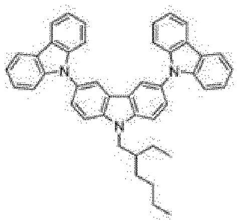

Ｈ４６

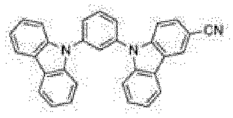

Ｈ４７

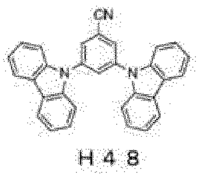

Ｈ４８

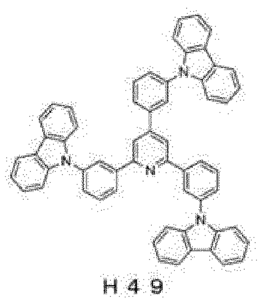

Ｈ４９

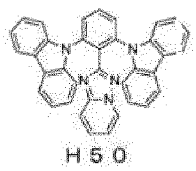

Ｈ５０

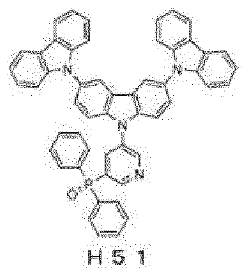

Ｈ５１

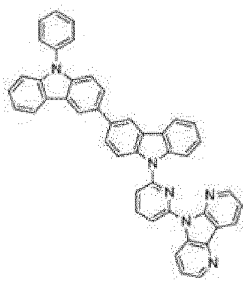
H52

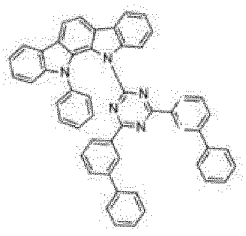
H53

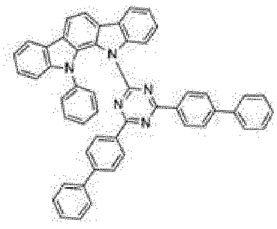
H54

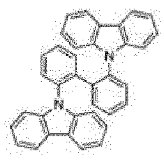
H55

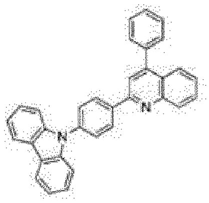
H56

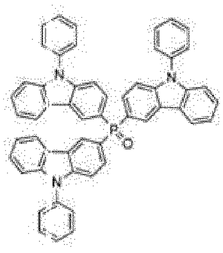
H57

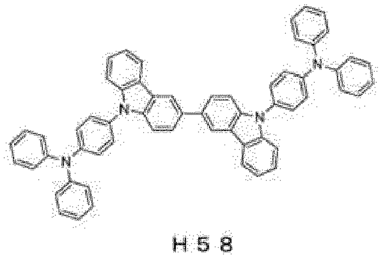
H58

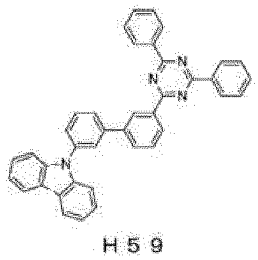
H59

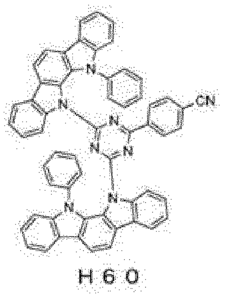
H60

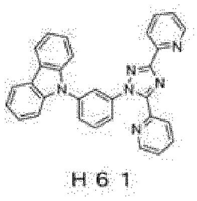
H61

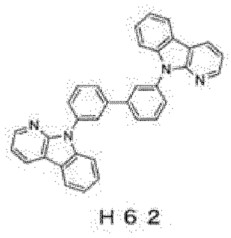
H62

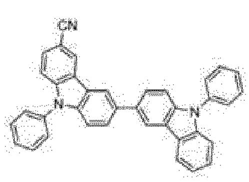
H63

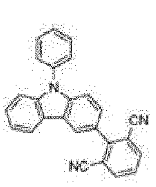
H64

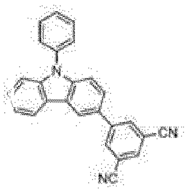
H65

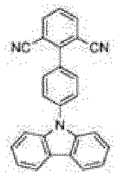
Ｈ６６

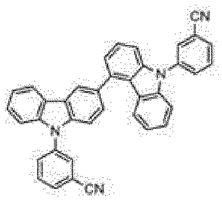
Ｈ６７

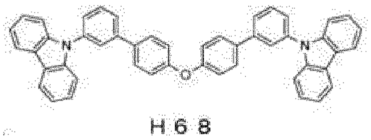
Ｈ６８

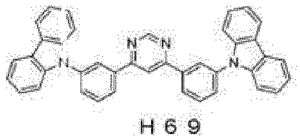
Ｈ６９

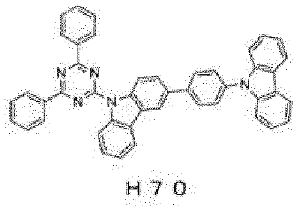
Ｈ７０

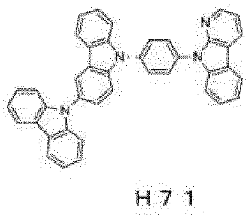
Ｈ７１

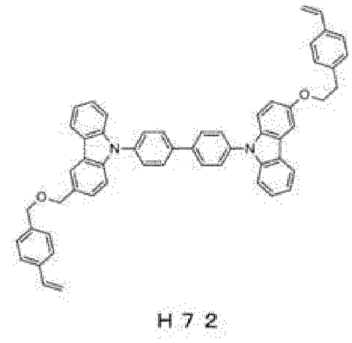
Ｈ７２

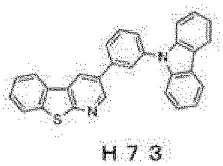
Ｈ７３

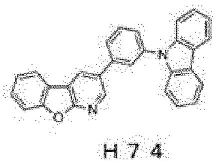
Ｈ７４

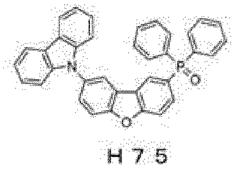
Ｈ７５

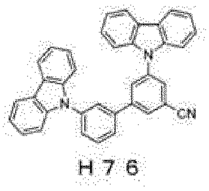
Ｈ７６

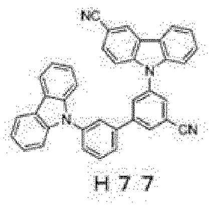
Ｈ７７

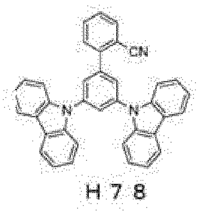
Ｈ７８

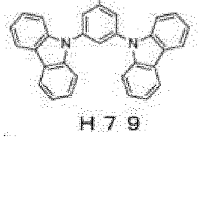
Ｈ７９

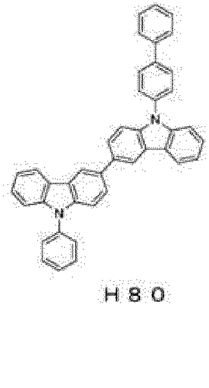
Ｈ８０

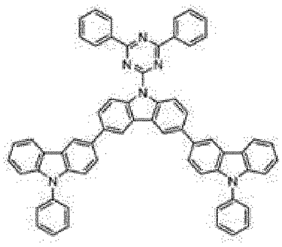

H81

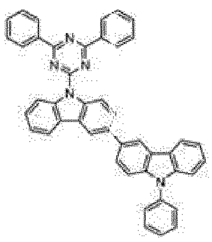

H82

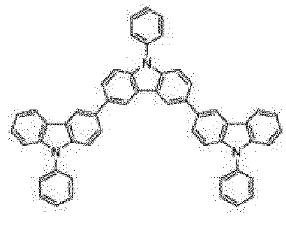

H83

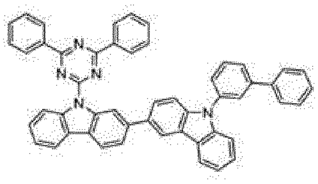

H84

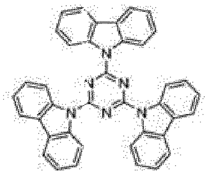

H85

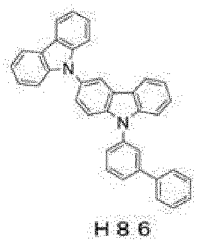

H86

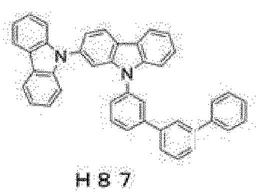

H87

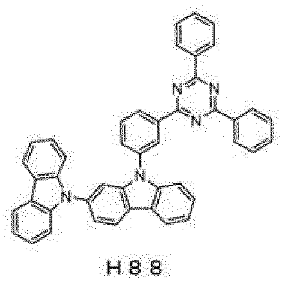

H88

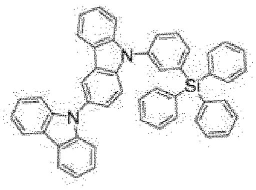

H89

The material for an organic light-emitting device according to an embodiment may include the blue luminescent compound, the phosphorescent complex, and the host material, wherein the host material may include the compound having the structure represented by Formula (5). In addition, an organic light-emitting device according to an embodiment, which will be described below, may include the fluorescence emitter or the blue luminescent compound and the host material, wherein the host material may include a compound having a structure represented by Formula (5) and a triphenylsilyl group as a substituent.

The compound having the triazine ring structure may be, but is not particularly limited to, a compound having a structure represented by Formula (6).

Formula (6)

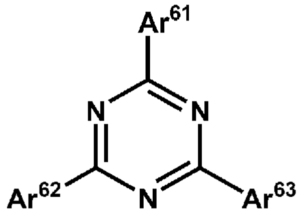

In Formula (6),

$Ar^{61}$ to $Ar^{63}$ may each independently be a substituted or unsubstituted aromatic hydrocarbon group or a substituted or

unsubstituted heterocyclic group.

In Formula (6), the aromatic hydrocarbon group and the heterocyclic group may be the same as described above.

In Formula (6), substituents substituting the aromatic hydrocarbon group or the heterocyclic group may be, but are not particularly limited to, those mentioned above as substituents substituting the groups of (5b) to (5h) in Formula (5).

The compound having the triazine ring structure may be a compound containing a substituted silyl group (a compound having a triazine ring structure having a substituted silyl group), or may be a compound containing a triphenylsilyl group (a compound having a triazine ring structure having a triphenylsilyl group). The compound having the triazine ring structure may be a compound containing a carbazole group (a compound having a triazine ring structure having a carbazole group).

The compound having the triazine ring structure may be used in combination with the compound having the carbazole ring structure or the compound having the ring structure in which at least one ring-forming carbon atom of a carbazole ring is substituted with a nitrogen atom.

Hereinafter, the compound having the triazine structure, which is the host material according to an embodiment, will be described in detail. However, the disclosure is not limited to such examples.

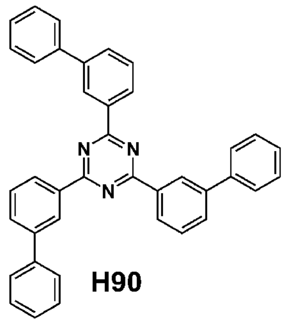

H90

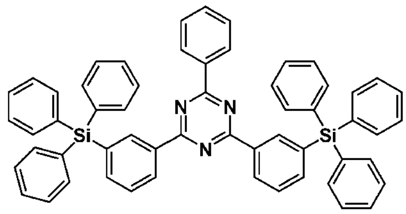

H91

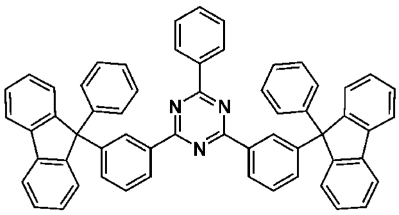

H92

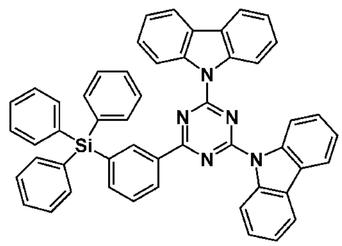

H93

The material for an organic light-emitting device according to an embodiment may include the blue luminescent compound, the phosphorescent complex, and the host material, wherein the host material may include a compound having a structure represented by Formula (6). In addition, the material for an organic light-emitting device according to another embodiment may include the blue luminescent compound, the phosphorescent complex, and the host material, wherein the host material may include a compound having a triphenylsilyl group. Furthermore, the material for an organic light-emitting device according to another embodiment may include the blue luminescent compound, the phosphorescent complex, and the host material, wherein the host material may include the compound having the structure represented by Formula (5) and the compound having the structure represented by Formula (6). Furthermore, the material for an organic light-emitting device according to another embodiment may include the blue luminescent compound, the phosphorescent complex, and the host material, wherein the host material may include a compound having the structure represented by Formula (5) and a triphenylsilyl group as a substituent, and a compound having the structure represented by Formula (6) and a triphenylsilyl group as a substituent.

In addition, an organic light-emitting device according to an embodiment, which will be described below, may include the blue luminescent compound, the phosphorescent complex, and the host material, wherein the host material may include the compound having the structure represented by Formula (6). The organic light-emitting device according to another embodiment may include the blue luminescent compound, the phosphorescent complex, and the host material, wherein the host material may include the compound having the structure represented by Formula (5) and the compound having the structure represented by Formula (6). The organic light-emitting device according to another embodiment may include the blue luminescent compound, the phosphorescent complex, and the host material, wherein the host material may include a compound having the structure represented by Formula (5) and a triphenylsilyl group as a substituent, and a compound having the structure represented by Formula (6) and a triphenylsilyl group as a substituent.

Furthermore, an emission layer may include a known host material. The emission layer may include, for example, at least one of bis[2-(diphenylphosphino)phenyl]etheroxide (DPEPO), 4,4'-bis(carbazol-9-yl)biphenyl (CBP), 3,3'-bis(carbazol-9-yl)biphenyl (mCBP), 1,3-bis(carbazol-9-yl)benzene (mCP), 2,8-bis(diphenylphosphoryl)dibenzo[b,d]furan (PPF), 4,4',4"-tris(carbazol-9-yl)triphenylamine (TcTa), and 1,3,5-tris(N-phenylbenzimidazol-2-yl)benzene (TPBi). However, embodiments are not limited thereto, and the emission layer may include, for example, tris(8-hydroxyquinolino)aluminum ($Alq_3$), poly(n-vinylcarbazole) (PVK), 9,10-di(naphthalen-2-yl)anthracene (ADN), 4,4',4"-tris(carbazol-9-yl)-triphenylamine (TCTA), 1,3,5-tris(N-phenylbenzimidazol-2-yl)benzene (TPBi), 3-tert-butyl-9,10-di(naphtho-2-yl)anthracene (TBADN), distyrylarylene (DSA), 4,4'-bis(9-carbazolyl)-2,2'-dimethyl-biphenyl (CDBP), 2-methyl-9,10-bis(naphthalen-2-yl)anthracene (MADN), hexaphenylcyclotriphosphazene (CP1), 1,4-bis(triphenylsilyl)benzene (UGH2), hexaphenylcyclotrisiloxane ($DPSiO_3$), octaphenylcyclotetrasiloxane ($DPSiO_4$), 2,8-bis(diphenylphosphoryl) dibenzofuran (PPF), or the like.

In the disclosure, as described above, the compound having the anthracene ring structure may be used as a host material. That is, the host material may be 9,10-di(naphthalen-2-yl)anthracene (ADN), 3-tert-butyl-9,10-di(naphtho-2-yl) anthracene (TBADN), or 2-methyl-9,10-bis(naphthalen-2-yl)anthracene (MADN) among the known host materials above.

When the emission layer includes the phosphorescent complex, the emission layer may not include the compound having the anthracene ring structure.

In addition, the emission layer may include a known dopant material. The emission layer may include, for example, a styryl derivative (e.g., 1,4-bis[2-(3-N-ethylcarbazolyl)vinyl]benzene (BCzVB), 4-(di-p-tolylamino)-4'-[(di-p)-tolylamino)styryl]stilbene (DPAVB), or N-(4-((E)-2-(6-((E)-4-(diphenylamino)styryl)naphthalen-2-yl)vinyl)phenyl)-N -phenylbenzeneamine (N-BDAVBi)), perylene or a derivative thereof (e.g., 2,5,8,11-tetra-tert-butylperylene (TBP)), pyrene or a derivative thereof (e.g., 1,1-dipyrene, 1,4-dipyrenylbenzene, 1,4-bis(N,N-diphenylamino)pyrene, or 2,5,8,11-tetra-tert-butylperylene (TBP)), or the like.

When the material for an organic light-emitting device is a material for an emission layer, an amount of the host material may be, but is not particularly limited to, 50 wt% or more based on the total weight of the material for an emission layer. In addition, the amount may be 70 wt% or more, for example, 75 wt% or more, for example, 90 wt% or more, for example, 95 wt% or more, or for example, 98 wt% or more. In addition, an amount of the host material may be, but is not particularly limited to, 99.95 wt% or less, for example, 99.9 wt% or less, or for example, 99.8 wt% or less, based on the total weight of the material for an organic light-emitting device (in particular, the material for an emission layer). When the amount is within the ranges above, an organic light-emitting device with high luminescence color purity and high luminescence efficiency may be obtained.

When the material for an organic light-emitting device (in particular, the material for an emission layer) includes the host material, an amount of the host material may be 1,000 parts by weight or more based on 100 parts by weight of the blue luminescent compound. In addition, the amount may be 2,000 parts by weight or more, for example, 3,000 parts by weight or more, based on 100 parts by weight of the blue luminescent compound. When the amount is within the ranges above, an organic light-emitting device with high luminescence color purity and high luminescence efficiency may be obtained. In addition, an amount of the host material may be, but is not particularly limited to, 200,000 parts by weight or less based on 100 parts by weight of the blue luminescent compound. In addition, the amount may be 150,000 parts by weight or less, for example, 100,000 parts by weight or less, based on 100 parts by weight of the blue luminescent compound. When the amount is within the ranges above, an organic light-emitting device with high luminescence color purity and high luminescence efficiency may be obtained. An amount (parts by weight) of the host material based on 100 parts by weight of the blue luminescent compound in an emission layer of an organic light-emitting device to be described below may be the same as described above.

Liquid composition

Another aspect of the disclosure relates to a liquid composition including the blue luminescent compound, the fluorescence emitter, or the material for an organic light-emitting device and a solvent.

The solvent is not particularly limited, and may be a solvent having a boiling point of about 100 °C to about 350 °C at atmospheric pressure [101.3 kilopascals (kPa), 1 atmosphere (atm)). The boiling point of the solvent at atmospheric pressure may be about 150 °C to about 320 °C, for example, about 180 °C to about 300 °C. When the boiling point of the solvent at atmospheric pressure is within the ranges above, the processability or film-forming capability of a wet film forming method, in particular, an inkjet method, may be improved. The solvent having a boiling point of about 100 °C to about 350 °C at atmospheric pressure is not particularly limited, and a known solvent may be appropriately used. Hereinafter, the solvent having a boiling point of about 100 °C to about 350 °C at atmospheric pressure will be described in detail, but the disclosure is not limited thereto. Examples of a hydrocarbon-based solvent may include octane, nonane, decane, undecane, dodecane, and the like. Examples of an aromatic hydrocarbon-based solvent may include toluene, xylene, ethylbenzene, n-propylbenzene, iso-propylbenzene, mesitylene, n-butylbenzene, sec-butylbenzene, 1-phenylpentane, 2-phenylpentane, 3-phenylpentane, phenylcyclopentane, phenylcyclohexane, 2-ethylbiphenyl, 3-ethylbiphenyl, and the like. Examples of an ether-based solvent may include 1,4-dioxane, 1,2-diethoxyethane, diethyleneglycoldimethylether, diethyleneglycoldiethylether, anisole, ethoxybenzene, 3-methylanisole, m-dimethoxybenzene, and the like. Examples of a ketone-based solvent may include 2-hexanone, 3-hexanone, cyclohexanone, 2-heptanone, 3-heptanone, 4-heptanone, cycloheptanone, and the like. Examples of an ester-based solvent may include butylacetate, butylpropionate, butylbutyrate, propylenecarbonate, methylbenzoate, ethylbenzoate, 1-propylbenzoate, 1-butylbenzoate, and the like. Examples of a nitrile-based solvent may include benzonitrile, 3-methylbenzonitrile, and the like. Examples of an amide-based solvent may include dimethylformamide, dimethylacetamide, N-methylpyrrolidone, and the like. Such solvents may be used alone or in combination of two or more.

In an embodiment, an amount of the blue luminescent compound, the fluorescence emitter, or the material for an organic light-emitting device in the liquid composition is not particularly limited.

In an embodiment, the liquid composition may be used as a coating liquid for forming an organic layer of an organic light-emitting device. In addition, the liquid composition may be used as a coating liquid for forming an emission layer among coating liquids for forming an organic layer.

## Organic light-emitting device

Another aspect of the disclosure relates to an organic light-emitting device including the blue luminescent compound or the fluorescence emitter (the blue luminescent compound and the phosphorescent complex). In addition, the organic light-emitting device may include the blue luminescent compound or the fluorescence emitter and the host material. In this regard, the phosphorescent complex included in the fluorescence emitter may be a platinum complex.

Another aspect of the disclosure relates to an organic light-emitting device including the material for an organic light-emitting device. In addition, the material for an organic light-emitting device in the organic light-emitting device may further include the host material. In addition, the phosphorescent complex included in the organic light-emitting device may be a platinum complex.

The host material included in the organic light-emitting device may be the compound having the carbazole ring structure, the compound having the ring structure in which at least one ring-forming carbon atom of a carbazole ring is substituted with a nitrogen atom, the compound having the triazine ring structure, or the compound having the anthracene ring structure. In addition, the host material included in the organic light-emitting device may include the compound having the structure represented by Formula (5). In addition, the host material included in the organic light-emitting device may include the compound having the structure represented by Formula (6). In addition, the host material included in the organic light-emitting device may include the compound having the structure represented by Formula (5) and the compound having the structure represented by Formula (6). The host material included in the organic light-emitting device may include a compound having the structure represented by Formula (5) and a triphenylsilyl group as a substituent, and a compound having the structure represented by Formula (6) and a triphenylsilyl group as a substituent.

In addition, the phosphorescent complex included in the organic light-emitting device may be the compound having the structure represented by Formula (4).

The organic light-emitting device according to an embodiment may include, but is not particularly limited to, a first electrode, a second electrode, and a single organic layer or a plurality of organic layers. The second electrode may be located on the first electrode.

Herein, when a portion of a layer, film, region, plate, or the like is said to be "on" or "above" another portion, this includes not only a case where the portion is "directly on" the other portion, but also a case where an intervening layer is present therebetween. In contrast, when a portion of a layer, film, region, plate, or the like is said to be "under" or "below" another portion, this includes not only a case where the portion is "directly under" the other portion, but also a case where an intervening layer is present therebetween. Herein, being located "on" includes not only being located on the top surface but also on the lower or bottom surface.

The organic light-emitting device according to an embodiment may include a first electrode, a second electrode, and a single layer or a plurality of layers between the first electrode and the second electrode. In this regard, the layer or layers may include at least one organic layer, and at least one of the organic layer may include the blue luminescent compound, the fluorescence emitter, or the material for an organic light-emitting device. The organic layer including the blue luminescent compound, the fluorescence emitter, or the material for an organic light-emitting device may include an emission layer. Such an organic light-emitting device may provide luminescence with high color purity.

As such, the emission layer may include at least one of the blue luminescent compound.

The emission layer may be a single layer including a single material or a single layer including a plurality of different materials. In addition, the emission layer may have a multi-layer structure with a plurality of layers including a plurality of different materials.

The emission layer is not particularly limited, and may include, for example, a host material and a dopant material. The blue luminescent compound and the fluorescence emitter may be used as a host material or a dopant material, for example, as a dopant material.

In an embodiment, the organic light-emitting device may include an emission layer, wherein the emission layer may include the blue luminescent compound, the fluorescence emitter, or the material for an organic light-emitting device. In detail, in an embodiment, the organic light-emitting device may include an emission layer, wherein the emission layer may include the blue luminescent compound and the phosphorescent complex. In another embodiment, the organic light-emitting device may include an emission layer, wherein the emission layer may include the blue luminescent compound and the phosphorescent complex, and the phosphorescent complex may be a platinum complex.

The emission layer may include the material for an organic light-emitting device. In view of the peak wavelength of the emission spectrum, luminescence color purity, luminescence efficiency, and device lifespan, the material for an organic light-emitting device may include the host material in addition to the blue luminescent compound. The material for an organic light-emitting device included in the emission layer may include the phosphorescent complex and the host material in addition to the blue luminescent compound. In addition, an amount or amount ratio of each of the blue luminescent compound, the phosphorescent complex, and the host material in the emission layer may be in the same range as the amount or amount ratio of the material for an organic light-emitting device.

A thickness of the emission layer may be, but is not particularly limited to, about 1 nm to about 100 nm, for example, about 10 nm to about 50 nm.

Examples of the film forming method of the emission layer may include, but are not particularly limited to, known film forming methods such as vacuum deposition, spin coating, Langmuir-Blodgett (LB) deposition, ink-jet printing, laser-printing, and laser-induced thermal imaging (LITI).

An emission wavelength of the organic light-emitting device is not particularly limited. The emission wavelength of the organic light-emitting device may be, for example, in the same range as the peak wavelength of emission in PL of the blue luminescent compound according to the disclosure. In specifications of currently commercialized products, in the case of blue luminescence, the organic light-emitting device may emit light with a peak in a wavelength region of about 445 nm to about 470 nm, for example, about 450 nm to about 470 nm, or for example, about 450 nm to about 465 nm.

In addition, a FWHM of a peak of an emission spectrum of the organic light-emitting device may be smaller. In addition, the FWHM of the peak of the emission spectrum may be 30 nm or less, for example, 25 nm or less. In addition, the FWHM of the peak of the emission spectrum may be 20 nm or less (the lower limit is greater than 0 nm).

Hereinafter, a case where the organic light-emitting device according to an embodiment further includes an organic layer, in addition to the emission layer, will be described in detail with reference to the accompanying drawings. In the description of the drawings, the same components will be denoted by the same reference numerals, and redundant descriptions thereof will be omitted. In addition, dimensional ratios in the drawings may be exaggerated for convenience of description, and thus may differ from actual ratios.

FIGS. 2 to 4 are each a schematic cross-sectional view illustrating an organic light-emitting device according to an embodiment. However, the structure of the organic light-emitting device according to the disclosure is not limited to those shown in FIGS. 2 to 4.

FIG. 2 is a schematic cross-sectional view illustrating an organic light-emitting device 10 according to an embodiment. The organic light-emitting device 10 according to an embodiment may include a substrate 1, a first electrode 2, a hole transport region 3, an emission layer 4, an electron transport region 5, and a second electrode 6, which are sequentially stacked in this stated order.

FIG. 3 is a schematic cross-sectional view illustrating an organic light-emitting device 10 according to another embodiment. The organic light-emitting device 10 according to an embodiment may include a substrate 1, a first electrode 2, a hole transport region 3, an emission layer 4, an electron transport region 5, and a second electrode 6, which are sequentially stacked in this stated order. In FIG. 3, the hole transport region 3 may include a hole injection layer 31 and a hole transport layer 32, which are sequentially stacked in this stated order. In addition, in FIG. 3, the electron transport region 5 may include an electron transport layer 52 and an electron injection layer 51, which are sequentially stacked in this stated order.

FIG. 4 is a schematic cross-sectional view illustrating an organic light-emitting device 10 according to another embodiment. The organic light-emitting device 10 according to an embodiment may include a substrate 1, a first electrode 2, a hole transport region 3, an emission layer 4, an electron transport region 5, and a second electrode 6, which are sequentially stacked in this stated order. In FIG. 4, the hole transport region 3 may include a hole injection layer 31, a hole transport layer 32, and an electron blocking layer 33, which are sequentially stacked in this stated order. In addition, in FIG. 4, the electron transport region 5 may include a hole blocking layer 53, an electron transport layer 52, and an electron injection layer 51, which are sequentially stacked in this stated order.

Hereinafter, the substrate 1, each region, and each layer will be described in detail.

Substrate 1

The organic light-emitting device 10 may have a substrate 1. As the substrate 1, any substrate used in a general organic light-emitting device may be used. For example, the substrate 1 may be a glass substrate, a semiconductor

substrate such as a silicon substrate, or a transparent plastic substrate.

First electrode 2

The first electrode 2 may have conductivity. In the organic light-emitting device 10 according to an embodiment, the first electrode 2 may be an anode. In addition, the first electrode 2 may be a pixel electrode. In addition, the first electrode 2 may be a reflective electrode, a semi-reflective electrode, or a transmissive electrode.

A material for forming the first electrode 2 is not particularly limited, and may be, for example, a metal, a metal alloy, or a conductive compound. When the first electrode 2 is a transmissive electrode, the first electrode 2 may include a transparent metal oxide, for example, indium tin oxide (ITO), indium zinc oxide (IZO), zinc oxide (ZnO), indium tin zinc oxide (ITZO), or the like. When the first electrode 2 is a semi-transmissive electrode or a reflective electrode, the first electrode 2 may include Ag, Mg, Cu, Al, Pt, Pd, Au, Ni, Nd, Ir, Cr, Li, Ca, LiF/Ca, LiF/Al, Mo, Ti, or a compound or mixture thereof (e.g., a mixture of Ag and Mg).

The first electrode 2 may be a single layer including a single material or a single layer including a plurality of different materials. In addition, the first electrode 2 may have a multi-layer structure with a plurality of layers including a plurality of different materials.

A thickness of the first electrode 2 may be, but is not particularly limited to, about 10 nm to about 1,000 nm, for example, about 50 nm to about 300 nm.

Hole transport region 3

The hole transport region 3 may be provided on the first electrode 2. The hole transport region 3 may include at least one of the hole injection layer 31, the hole transport layer 32, a hole buffer layer (not shown), and the electron blocking layer 33.

The hole transport region 3 may be a single layer including a single material or a single layer including a plurality of different materials. In addition, the hole transport region 3 may have a multi-layer structure with a plurality of layers including a plurality of different materials.

For example, the hole transport region 3 may have a single-layer structure including the hole injection layer 31 or the hole transport layer 32. In addition, for example, the hole transport region 3 may have a single-layer structure including a hole injection material and a hole transport material. In addition, for example, the hole transport region 3 may have a hole injection layer 31/hole transport layer 32 structure, wherein constituting layers are sequentially stacked from the first electrode 2. In addition, for example, the hole transport region 3 may have a hole injection layer 31/hole transport layer 32/hole buffer layer (not shown) structure. In addition, for example, the hole transport region 3 may have a hole injection layer 31/hole buffer layer (not shown) structure, wherein constituting layers are sequentially stacked from the first electrode 2. In addition, for example, the hole transport region 3 may have a hole transport layer 32/hole buffer layer (not shown) structure, wherein constituting layers are sequentially stacked from the first electrode 2. In addition, for example, the hole transport region 3 may have a hole injection layer 31/hole transport layer 32/electron blocking layer 33 structure, wherein constituting layers are sequentially stacked from the first electrode 2. However, the structure of the hole transport region 3 is not limited to the examples above.

The hole injection layer 31 or other layers constituting the hole transport region 3 are not particularly limited, and may include, for example, a known hole injection material. Examples of the hole injection material may include a phthalocyanin compound such as copper phthalocyanin, N,N'-diphenyl-N,N'-bis-[4-phenyl-m-toly-amino)-phenyl]-biphenyl-4,4'-diamine (DNTPD), 4,4',4"-tris(3-methylphenylphenylamino)triphenylamine (m-MTDATA), 4,4',4"-tris(N,N-diphenylamino)triphenylamine (TDATA), 4,4',4"-tris{N,-(2-naphthyl)-N-phenylamino}-triphenylamine (2-TNATA), poly(3,4-ethylenedioxythiophene)/poly(4-styrenesulfonate) (PEDOT/PSS), polyaniline/dodecylbenzenesulfonic acid (PANI/DBSA), polyaniline/camphor sulfonic acid (PANI/CSA), polyaniline/poly(4-styrenesulfonate) (PANI/PSS), N,N'-di(naphthalene-1-yl)-N,N'-diphenyl-benzidine (NPB), polyetherketone including triphenylamine (TPAPEK), 4-isopropyl-4'-methyldiphenyliodonium tetrakis(pentafluorophenyl)borate, dipyrazino[2,3-f:2',3'-h]quinoxaline-2,3,6,7,10,11-hexacarbonitrile (HAT-CN), 1,3,4,5,7,8-hexafluorotetracyano-2,6-naphthoquinodimethane (F6-TCNNQ), and the like.

In addition, the hole transport layer 32 or other layers constituting the hole transport region 3 are not particularly limited, and may include, for example, a known hole transport material. Examples of the hole transport material may include N-phenylcarbazole, a carbazole-based derivative such as polyvinyl carbazole, a fluorene-based derivative, N,N'-bis(3-methylphenyl)-N,N'-diphenyl-[1,1-biphenyl]-4,4'-diamine (TPD), a triphenylamine-based derivative such as 4,4',4"-tris(N-carbazolyl)triphenylamine (TCTA), N,N'-di(naphthalene-1-yl)-N,N'-diphenyl-benzidine (NPB), 4,4'-cyclohexylidenebis[N,N-bis(4-methylphenyl)benzeneamine] (TAPC), 4,4'-bis[N,N'-(3-tolyl)amino]-3,3'-dimethylbiphenyl (HMTPD), 1,3-bis(N-carbazolyl)benzene (mCP), Compound HTM1, Compound HTM2, Compound HT1, and the like.

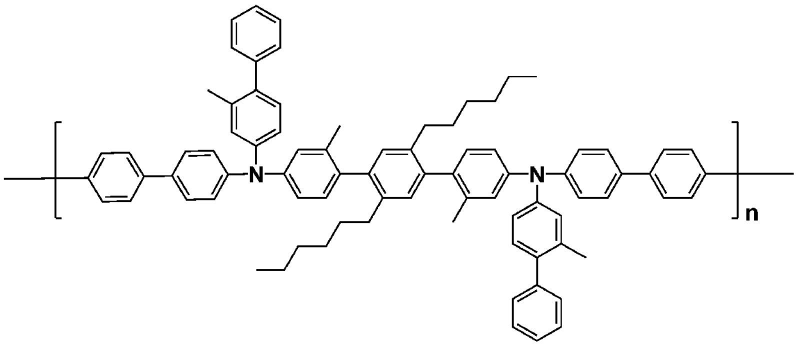

(n is an integer of 1 or more)

HTM1

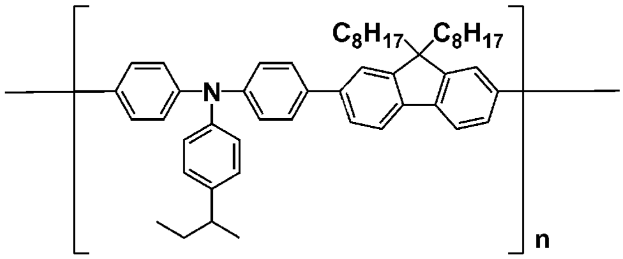

(n is an integer of 1 or more)

HTM2

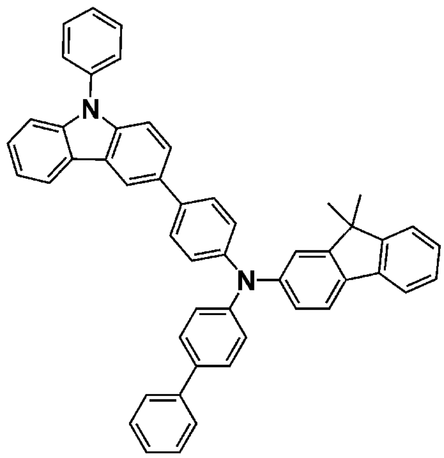

HT1

The hole transport region 3 may further include a charge generation material, in addition to the hole injection material or the hole transport material, to improve conductivity. The charge generation material may be homogeneously or non-homogeneously dispersed in the hole transport region 3 or each layer thereof. The charge generation material is not particularly limited, and may be, for example, a known charge generation material. The charge generation material may be, for example, a p-dopant. Examples of the p-dopant may include a quinone derivative such as tetracyanoquinonedimethane (TCNQ) or 2,3,5,6-tetrafluoro-tetracyanoquinodimethane (F4-TCNQ), a metal oxide such as tungsten oxide or molybdenum oxide, a cyano group-containing compound, and the like.

The buffer layer (not shown) may compensate for an optical resonance distance according to a wavelength of light emitted from the emission layer 4 to improve luminescence efficiency. Materials included in the hole buffer layer (not shown) are not particularly limited, and materials used in a known hole buffer layer may be used. For example, the compounds that may be included in the hole transport region 3 may be used.

The electron blocking layer 33 may prevent injection of electrons from the electron transport region 5 to the hole transport region 3. Materials included in the electron blocking layer 33 are not particularly limited, and materials used in a known electron blocking layer may be used. For example, the host material, such as Compounds H55, H86, and H87, included in the emission layer (the material for an organic light-emitting device) may be used.

A thickness of the hole transport region 3 may be, but is not particularly limited to, about 1 nm to about 1,000 nm, for example, about 10 nm to about 500 nm. In addition, regarding each layer constituting the hole transport region 3, a thickness of the hole injection layer 31 may be, but is not particularly limited to, about 3 nm to about 200 nm. A thickness of the hole transport layer 32 may be, but is not particularly limited to, about 3 nm to about 200 nm. A thickness of the electron blocking layer 33 may be, but is not particularly limited to, about 1 nm to about 100 nm. In addition, a thickness of the hole buffer layer (not shown) is not particularly limited, as long as the hole buffer layer functions as a hole buffer layer and does not interfere with functions of an organic light-emitting device. When the thickness of the hole transport region 3, the hole injection layer 31, the hole transport layer 32, or the electron blocking layer 33 is within the ranges above, improved hole transport characteristics may be obtained while suppressing a substantial increase in driving voltage.

Examples of the film forming method of the hole transport region 3 or each layer thereof may include, but are not particularly limited to, known film forming methods such as vacuum deposition, spin coating, LB deposition, ink-jet printing, laser-printing, and LITI.

Emission layer 4

The emission layer 4 may be located on the hole transport region 3. Details of the emission layer 4 may be the same as described above.

Electron transport region 5

The electron transport region 5 may be located on the emission layer 4. The electron transport region 5 may include at least one of the electron injection layer 51, the electron transport layer 52, and the hole blocking layer 53, but embodiments are not limited thereto.

The electron transport region 5 may be a single layer including a single material or a single layer including a plurality of different materials. In addition, the electron transport region 5 may have a multi-layer structure with a plurality of layers including a plurality of different materials. For example, the electron transport region 5 may have a single-layer structure including the electron injection layer 51 or the electron transport layer 52. In addition, for example, the electron transport region 5 may have a single-layer structure including an electron injection material and an electron transport material. In addition, for example, the electron transport region 5 may have an electron transport layer 52/electron injection layer 51 structure, wherein constituting layers are sequentially stacked from the emission layer 4. In addition, for example, the electron transport region 5 may have a hole blocking layer 53/electron transport layer 52/electron injection layer 51 structure, wherein constituting layers are sequentially stacked from the emission layer 4. However, the structure of the electron transport region 5 is not limited to the examples above.

The electron injection layer 51 or other layers constituting the electron transport region 5 are not particularly limited, and may include, for example, a known electron injection material. Examples of the electron injection material may include a lanthanide metal such as LiF, lithium quinolate (LiQ), $Li_2O$, BaO, NaCl, CsF, or Yb, or a metal halide such as RbCl. The electron injection layer 51 is not particularly limited, and may include, for example, the electron transport material and an insulating organometallic salt. The organometallic salt is not particularly limited, and may be, for example, a material with an energy band gap of 4 eV or more. The organometallic salt may be, for example, an acetate metallic salt, a benzoate metallic salt, an acetoacetate metallic salt, an acetylacetonate metallic salt, or a stearate metallic salt.

The electron transport layer 52 or other layers constituting the electron transport region 5 are not particularly limited, and may include, for example, a known electron transport material. Examples of the electron transport material may include an anthracene-based compound, tris(8-hydroxyquinolinolato)aluminum) ($Alq_3$), 1,3,5-tri[(3-pyridyl)-pen-3-yl]benzene, 2,4,6-tris(3'-pyridine-3-yl)biphenyl-3-yl)-1,3,5-triazine, 2-(4-(N-phenylbenzimidazolyl-1-ylphenyl)-9,10-dinaphthylanthracene, 1,3,5-tri(1-phenyl-1H-benzo[d]imidazol-2-yl)phenyl (TPBi), 2,9-dimethyl-4,7-diphenyl-1,10-phenanthroline (BCP), 4,7-diphenyl-1,10-phenanthroline (Bphen), 3-(4-biphenyl)-4-phenyl-5-tert-butylphenyl-1,2,4-triazole (TAZ), 4-(naphthalen-1-yl)-3,5-diphenyl-4H-1,2,4-triazole (NTAZ), 2-(4-biphenyl)-5-(4-tert-butylphenyl)-1,3,4-oxadiazole (tBu-PBD), bis(2-methyl-8-quinolinolato-N1,O8)-(1,1'-biphenyl-4-olato)aluminum (BAlq), berylliumbis(benzoquinoline-10-olato) ($Bebq_2$), 9,10-di(naphthalen-2-yl)anthracene (ADN), lithium quinolate (LiQ), Compound ET1, and the like. In addition, TRE314 (product of Toray Co., Ltd., electron transport material) or the like may be used.

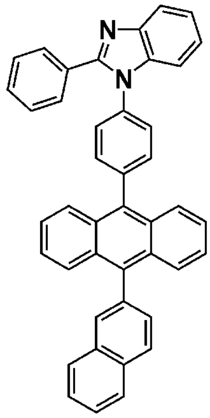

ET1

The hole blocking layer 53 may prevent injection of holes from the hole transport region 3 to the electron transport region 5. Materials included in the hole blocking layer 53 are not particularly limited, and materials used in a known hole blocking layer may be used. The hole blocking layer 53 may include, for example, a known hole blocking material. Examples of the hole blocking material may include 2,9-dimethyl-4,7-diphenyl-1,10-phenanthroline (BCP), 4,7-diphenyl-1,10-phenanthroline (BPhen), and the like. In addition, for example, the host material, such as Compounds H77 and H87, included in the emission layer (the material for an organic light-emitting device) may be used.

A thickness of the electron transport region 5 may be, but is not particularly limited to, about 0.1 nm to about 200 nm, for example, about 30 nm to about 150 nm. In addition, regarding each layer constituting the electron transport region 5, a thickness of the electron transport layer 52 may be, but is not particularly limited to, about 10 nm to about 100 nm, for example, about 15 nm to about 50 nm. A thickness of the hole blocking layer 53 may be, but is not particularly limited to, about 1 nm to about 100 nm, for example, about 5 nm to about 30 nm. A thickness of the electron injection layer 51 may be, but is not particularly limited to, about 0.1 nm to about 10 nm, for example, about 0.3 nm to about 9 nm. When the thickness of the electron injection layer 51 is within the ranges above, improved electron injection characteristics may be obtained while suppressing a substantial increase in driving voltage. In addition, when the thickness of the electron transport region 5, the electron injection layer 51, the electron transport layer 52, or the hole blocking layer 53 is within the ranges above, improved hole transport characteristics may be obtained while suppressing a substantial increase in driving voltage.

Examples of the film forming method of the electron transport region 5 or each layer thereof may include, but are not particularly limited to, known film forming methods such as vacuum deposition, spin coating, LB deposition, ink-jet printing, laser-printing, and LITI.

The second electrode 6 may be located on the electron transport region 5. The second electrode 6 may have conductivity. In the organic light-emitting device 10 according to an embodiment, the second electrode 6 may be a common electrode or a cathode. In addition, the second electrode 6 may be a reflective electrode, a semi-reflective electrode, or a transmissive electrode.

A material for forming the second electrode 6 is not particularly limited, and may be, for example, a metal, a metal alloy, or a conductive compound. When the second electrode 6 is a reflective electrode, the second electrode 6 may include a transparent metal oxide, for example, ITO, IZO, ZnO, ITZO, or the like. When the second electrode 6 is a semi-transmissive electrode or a reflective electrode, the second electrode 6 may include Ag, Mg, Cu, Al, Pt, Pd, Au, Ni, Nd, Ir, Cr, Li, Ca, LiF/Ca, LiF/Al, Mo, Ti, or a compound or mixture thereof (e.g., a mixture of Ag and Mg).

The second electrode 6 may be a single layer including a single material or a single layer including a plurality of different materials. In addition, the second electrode 6 may have a multi-layer structure with a plurality of layers including a plurality of different materials.

A thickness of the second electrode 6 may be, but is not particularly limited to, about 10 nm to about 1,000 nm.

The second electrode 6 may be connected to an auxiliary electrode (not shown). By connecting the second electrode 6 to the auxiliary electrode, the resistance of the second electrode 6 may be reduced.

In addition, a capping layer (not shown) may be further located on the second electrode 6. Examples of the capping layer (not shown) may include, but are not particularly limited to, $\alpha$-NPD, NPB, TPD, m-MTDATA, $Alq_3$, CuPc, N4,N4,N4',N4'-tetra(biphenyl-4-yl)biphenyl-4,4'-diamine (TPD15), 4,4',4"-tri-9-carbazolyltriphenylamine (TCTA), N,N'-bis(naphthalen-1-yl), and the like.

Materials constituting each layer and each electrode may be used alone or in combination of two or more.

In the organic light-emitting device 10 of FIGS. 2 to 4, the blue luminescent compound, the fluorescence emitter, or the material for an organic light-emitting device may be included in the emission layer 4, but may also be included in organic layers other than the emission layer 4. In addition, the blue luminescent compound, the fluorescence emitter, or the

material for an organic light-emitting device may be included in the emission layer 4 and the organic layers other than the emission layer 4.

In the organic light-emitting device 10 of FIGS. 2 to 4, since a voltage is applied to each of the first electrode 2 and the second electrode 6, holes provided from the first electrode 2 may move toward the emission layer 4 through the hole transport region 3, and electrons provided from the second electrode 6 may move toward the emission layer 4 through the electron transport region 5. The holes and the electrons may recombine in the emission layer 4 to produce excitons, and these excitons may transition from an excited state to a ground state to thereby generate light.

While embodiments of the disclosure have been described in detail, it will be understood that these embodiments are illustrative and not intended to be limiting, and the scope of the disclosure should be construed in accordance with the appended claims.

Examples

Hereinafter, the disclosure will be described in more detail with reference to the following examples, but the technical scope of the disclosure is not limited thereto. Simulation evaluation of condensed cyclic compound

According to "High-Performance Dibenzoheteraborin-Based Thermally Activated Delayed Fluorescence Emitters: Molecular Architectonics for Concurrently Achieving Narrowband Emission and Efficient Triplet-Singlet Spin Conversion", In Seob Park, Kyohei Matsuo, Naoya Aizawa, and Takuma Yasuda, Advanced Functional Materials 2018, 28, 1802031, the spectrum width of fluorescence (the FWHM of the fluorescence spectrum) has a close relationship with the reorganization energy [$E(S_0@S_1)$-$E(S_0@S_0)$] that is expressed by the difference between the ground state ($S_0$) energy of the stable structure in the first excitation singlet state ($S_1$) [$E(S_0@S_1)$] and the ground state ($S_0$) energy of the stable structure in the ground state ($S_0$) [$E(S_0@S_0)$].

Identification of relationship between reorganization energy and spectrum width of fluorescence

First, the relationship between the reorganization energy [$E(S_0@S_1)$-$E(S_0@S_0)$] and the spectrum width (FWHM) of fluorescence was identified as follows.

Calculation by DFT

For Condensed Cyclic Compounds R1 to R3 known in the art, the following calculations were performed by DFT.

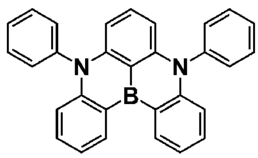

R1

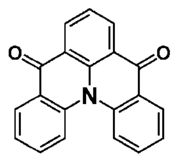

R2

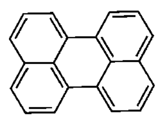

R3

The ground state ($S_0$) energy of the stable structure in the first excitation singlet state ($S_1$) [$E(S_0@S_1)$] and the ground state ($S_0$) energy of the stable structure in the ground state ($S_0$) [$E(S_0@S_0)$] were calculated, and from the difference therebetween, the reorganization energy [$E(S_0@S_1)$]-[$E(S_0@S_0)$] (eV) was calculated.

In addition, the first excitation singlet state ($S_1$) energy of the stable structure in the first excitation singlet state ($S_1$) [$E(S_1@S_1)$] was calculated, and from the difference between this value and the ground state ($S_0$) energy of the stable structure in the ground state ($S_0$) [$E(S_0@S_0)$], the adiabatic first excitation singlet state ($S_1$) energy [$E(S_1@S_1)$]-[$E(S_0@S_0)$] (eV) was calculated.

Then, the emission wavelength (nm) obtained by converting the adiabatic first excitation singlet state ($S_1$) energy into a light wavelength (nm) was calculated.

In addition, the oscillator strength (f) of the stable structure in the first excitation singlet state ($S_1$) was calculated.

In addition, the highest occupied molecular orbital (HOMO) energy and the lowest unoccupied molecular orbital (LUMO) energy were calculated.

In this regard, calculation by DFT was performed using Gaussian 16 (Gaussian Inc.) as the calculation software, according to the following calculation methods (I), (II), and (III):

(I) $S_0$ calculation method: structural optimization calculation by DFT including functional B3LYP, basis function 6-31G(d, p), and toluene solvent effect (PCM);

(II) $S_1$ calculation method: structural optimization calculation by time-dependent DFT (TDDFT) including functional B3LYP, basis function 6-31G(d, p), and toluene solvent effect (PCM); and

(III) $S_0$ calculation method: calculation on input structure by DFT including functional B3LYP, basis function 6-31G(d,

p), and toluene solvent effect (PCM).

In detail, the calculation of each item was performed by using the following calculation methods:

- Ground state ($S_0$) energy of stable structure in ground state ($S_0$) [$E(S_0@S_0)$]: Calculation method (I);
- First excitation singlet state ($S_1$) energy of stable structure in first excitation singlet state ($S_1$) [$E(S_1@S_1)$]: Calculation method (II);
- Ground state ($S_0$) energy of stable structure in first excitation singlet state ($S_1$) [$E(S_0@S_1)$]: Calculation methods (II) and (III);
- Reorganization energy [$E(S_0@S_1)$]-[$E(S_0@S_0)$]: Calculation methods (I), (II), and (III);
- Adiabatic first excitation singlet state ($S_1$) energy [$E(S_1@S_1)$]-[$E(S_0@S_0)$]: Calculation methods (I) and (II);
- Emission wavelength (nm): Calculation methods (I) and (II);
- Oscillator strength (f) of stable structure in first excitation singlet state ($S_1$): Calculation method (II); and
- HOMO and LUMO: Calculation method (I).

FIG. 5 is an explanatory diagram qualitatively illustrating each energy relationship.

Measurement of spectrum width (FWHM) of fluorescence

For each of toluene solutions respectively including Condensed Cyclic Compounds R1 to R3 at a concentration of $1\times10^{-5}$ M (moles per liter), the PL peak wavelength (nm) of fluorescence and the spectrum width of fluorescence (the FWHM of the fluorescence spectrum peak) were measured at room temperature with an excitation wavelength of 320 nm with a spectrofluorophotometer F-7000 manufactured by Hitachi High-Tech Science Co., Ltd.

The results thereof are shown in Table 1.

Table 1

| | Compound | Calculated value of fluorescent wavelength (nm) | Calculated value of reorganization energy (eV) | Found value of fluorescence peak wavelength (nm) | Found value of FWHM (nm) |
|---|---|---|---|---|---|
| Reference Calculation Example 1 | R1 | 415 | 0.109 | 453 | 22 |
| Reference Calculation Example 2 | R2 | 419 | 0.132 | 451 | 26 |
| Reference Calculation Example 3 | R3 | 474 | 0.164 | 445 | 42 |

In this regard, a graph of the FWHM of fluorescence in PL measured in Condensed Cyclic Compounds R1 to R3 versus the reorganization energy (eV) calculated by DFT is shown in FIG. 6. As shown in FIG. 6, it was confirmed that there was a relationship between the reorganization energy (eV) calculated by DFT and the FWHM of fluorescence.

Synthesis Example 1

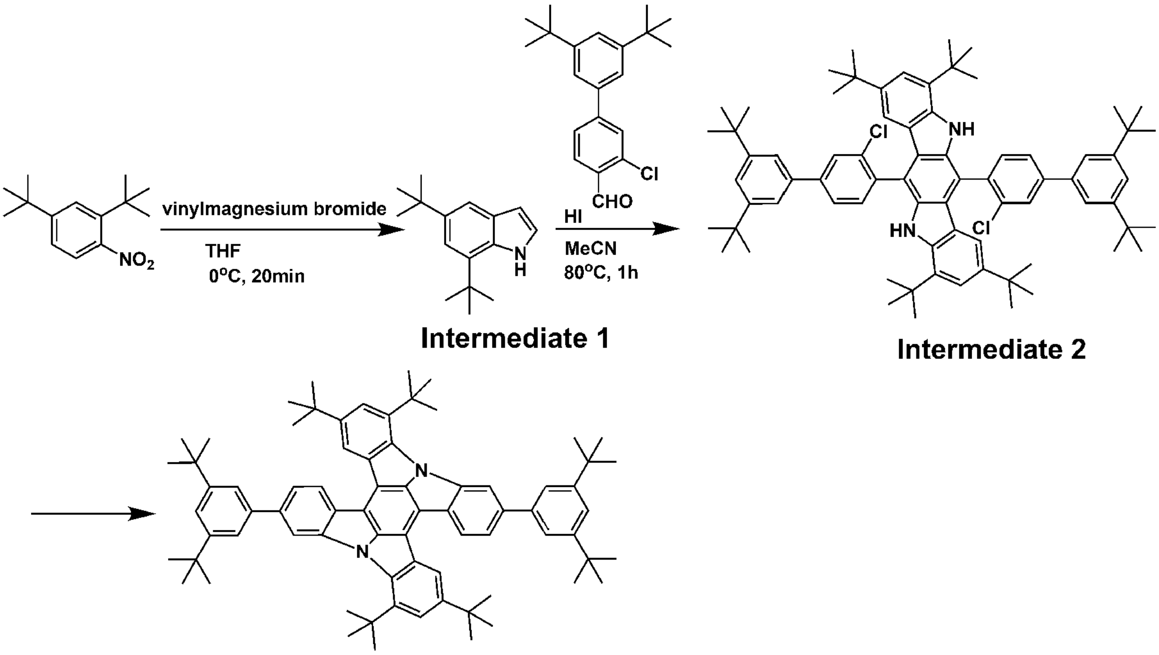


Intermediate 1

Intermediate 2

Compound 1

Synthesis of Intermediate 1

Under a nitrogen atmosphere, 2,4-di-tert-butylnitrobenzene [6.4 grams (g), 27 millimoles (mmol)) and 270 milliliters (mL) of tetrahydrofuran (THF) were added to a 500 mL three-neck flask and cooled to -40 °C. Vinyl magnesium bromide (81 mL of 1 mole per liter (M) THF solution) was added dropwise thereto. The temperature was raised to 0 °C, and the reaction solution was stirred for 20 minutes. A saturated aqueous ammonium chloride solution was added to the reaction solution, and the mixture was extracted with ethyl acetate. The extracted organic layer was washed with water and a saturated saline solution and dried with anhydrous sodium sulfate. The remaining solvent was removed under reduced pressure to provide a red, oily residue. The residue was purified by silica gel column chromatography (hexane and dichloromethane, 4:1 v/v) to provide Intermediate 1 as a brown solid (2.2 g, 36 % yield). The above process was repeated to obtain more Intermediate 1.

1H-NMR (300MHz, $CD_2Cl_2$): δ1.37(s, 9H), 1.50(s, 9H), 6.47-6.49(m, 1H), 7.18-7.20(m, 2H), 7.46(s, 1H), 8.26(br, 1H); LC-MS: 230($[M+H]^+$).

Synthesis of Intermediate 2

Under a nitrogen atmosphere, Intermediate 1 (3.4 g, 15 mmol), 3',5'-di-tert-butyl-3-chloro-[1,1'-biphenyl]-4-carbaldehyde (4.9 g, 15 mmol), and acetonitrile (150 mL) were added to a 500 mL three-neck flask. After raising the temperature up to 80 °C, hydroiodic acid (57 mass%, 0.42 mL, 3.0 mmol) was added thereto, and the reaction solution was stirred for an hour. After cooling to room temperature, the precipitated solid was filtered and washed with cooled acetonitrile to provide a light orange powder containing Intermediate 2 (7.0 g).

Synthesis of Compound 1

Under a nitrogen atmosphere, Intermediate 2 (7.0 g), potassium carbonate (9.0 g, 65 mmol), copper(I) iodide (12 g, 63 mmol), 1,10-phenanthroline (12 g, 67 mmol), and 1,3-dimethylimidazolidinone (220 mL) were added to a 500 mL three-neck flask, and the reaction solution was heated and stirred at 220 °C for 4 hours. After cooling to room temperature, water was added to the reaction solution, and the precipitated solid was filtered. The filtered solid, methanol (90 mL), and ethylenediamine (10 mL) were combined in a 300 mL recovery flask and stirred at room temperature, and the solid was collected by filtration to provide Compound 1 as a yellow solid (5.7 g, 88% yield):

1H-NMR (300MHz, toluene-d8): δ1.46(s, 36H), 1.62(s, 18H), 1.95(s, 18H), 7.65(t, J=1.8Hz, 2H), 7.85(d, J=2.1Hz, 2H), 7.94(d, J=1.8Hz, 4H), 8.05(d, J=7.5Hz, 2H), 8.96(d, J=1.8Hz, 2H), 9.03(s, 2H), 9.08(d, J=8.1Hz, 2H);

LC-MS: 1005([M+H]+).

For Compound 1, the dihedral angles between a nitrogen-containing aromatic heterocyclic skeleton and two 3,5-di-tert-butylphenyl groups bonded to the nitrogen-containing aromatic heterocyclic skeleton via a single bond were calculated. The dihedral angles were calculated with GaussView (Gaussian Inc.) from the most stable structure calculated by DFT with Gaussian 16 (Gaussian Inc.) as the calculation software, according to Calculation method (I), were 138.74° and 138.75°, respectively. Evaluation of compound by calculation: Calculation of oscillator strength (f), reorganization energy, and emission wavelength of compound of disclosure

By the same method as described in the "Identification of relationship between reorganization energy and spectrum width of fluorescence" section, the reorganization energy and peak wavelength (emission wavelength) of fluorescence were calculated for Compound 1 (Calculation Example 1) according to the disclosure obtained in Synthesis Example 1, Comparative Compound 1 (Comparative Calculation Example 1) outside the scope of the disclosure, and other compounds (Calculation Examples 2 to 12). Table 2 provides data for various substitution positions of tert-butyl groups, and Table 3 provides data for various substitution positions of aromatic substituents.

Table 2

| | Structural formula | Emission wavelength (nm) | Reorganization energy (eV) |
|---|---|---|---|
| Calculation Example 1 | 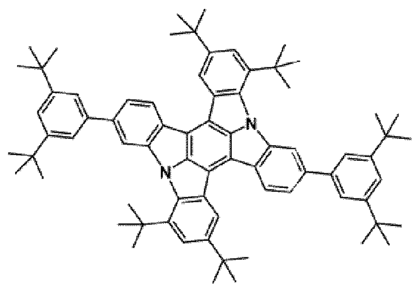 Compound 1 | 443 | 0.101 |
| Comparative Calculation Example 1 | 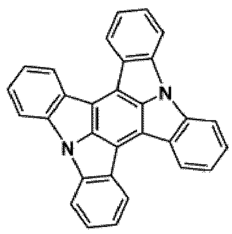 Comparative Compound 1 | 430 | 0.055 |
| Calculation Example 2 | 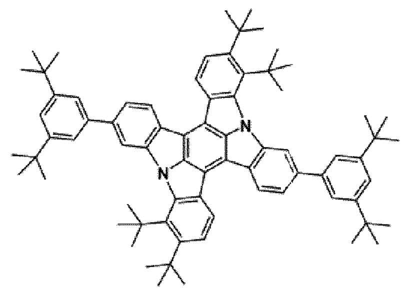 | 450 | 0.101 |
| Calculation Example 3 | 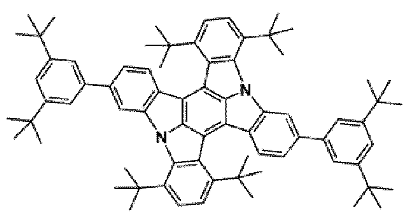 | 466 | 0.100 |

(continued)

| | Structural formula | Emission wavelength (nm) | Reorganization energy (eV) |
|---|---|---|---|
| Calculation Example 4 | 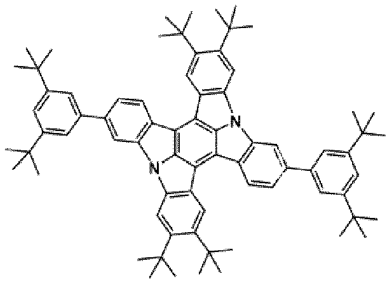 | 450 | 0.087 |
| Calculation Example 5 | 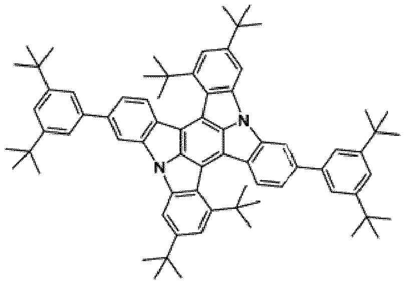 | 458 | 0.079 |
| Calculation Example 6 | 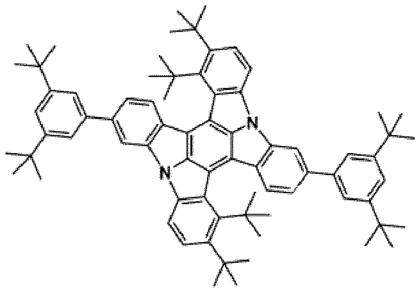 | 481 | 0.078 |

Table 3

| | Structural formula | Emission wavelength (nm) | Reorganization energy (eV) |
|---|---|---|---|
| Calculation Example 7 | 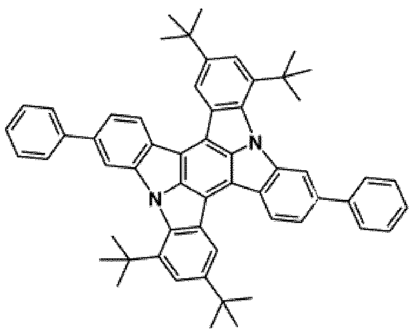 | 444 | 0.093 |

(continued)

| | Structural formula | Emission wavelength (nm) | Reorganization energy (eV) |
|---|---|---|---|
| Calculation Example 8 | 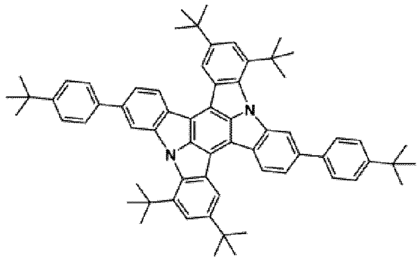 | 446 | 0.107 |
| Calculation Example 9 | 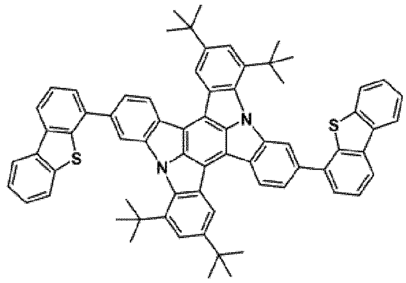 | 442 | 0.090 |
| Calculation Example 10 | 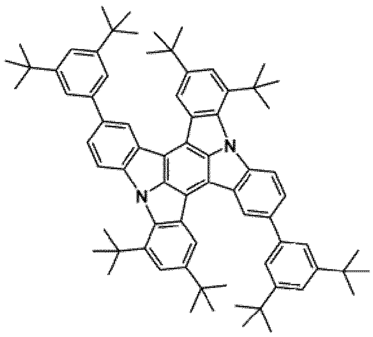 | 442 | 0.059 |
| Calculation Example 11 | 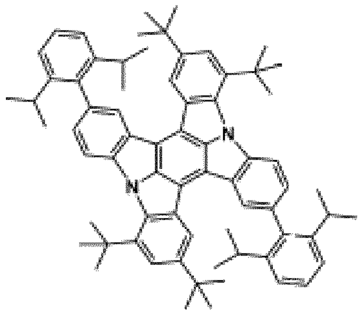 | 436 | 0.051 |
| Calculation Example 12 | 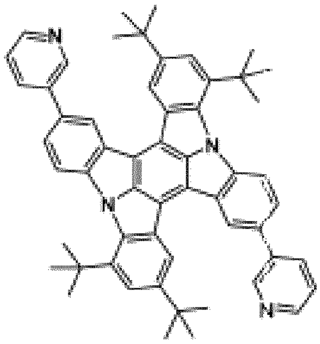 | 437 | 0.053 |

As shown in Tables 2 and 3, effects were particularly improved in terms of emission wavelength and reorganization energy when the substituent positions were as in Calculation Example 1.

Evaluation of $\beta_{sum}$

$\beta_{sum}$ was calculated as follows.

$\beta_1$, $\beta_2$, $\beta_3$, $\beta_4$, $\beta_5$, and $\beta_6$ were calculated with GaussView (Gaussian Inc.) from the most stable structure calculated according to (I) $S_0$ calculation method: structural optimization calculation by DFT including functional B3LYP, basis function 6-31G(d,p), and toluene solvent effect (PCM) in the "Calculation by DFT" section.

The calculation method will be described using $\beta_{sum}$ of Compound 1 (Calculation Example 15 in Table 5) as an example. Bn" is considered for atoms in substituent $Ar_1$ in Formula (1), and the distance from B1, $\alpha\cdot|(\sin\theta)|\cdot|(\sin\varphi)|$ , is calculated for each Bn". Then, when all of B2" to Bn" are in the same direction on the z-axis (when all values of $\sin\varphi$ have the same sign), $\beta$ is the largest distance among the distances from the initial point of B1 to each point, and thus becomes the maximum value among all values of $\alpha\cdot|(\sin\theta)|\cdot|(\sin\varphi)|$.

In addition, when there are points where z-coordinates have different signs (when there are points where $\sin\varphi$ is positive and where $\sin\varphi$ is negative), the sum of the maximum value of $\alpha\cdot|(\sin\theta)|\cdot|(\sin\varphi)|$ at the point where $\sin\varphi$ is positive and the maximum value of $\alpha\cdot|(\sin\theta)|\cdot|(\sin\varphi)|$ at the point where $\sin\varphi$ is negative becomes $\beta$.

In detail, in the case of $Ar_1$, the results shown in Table 4 were obtained. The point where $\sin\varphi$ was positive and $\alpha\cdot|(\sin\theta)|\cdot|(\sin\varphi)|$ was the largest was B2", and the values of $\alpha$, $\theta$, and $\varphi$ were $\alpha 2$, $\theta 2$, and $\varphi 2$, respectively. In addition, the point where $\sin\varphi$ was negative and $\alpha\cdot|(\sin\theta)|\cdot|(\sin\varphi)|$ was the largest was B3", and the values of $\alpha$, $\theta$, and $\varphi$ were $\alpha 3$, $\theta 3$, and $\varphi 3$, respectively. Then, the sum of respective values of $\alpha\cdot|(\sin\theta)|\cdot|(\sin\varphi)|$ was expressed as $\beta_1$.

For $Ar_2$, $R_1$, $R_2$, $R_3$, and $R_4$ of Compound 1, the same calculations were performed to calculate $\beta_2$, $\beta_3$, $\beta_4$, $\beta_5$, $\beta_6$, and $\beta_{sum}$, and the values thereof are also shown in Table 4. In addition, $\beta_{sum}$ was calculated for the compounds shown in Table 5.

Table 4

| | $\alpha 2$ | $\theta 2$ | $\varphi 2$ | sin $\varphi 2$ sign | $\alpha 2\cdot|(\sin\theta 2)|\cdot|(\sin\varphi 2)|$ | $\alpha 3$ | $\theta 3$ | $\varphi 3$ | sin $\varphi 3$ sign | $\alpha 3\cdot|(\sin\theta 3)|\cdot|(\sin\varphi 3)|$ | $\beta$ |
|---|---|---|---|---|---|---|---|---|---|---|---|
| $Ar_1$ | 4.37 | 124.06 | 117.93 | positive (+) | 3.20 | 4.77 | 143.07 | -68.78 | negative (-) | 2.67 | $\beta_1$ = 5.9 |
| $Ar_2$ | 4,37 | 124.06 | 117.93 | positive (+) | 3.20 | 4.78 | 143.07 | -68.79 | negative (- ) | 2.68 | $\beta_2$ = 5.9 |
| $R_1$ | 1.55 | 109.60 | 120.04 | positive (+) | 1.26 | 1.55 | 109.61 | -119.99 | negative (-) | 1.26 | $\beta_3$ = 2.5 |
| $R_2$ | 1.55 | 108.75 | 116.93 | positive (+) | 1.31 | 1.55 | 108.51 | -117.28 | negative (-) | 1.31 | $\beta_4$ = 2.6 |
| $R_3$ | 1.55 | 109.60 | 120.04 | positive (+) | 1.26 | 1.55 | 109.61 | -119.99 | negative (-) | 1.26 | $\beta_5$ = 2.5 |
| $R_4$ | 1.55 | 108.74 | 116.92 | positive (+) | 1.31 | 1.55 | 108.51 | 117.30 | negative (-) | 1.31 | $\beta_6$ = 2.6 |
| | | | | | | | | | | | $\beta_{sum}$ = 22.0 |

Table 5

| | Structural formula | $\beta_1$ | $\beta_2$ | $\beta_3$ | $\beta_4$ | $\beta_5$ | $\beta_6$ | $\beta_{sum}$ | Emission wavelength (nm) | Reorganization energy (eV) |
|---|---|---|---|---|---|---|---|---|---|---|
| Comparative Calculation Example 2 | 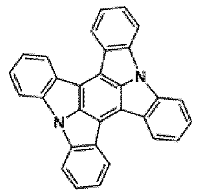 Comparative Compound 1 | - | - | - | - | - | - | - | 430 | 0.055 |
| Calculation Example 13 | 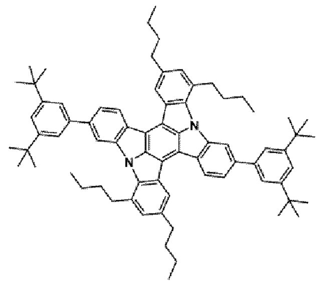 | 23 | 23 | 1.4 | 2.4 | 1.4 | 2.4 | 12.2 | 453 | 0.086 |
| Calculation Example 14 | 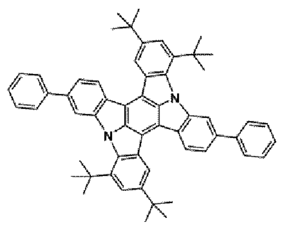 | 1.5 | 1.5 | 2.5 | 2.6 | 2.5 | 2.6 | 13.3 | 444 | 0.093 |
| Calculation Example 15 | 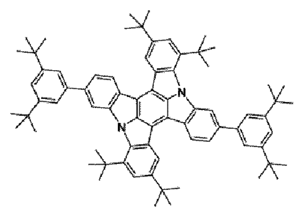 Compound 1 | 5.9 | 5.9 | 2.5 | 2.6 | 2.5 | 2.6 | 22.0 | 443 | 0.101 |

(continued)

| | Structural formula | $\beta_1$ | $\beta_2$ | $\beta_3$ | $\beta_4$ | $\beta_5$ | $\beta_6$ | $\beta_{sum}$ | Emission wavelength (nm) | Reorganization energy (eV) |
|---|---|---|---|---|---|---|---|---|---|---|
| Calculation Example 16 | 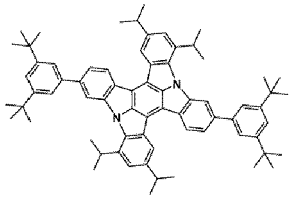 | 5.8 | 5.8 | 2.5 | 2.3 | 2.5 | 2.6 | 21.6 | 448 | 0.089 |
| Calculation Example 17 | 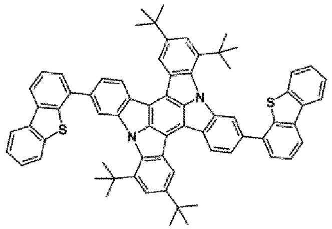 | 5.6 | 5.6 | 2.5 | 2.6 | 2.5 | 2.6 | 21.5 | 443 | 0.077 |
| Calculation Example 18 | 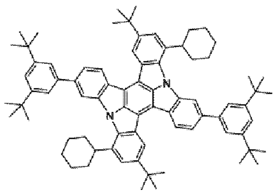 | 6.0 | 6.1 | 2.5 | 2.5 | 2.5 | 2.5 | 22.2 | 448 | 0.085 |
| Calculation Example 19 | 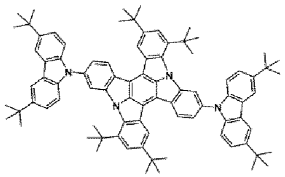 | 10.0 | 9.8 | 2.5 | 2.6 | 2.5 | 2.6 | 30.0 | 452 | 0.111 |

As shown in Table 5, it was found that the emission wavelength and color purity were further improved when $\beta_{sum}$ was greater than 13 and less than 28.

Evaluation of compound

Measurement of spectrum width (FWHM) of fluorescence: toluene solution

Toluene solutions respectively including Compound 1 according to the disclosure and Comparative Compound 1 at a concentration of $1\times10^{-5}$ M were prepared. For each of the prepared toluene solutions, PL measurement was performed at room temperature with an excitation wavelength of 320 nm using a spectrofluorophotometer F-7000 manufactured by Hitachi High-Tech Science Co., Ltd. The results of evaluating the peak wavelength (nm) of the fluorescence spectrum in PL and the FWHM (nm) of the peak of the fluorescence spectrum are shown in Table 6 and FIG. 7.

Table 6

| Compound | Emission wavelength (nm) | FWHM (nm) |
|---|---|---|
| Compound 1 | 453 | 12 |
| Comparative Compound 1 | 446 | 12 |

As shown in Table 6 and FIG. 7, Compound 1 functioned as a blue luminescent material with high color purity.

Measurement of spectrum width (FWHM) of fluorescence: deposited film

Compound 1 was co-deposited on a quartz substrate at a weight ratio of 1 wt% based on the total weight of Host Compounds H89 and H93 together with Host Compounds H89 and H93 at a vacuum degree of $10^{-5}$ pascals (Pa) to prepare a thin film with a thickness of 50 nm. Host Compounds H89 and H93 respectively had structures represented by the following formulae, and were used at a weight ratio of H89:H93=60:40. In addition, a thin film was prepared for Comparative Compound 1 in the same manner as described above.

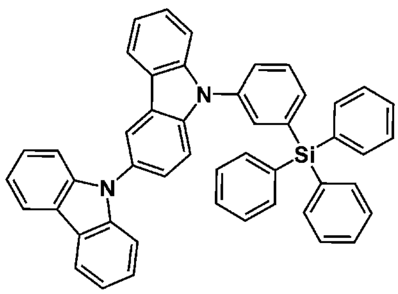

H89

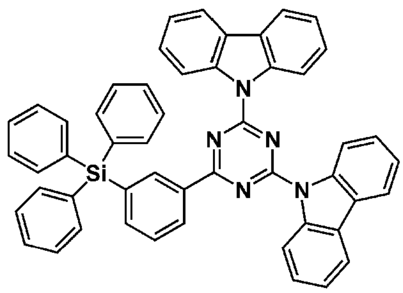

H93

The emission spectrum of each of the prepared thin films was measured at room temperature with an excitation wavelength of 360 nm with a spectrofluorophotometer F-7000 manufactured by Hitachi High-Tech Co., Ltd., and the peak wavelength (nm) of luminescence, and the FWHM (nm) of the peak of the emission spectrum were evaluated.

Measurement of PL quantum yield (PLQY)

PLQY was measured by using Quantaurus-QY absolute PLQY measurement device C11347-01 manufactured by Hamamatsu Photonics Co., Ltd. In the measurement, the excitation wavelength was measured by scanning at intervals of 10 nm from 280 nm to 350 nm, and the excitation wavelength region in which the compound absorption value showed 20 % or more of the excitation light intensity ratio was adopted. The value of PLQY was taken as the highest value in the adopted excitation wavelength region.

The results thereof are shown in Table 7. The fluorescence spectrum is also shown in FIG. 8.

Table 7

| Compound | Fluorescence peak wavelength (nm) | FWHM (nm) | PLQY (%) |
|---|---|---|---|
| Compound 1 | 461 | 17 | 86 |
| Comparative Compound 1 | 457 | 24 | 65 |

As shown in Table 7 and FIG. 8, it was found that Compound 1 had an emission peak wavelength of 461 nm and emitted blue light, as in the case of the solution including Compound 1. In addition, it was found that, since Compound 1 had an FWHM of 17 nm, which is almost the same as that shown in the results of the solution, the tert-butyl groups introduced at two positions may suppress intermolecular aggregation in the thin film state. Furthermore, the PLQY of Compound 1 was improved by 21 % compared to that of Comparative Compound 1. This is assumed to be the result of the introduced substituents effectively suppressing intermolecular aggregation.

Manufacture and evaluation of organic light-emitting device

Preparation of material for forming each layer

As materials for forming each layer of an organic light-emitting device, the following materials were prepared in addition to Compound 1 obtained in Synthesis Example 1, Compound H89, and Compound H93.

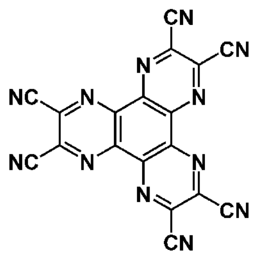

HAT-CN

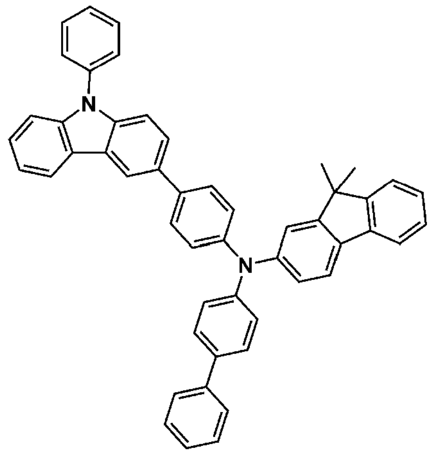

HT1

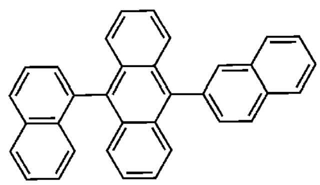

HT4

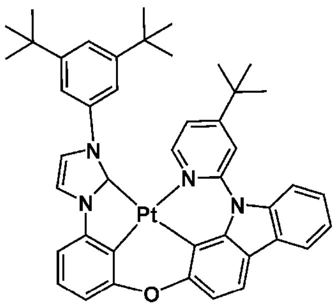

P120

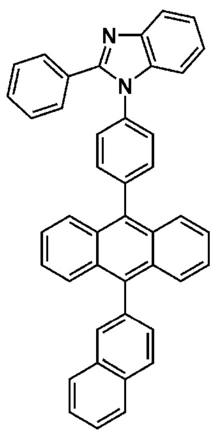

ET1

Manufacture 1 of organic light-emitting device

Example 1

An electrode-patterned ITO glass substrate was cut to a size of 50 mm x 50 mm x 0.7 mm, sonicated with acetone, isopropyl alcohol, and pure water, in this stated order, each for 15 minutes, and then cleaned by exposure to UV ozone for 30 minutes. The following layers were deposited on the ITO electrode (anode) of the glass substrate in a vacuum deposition apparatus.

First, HAT-CN was deposited on the ITO electrode to form a hole injection layer with a thickness of 10 nm. Next, Compound HT1 was deposited on the hole injection layer to form a hole transport layer with a thickness of 140 nm. Then, Compound H89 was deposited on the hole transport layer to form an electron blocking layer with a thickness of 5 nm. Thus, a hole transport region was formed.

Compound H89, Compound H93, and Compound 1 obtained in Synthesis Example 1 were co-deposited on the hole transport region obtained above to form an emission layer with a thickness of 40 nm. In this regard, the formation of the emission layer was performed such that the weight ratio of Compound H89 and Compound H93 (Compound H89:Compound H93) in the emission layer was 60:40. In addition, the formation of the emission layer was performed such that the concentration of Compound 1 was 1.5 wt% based on the total weight of Compound H89, Compound H93, and Compound 1 (that is, the total weight of the emission layer). Compounds H89 and H93 are host materials.

Compound H93 was vacuum-deposited on the emission layer obtained above to form a hole blocking layer with a thickness of 5 nm. Next, Compound ET1 and LiQ were co-deposited on the hole blocking layer at a weight ratio of Compound ET1:LiQ of 5:5 (parts by weight) to form an electron transport layer with a thickness of 30 nm. Then, LiQ was deposited on the electron transport layer to form an electron injection layer with a thickness of 1 nm. As a result, an electron transport region was formed.

Al (cathode) with a thickness of 100 nm was deposited on the electron injection layer to thereby manufacture an organic light-emitting device.

Then, in a glove box under a nitrogen atmosphere with water concentration of 1 ppm or less and oxygen concentration of 1 ppm or less, a glass sealing tube with a desiccating agent and an ultraviolet curing resin (manufactured by MORESCO, product name WB90US) were used to seal the organic light-emitting device to complete the manufacturing process.

Comparative Examples 1 to 3

Organic light-emitting devices were manufactured in the same manner as in Example 1, except that Comparative Compounds 1 to 3 were each used instead of Compound 1.

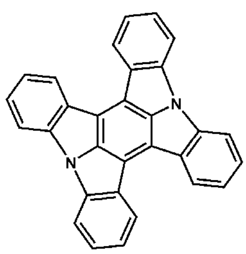

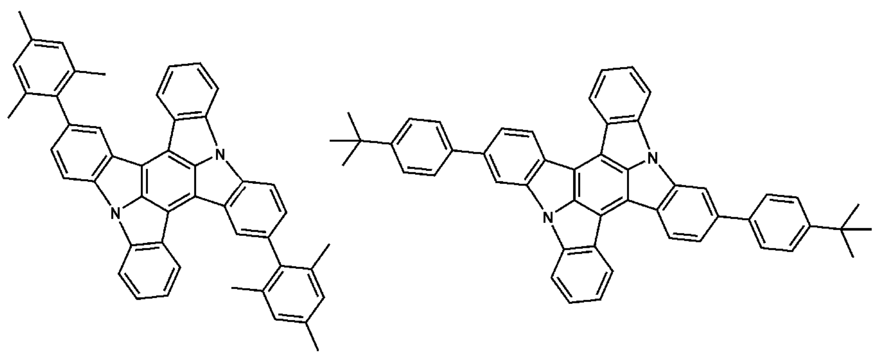

**Comparative Compound 1** **Comparative Compound 2** **Comparative Compound 3**

Manufacture 2 of organic light-emitting device

Example 2

An organic light-emitting device was manufactured in the same manner as in Example 1, except that Compound HT4 was used instead of Compound H89 and Compound H93 in forming an emission layer.

Manufacture 3 of organic light-emitting device

Example 3

An electrode-patterned ITO glass substrate was cut to a size of 50 mm x 50 mm x 0.7 mm, sonicated with acetone, isopropyl alcohol, and pure water, in this stated order, each for 15 minutes, and then cleaned by exposure to UV ozone for 30 minutes. The following layers were deposited on the ITO electrode (anode) of the glass substrate.

First, HAT-CN was deposited on the ITO electrode to form a hole injection layer with a thickness of 10 nm. Next, Compound HT1 was deposited on the hole injection layer to form a hole transport layer with a thickness of 140 nm. Then, Compound H89 was deposited on the hole transport layer to form an electron blocking layer with a thickness of 5 nm. As a result, a hole transport region was formed.

Compound H89, Compound H93, Phosphorescent Complex P120, and Compound 1 obtained in Synthesis Example 1 were co-deposited on the hole transport region obtained above to form an emission layer with a thickness of 40 nm. The formation of the emission layer was performed such that the weight ratio of Compound H89, Compound H93, and Phosphorescent Complex P120 (Compound H89:Compound H93:Phosphorescent Complex P120) in the emission layer was 60:40:13. In addition, the formation of the emission layer was performed such that the concentration of Compound 1 was 0.4 wt% based on the total weight of Compound H89, Compound H93, Phosphorescent Complex P120, and Compound 1 (that is, the total weight of the emission layer). Compounds H89 and H93 are host materials.

Compound H93 was vacuum-deposited on the emission layer obtained above to form a hole blocking layer with a thickness of 5 nm. Next, Compound ET1 and LiQ were co-deposited on the hole blocking layer at a weight ratio of Compound ET1:LiQ of 5:5 (parts by weight) to form an electron transport layer with a thickness of 30 nm. Then, LiQ was deposited on the electron transport layer to form an electron injection layer with a thickness of 1 nm. As a result, an electron transport region was formed.

Al (cathode) with a thickness of 100 nm was deposited on the electron injection layer to thereby manufacture an organic light-emitting device.

Then, in a glove box under a nitrogen atmosphere with water concentration of 1 ppm or less and oxygen concentration of 1 ppm or less, a glass sealing tube with a desiccating agent and an ultraviolet curing resin (manufactured by MORESCO, product name WB90US) were used to seal the organic light-emitting device to complete the manufacturing process

Comparative Examples 4 to 6

Organic light-emitting devices were manufactured in the same manner as in Example 3, except that Comparative Compounds 1 to 3 were each used instead of Compound 1.

Manufacture 4 of organic light-emitting device

Example 4

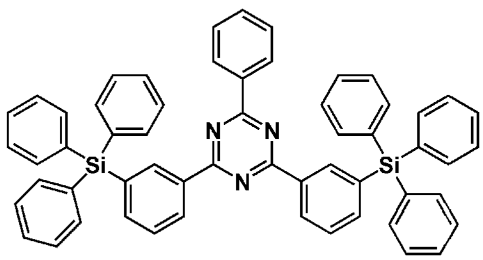

H91

An electrode-patterned ITO glass substrate was cut to a size of 50 mm x 50 mm x 0.7 mm, sonicated with acetone, isopropyl alcohol, and pure water, in this stated order, each for 15 minutes, and then cleaned by exposure to UV ozone for 30 minutes. The following layers were deposited on the ITO electrode (anode) of the glass substrate.

First, HAT-CN was deposited on the ITO electrode to form a hole injection layer with a thickness of 10 nm. Next, Compound HT1 was deposited on the hole injection layer to form a hole transport layer with a thickness of 60 nm. Then, Compound H89 was deposited on the hole transport layer to form an electron blocking layer with a thickness of 5 nm. As a result, a hole transport region was formed.

Compound H89, Compound H93, Phosphorescent Complex P120, and Compound 1 obtained in Synthesis Example 1 were co-deposited on the hole transport region obtained above to form an emission layer with a thickness of 40

nm. In this regard, the formation of the emission layer was performed such that the weight ratio of Compound H89, Compound H93, and Phosphorescent Complex P120 (Compound H89:Compound H93:Phosphorescent Complex P120) in the emission layer was 65:35:8. In addition, the formation of the emission layer was performed such that the concentration of Compound 1 was 0.4 wt% based on the total weight of Compound H89, Compound H93, Phosphorescent Complex P120, and Compound 1 (that is, the total weight of the emission layer). Compounds H89 and H93 are host materials.

Compound H91 was vacuum-deposited on the emission layer obtained above to form a hole blocking layer with a thickness of 5 nm. Next, Compound H91 and LiQ were co-deposited on the hole blocking layer at a weight ratio of Compound H91:LiQ of 5:5 (parts by weight) to form an electron transport layer with a thickness of 31 nm. Then, LiF was deposited on the electron transport layer to form an electron injection layer with a thickness of 1.5 nm. As a result, an electron transport region was formed.

Al (cathode) with a thickness of 80 nm was deposited on the electron injection layer to thereby manufacture an organic light-emitting device.

Then, in a glove box under a nitrogen atmosphere with water concentration of 1 ppm or less and oxygen concentration of 1 ppm or less, a glass sealing tube with a desiccating agent and an ultraviolet curing resin (manufactured by MORESCO, product name WB90US) were used to seal the organic light-emitting device to complete the manufacturing process.

Luminance, external quantum yield, and device lifespan

The peak wavelength of luminescence, spectrum width of luminescence, external quantum yield, and device lifespan at a luminance of 1,000 candela per square meter ($cd/m^2$) were evaluated according to the following method.

The organic light-emitting device was allowed to emit light while the voltage applied thereto was changed by using a DC constant voltage power supply (source meter 2400 manufactured by KEITHLEY), and the luminance, emission spectrum, and luminescence amount at this time were measured with a luminance measurement device (SR-3 manufactured by Topcon).

The external quantum yield was calculated from the luminescence amount, luminance, and current value at the time of measurement, and the external quantum yield at a luminance of 1,000 $cd/m^2$ was obtained.

The device lifespan (durability) was defined as LT95 (hour) by measuring the amount of time taken when the emission luminance, which decays as time lapses, becomes 95 % of the initial luminance when the device is continuously driven on a current value with an initial luminance of 1,000 $cd/m^2$.

The peak wavelength of luminescence and spectrum width of luminescence were read from the measurement results of the emission spectrum. The wavelength at the maximum intensity value of the emission spectrum was defined as the peak wavelength of luminescence, and the wavelength corresponding to half of the maximum intensity value of the emission spectrum was defined as the FWHM.

The evaluation results are shown in Tables 8 and 9. In Table 8, the external quantum yield and device lifespan are shown as relative values with respect to the value of Comparative Example 1 defined as 1. In addition, in Table 9, the external quantum yield and device lifespan are shown as relative values with respect to the value of Comparative Example 4 defined as 1.

Table 8

| | Compound | Peak wavelength (nm) | FWHM (nm) | External quantum yield | Device life span |
|---|---|---|---|---|---|
| Example 1 | Compound 1 | 461 | 16 | 1.7 | 1.6 |
| Example 2 | Compound 1 | 458 | 13 | 2.2 | 3.1 |
| Comparative Example 1 | Comparative Compound 1 | 457 | 25 | 1 | 1 |
| Comparative Example 2 | Comparative Compound 2 | 459 | 16 | 1.3 | 0.17 |
| Comparative Example 3 | Comparative Compound 3 | 470 | 29 | 1.3 | 0.21 |
| ※ External quantum yield and device lifespan are relative values with respect to value of Comparative Example 1 defined as 1 | | | | | |

Table 9

| | Compound | Peak wavelength (nm) | FWHM (nm) | External quantum yield | Device life span |
|---|---|---|---|---|---|
| Example 3 | Compound 1 | 462 | 19 | 1.7 | 2.2 |
| Example 4 | Compound 1 | 462 | 20 | 2.5 | 3.8 |
| Comparative Example 4 | Comparative Compound 1 | 461 | 20 | 1 | 1 |
| Comparative Example 5 | Comparative Compound 2 | 461 | 22 | 1.2 | 0.065 |
| Comparative Example 6 | Comparative Compound 3 | 466 | 23 | 1.5 | 1.4 |
| ※ External quantum yield and device lifespan are relative values with respect to value of Comparative Example 4 defined as 1 | | | | | |

As shown in Tables 8 and 9, the blue luminescent compound (Compound 1) according to the disclosure emitted light with a narrow spectrum with a peak wavelength within the blue wavelength region and exhibited blue luminescence having high color purity. In addition, it was confirmed that the organic light-emitting device according to the disclosure had an increased luminescence efficiency and lifespan.

According to the one or more embodiments, a compound with high color purity due to a small FWHM of an emission spectrum, with a peak wavelength within the blue wavelength region (e.g., a wavelength of about 445 nm to about 470 nm), and capable of improving the luminescence efficiency and lifespan of an organic light-emitting device may be provided.

It should be understood that embodiments described herein should be considered in a descriptive sense only and not for purposes of limitation. Descriptions of features or aspects within each embodiment should typically be considered as available for other similar features or aspects in other embodiments. While one or more embodiments have been described with reference to the figures, it will be understood by those of ordinary skill in the art that various changes in form and details may be made therein without departing from the scope as defined by the following claims.

## Claims

**1.** A blue luminescent compound comprising:

a nitrogen-containing aromatic heterocyclic skeleton; and
at least two aromatic substituents bonded to the nitrogen-containing aromatic heterocyclic skeleton,
wherein the nitrogen-containing aromatic heterocyclic skeleton comprises, as substituents, four branched or cyclic alkyl groups each having 3 to 20 carbon atoms, and the nitrogen-containing aromatic heterocyclic skeleton and the at least two aromatic substituents are not located on the same plane; and
wherein the blue luminescent compound is represented by Formula (1), wherein $\beta_{sum}$ as defined below is greater than 13 and less than 28:

Formula (1)

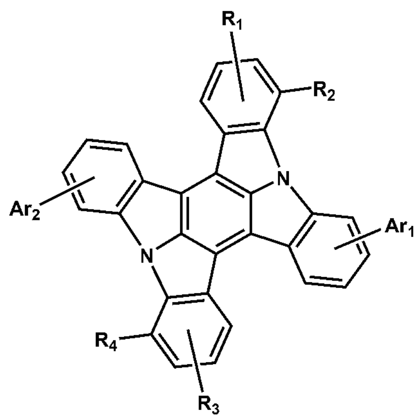

wherein, in Formula (1),
$R_1$ to $R_4$ are each independently a substituted or unsubstituted branched or cyclic alkyl group having 3 to 20 carbon atoms or a substituted or unsubstituted trialkylsilyl group having 3 to 60 carbon atoms, and
$Ar_1$ and $Ar_2$ are each independently a substituted or unsubstituted aromatic hydrocarbon cyclic group having 6 to 14 ring atoms or a substituted or unsubstituted aromatic heterocyclic group having 5 to 14 ring atoms:
$\beta_{sum}$
wherein, when $\beta_1$ is a size of substituent $Ar_1$ in Formula (1) in a direction perpendicular to a core represented by Formula (1a),
$\beta_2$ is a size of substituent $Ar_2$ in Formula (1) in the direction perpendicular to the core represented by Formula (1a),
$\beta_3$ is a size of substituent $R_1$ in Formula (1) in the direction perpendicular to the core represented by Formula (1a),
$\beta_4$ is a size of substituent $R_2$ in Formula (1) in the direction perpendicular to the core represented by Formula (1a),
$\beta_5$ is a size of substituent $R_3$ in Formula (1) in the direction perpendicular to the core represented by Formula (1a), and
$\beta_6$ is a size of substituent $R_4$ in Formula (1) in the direction perpendicular to the core represented by Formula (1a),

$$\beta_{sum}=\beta_1+\beta_2+\beta_3+\beta_4+\beta_5+\beta_6$$

Formula (1a)

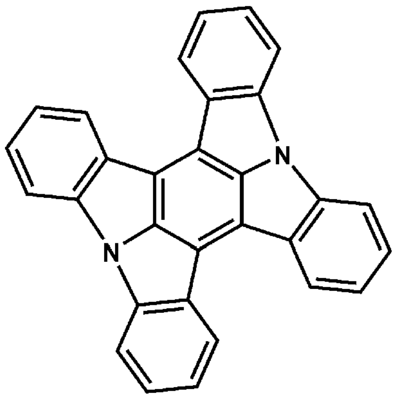

.

2. The blue luminescent compound of claim 1, wherein, in Formula (1), $R_1$ to $R_4$ are each independently an isopropyl group, an isobutyl group, a sec-butyl group, a tert-butyl group, an isopentyl group, a neopentyl group, a tert-pentyl group, a sec-isoamyl group, an isohexyl group, a neohexyl group, a 4-methylhexyl group, a 5-methylhexyl group, a 1-ethylhexyl group, a 2-ethylhexyl group, a 3-ethylhexyl group, a 4-ethylhexyl group, a 2-ethylpentyl group, a heptan-3-yl group, a heptan-4-yl group, a 4-methylhexan-2-yl group, a 3-methylhexan-3-yl group, a 2,3-dimethylpentan-2-yl group, a 2,4-dimethylpentan-2-yl group, a 4,4-dimethylpentan-2-yl group, a 6-methylheptyl group, a 2-ethylhexyl group, an octan-2-yl group, a 6-methylheptan-2-yl group, a 6-methyloctyl group, a 3,5,5-trimethylhexyl group, a nonan-4-yl group, a 2,6-dimethylheptan-3-yl group, a 3,6-dimethylheptan-3-yl group, a 3-ethylheptan-3-yl group, a 3,7-dimethyloctyl group, a 8-methylnonyl group, a 3-methylnonan-3-yl group, a 4-ethyloctan-4-yl group, a 9-methyldecyl group, an undecan-5-yl group, a 3-ethylnonan-3-yl group, a 5-ethylnonan-5-yl group, a 2,2,4,5,5-pentamethylhexan-4-yl group, a 10-methylundecyl group, a 11-methyldodecyl group, a tridecan-6-yl group, a tridecan-7-yl group, a 7-ethylundecan-2-yl group, a 3-ethylundecan-3-yl group, a 5-ethylundecan-5-yl group, a 12-methyltridecyl group, a 13-methyltetradecyl group, a pentadecan-7-yl group, a pentadecan-8-yl group, a 14-methylpentadecyl group, a 15-methylhexadecyl group, a heptadecan-8-yl group, a heptadecan-9-yl group, a 3,13-dimethylpentadecan-7-yl group, a 2,2,4,8,10,10-hexamethylundecan-5-yl group, a 16-methylheptadecyl group, a 17-methyloctadecyl group, a nonadecan-9-yl group, a nonadecan-10-yl group, a 2,6,10,14-tetramethylpentadecan-7-yl group, a 18-methylnonadecyl group, a cyclopropyl group, a cyclobutyl group, a cyclopentyl group, a cyclohexyl group, a cycloheptyl group, a cyclooctyl group, a cyclononyl group, a cyclodecyl group, a cycloundecyl group, a cyclododecyl group, a cyclotridecyl group, a cyclotetradecyl group, a cyclopentadecyl group, a cyclohexadecyl group, a cycloheptadecyl group, a cyclooctadecyl group, a cyclononadecyl group, a cycloeicosyl group, a trimethylsilyl group, a triethylsilyl group, a tri-iso-propylsilyl group, a tri-tert-butylsilyl group, a di-tert-butyl(methyl)silyl group, or a tert-butyldimethylsilyl group, each substituted or unsubstituted.

3. The blue luminescent compound of claims 1 or 2, wherein, in Formula (1), $Ar_1$ and $Ar_2$ are each independently a phenyl group, a biphenyl group, a naphthyl group, a fluorenyl group, an anthracenyl group, a phenanthrenyl group, a

thiophenyl group, a furanyl group, a pyranyl group, an imidazonyl group, a thiazonyl group, an oxazonyl group, an oxadiazonyl group, a pyridyl group, a bipyridyl group, a pyrimidyl group, a triazinyl group, a triazolyl group, a pyridazinyl group, a pyridinyl group, a quinolinyl group, a quinazolinyl group, a quinoxalinyl group, a phenoxadinyl group, a phthalazinyl group, a pyridpyrimidinyl group, a pyridpyrazinyl group, a pyrazinopyrazinyl group, an isoquinolinyl group, an indolyl group, a carbazolyl group, a benzoxazolyl group, a benzimidazolyl group, a benzothiazolyl group, a benzothiophenyl group, a dibenzothiophenyl group, a thienothiophenyl group, a benzofuranyl group, a phenanthrolinyl group, a thiazolyl group, an isoxazolyl group, an oxadiazolyl group, a thiadiazolyl group, a phenothiazinyl group, a dibenzosilolyl group, a dibenzofuranyl group, or a xanthonyl group, each substituted or unsubstituted.

**4.** The blue luminescent compound of any of claims 1-3, wherein the blue luminescent compound is a compound represented by Formula (2):

Formula (2)

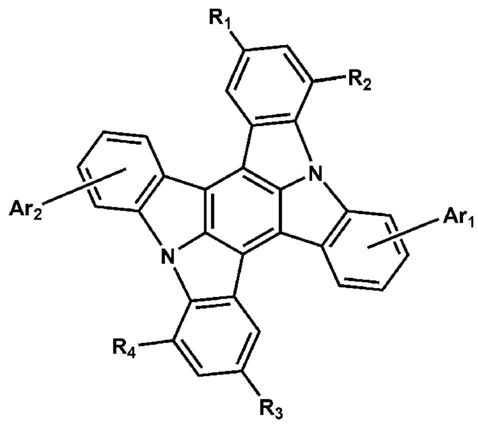

wherein, in Formula (2),

$R_1$ to $R_4$ are each independently a substituted or unsubstituted branched or cyclic alkyl group having 3 to 20 carbon atoms or a substituted or unsubstituted trialkylsilyl group having 3 to 60 carbon atoms, and
$Ar_1$ and $Ar_2$ are each independently a substituted or unsubstituted aromatic hydrocarbon cyclic group having 6 to 14 ring atoms or a substituted or unsubstituted aromatic heterocyclic group having 5 to 14 ring atoms;
preferably wherein the blue luminescent compound is a compound represented by Formula (3):

Formula (3)

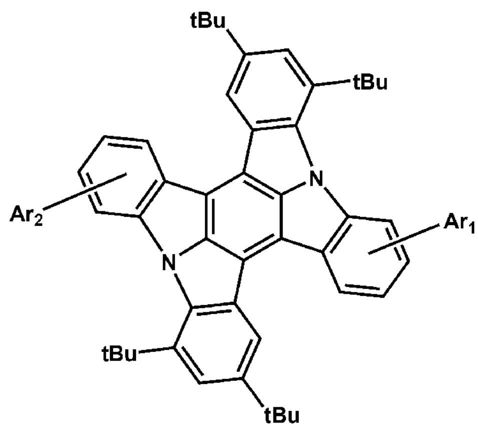

wherein, in Formula (3),
$Ar_1$ and $Ar_2$ are each independently a substituted or unsubstituted aromatic hydrocarbon cyclic group having 6 to 14 ring atoms or a substituted or unsubstituted aromatic heterocyclic group having 5 to 14 ring atoms, and

tBu represents a tert-butyl group.

5. The blue luminescent compound of any of claims 1-4, wherein a peak wavelength of a fluorescence spectrum in photoluminescence (PL) of the blue luminescent compound is 445 nm to 470 nm; and/or wherein a full width at half maximum of a peak of a fluorescence spectrum in photoluminescence of the blue luminescent compound is 20 nm or less.

6. The blue luminescent compound of any of claims 1-5, wherein the blue luminescent compound is one of Compounds 1 to 48:

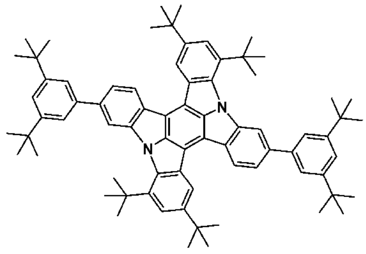
1

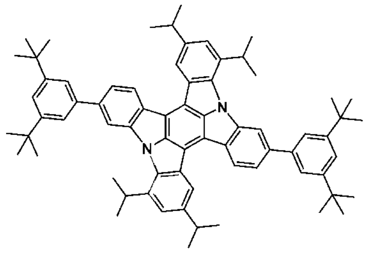
2

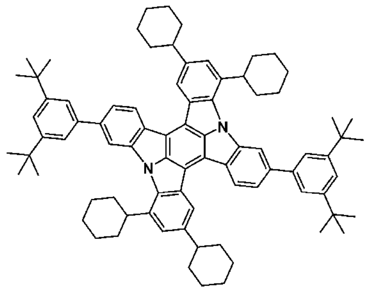
3

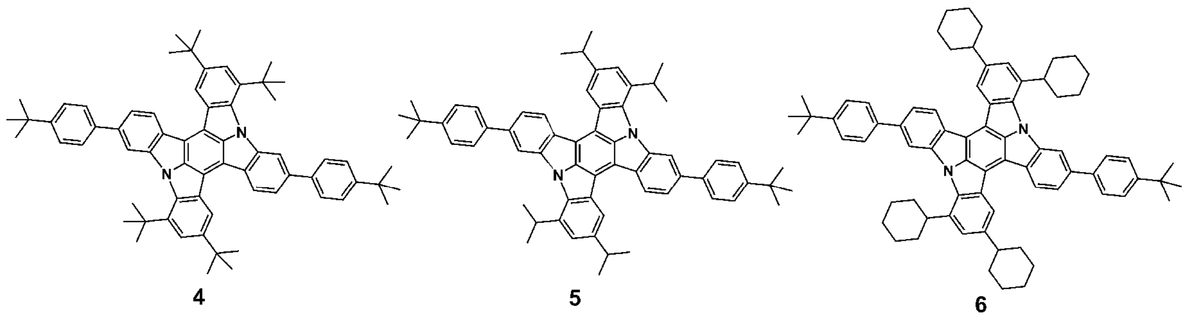
4

5

6

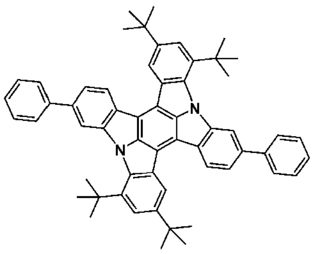
7

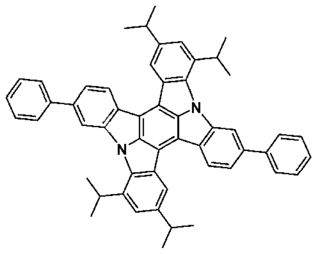
8

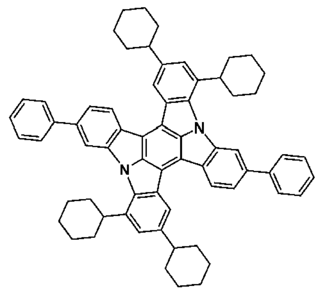
9

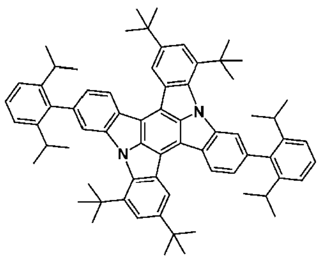
10

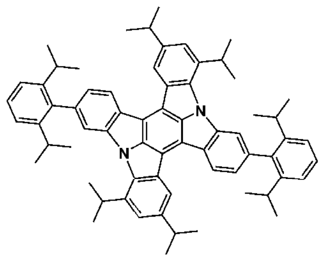
11

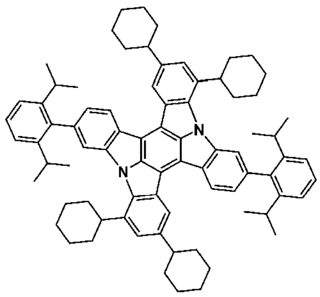
12

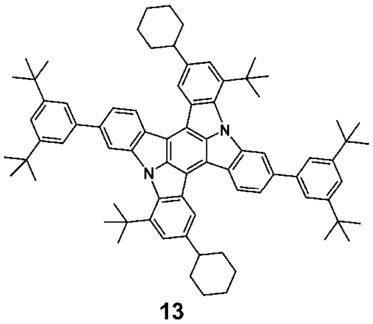

**13**

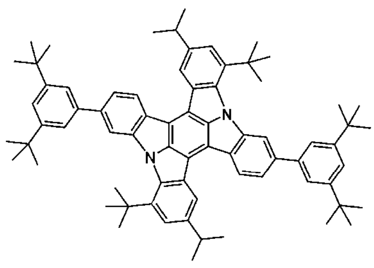

**14**

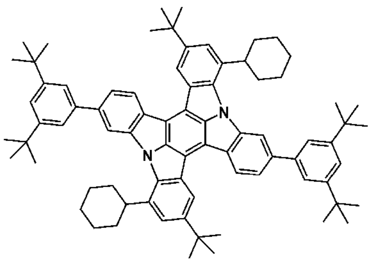

**15**

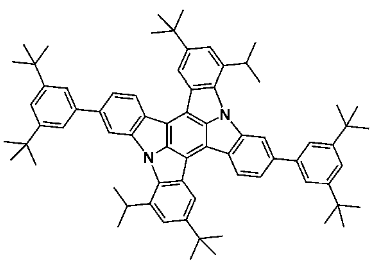

**16**

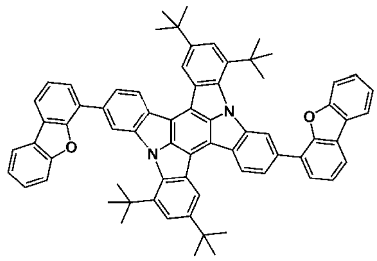

**17**

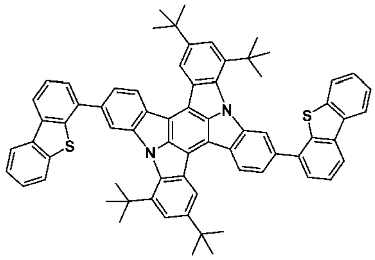

**18**

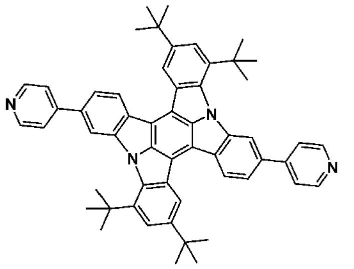

**19**

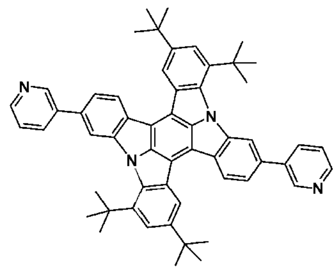

**20**

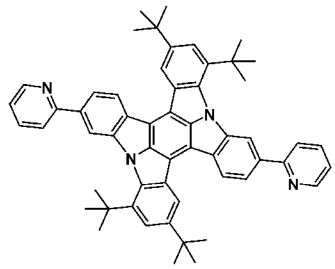

**21**

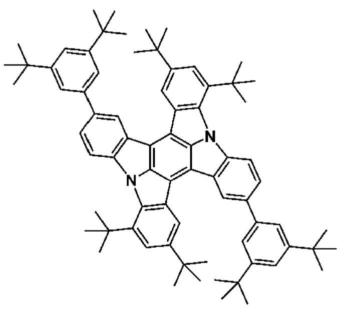

**22**

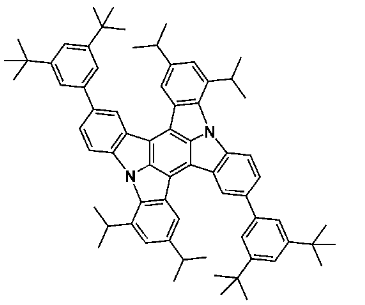

**23**

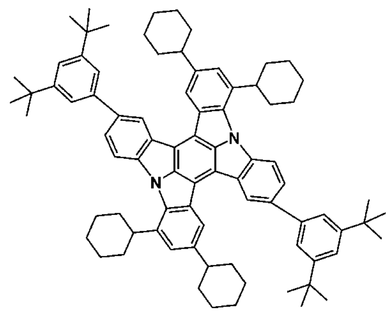

**24**

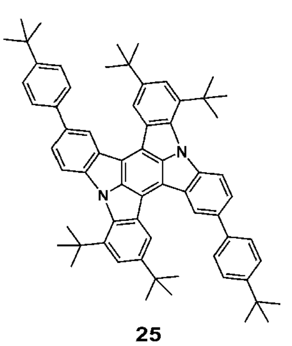

**25**

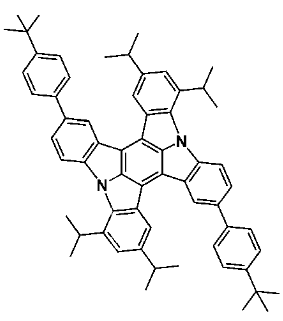

**26**

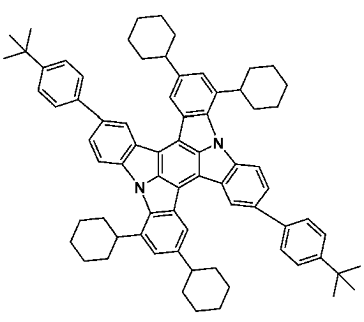

**27**

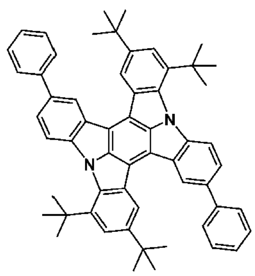
28

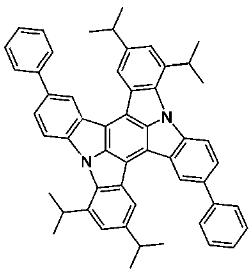
29

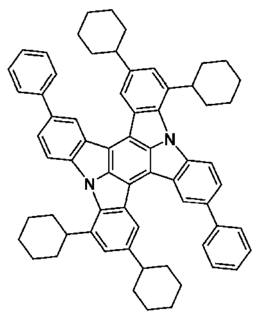
30

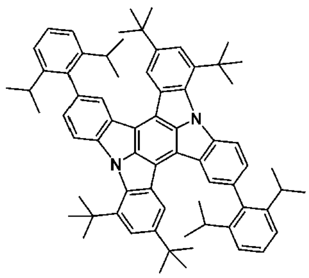
31

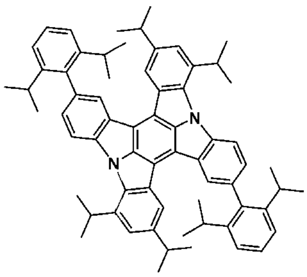
32

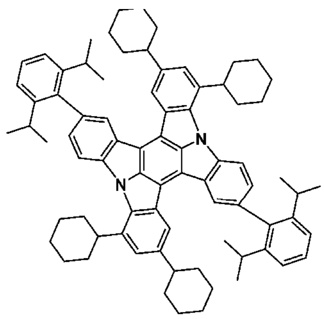
33

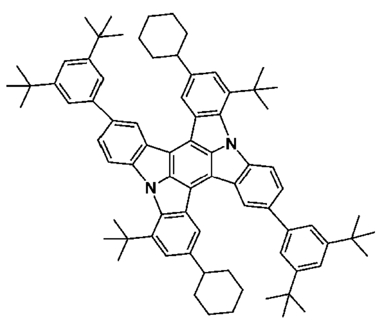
34

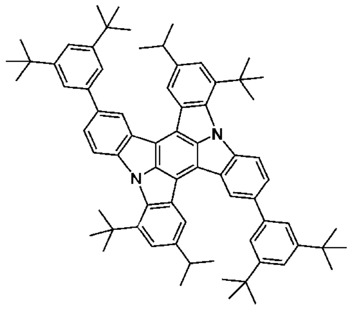
35

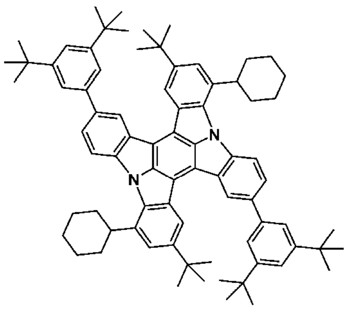
36

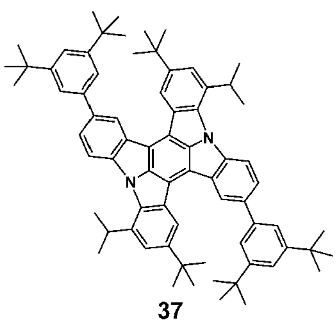
37

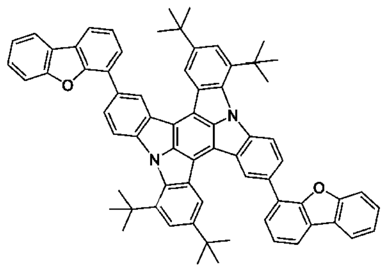
38

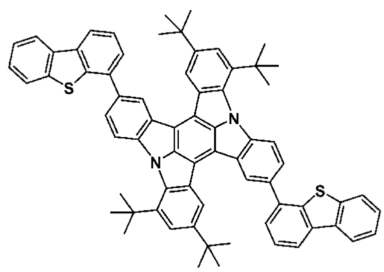
39

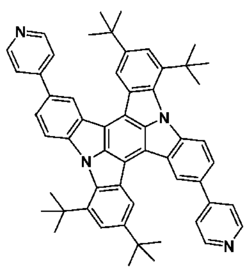

40

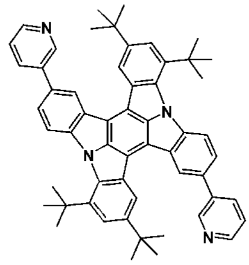

41

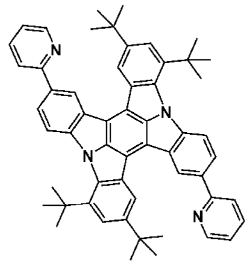

42

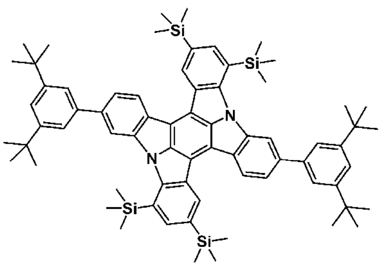

43

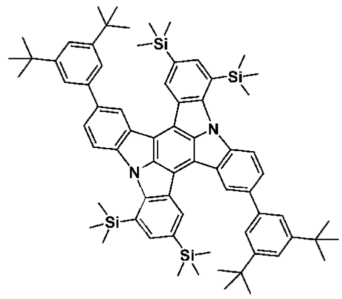

44

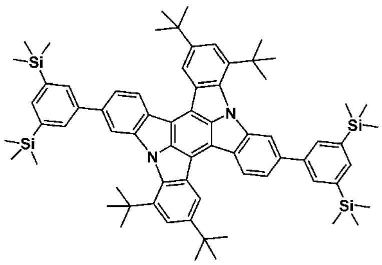

45

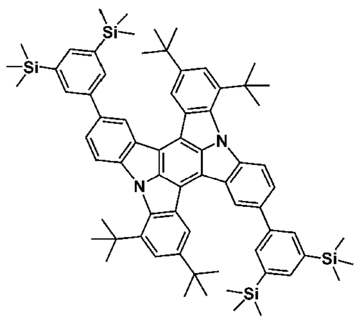

46

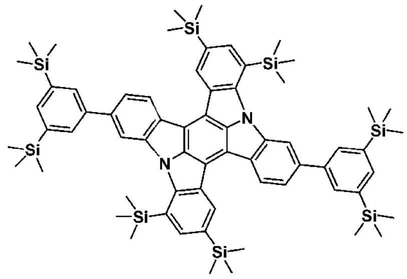

47

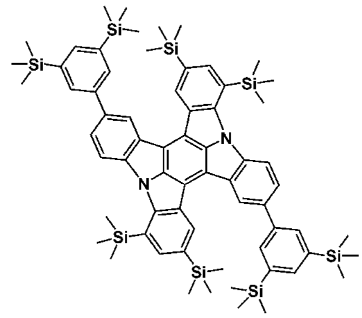

48

.

**7.** An organic light-emitting device comprising an emission layer comprising the blue luminescent compound according to any of claims 1-6.

**8.** The organic light-emitting device of claim 7, wherein the emission layer further comprises a phosphorescent complex.

**9.** The organic light-emitting device of claim 8, wherein the phosphorescent complex is a platinum complex; and/or

wherein the phosphorescent complex is a compound having a structure of Formula (4):

Formula (4)

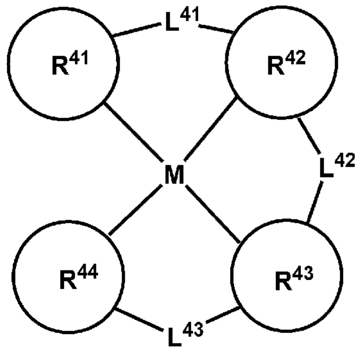

wherein, in Formula (4), M is a metal ion having a coordination number of 4,
$R^{41}$, $R^{42}$, $R^{43}$, and $R^{44}$ are each independently a substituted or unsubstituted hydrocarbon cyclic group or a substituted or unsubstituted heterocyclic group,
$L^{41}$ is a linking group linking $R^{41}$ and $R^{42}$,
$L^{42}$ is a linking group linking $R^{42}$ and $R^{43}$, and
$L^{43}$ is a linking group linking $R^{43}$ and $R^{44}$.

**10.** The organic light-emitting device of any of claims 7-9, wherein the emission layer further comprises a host material.

**11.** The organic light-emitting device of claim 10, wherein the host material comprises a compound having a triphenylsilyl group.

**12.** The organic light-emitting device of claims 10 or 11, wherein the host material comprises a compound having a carbazole ring structure, a compound having a ring structure in which at least one ring-forming carbon atom of a carbazole ring is substituted with a nitrogen atom, a compound having a triazine ring structure, a compound having an anthracene ring structure, or any combination thereof;

preferably wherein the compound having the carbazole ring structure or the compound having the ring structure in which at least one ring-forming carbon atom of a carbazole ring is substituted with a nitrogen atom is a compound having a structure represented by Formula (5):

Formula (5)

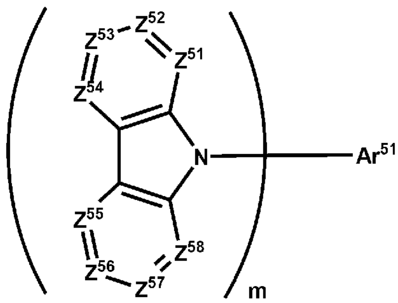

wherein, in Formula (5),
$Z^{51}$ is CH, $CR^{51}$, or N,
$Z^{52}$ is CH, $CR^{52}$, or N,
$Z^{53}$ is CH, $CR^{53}$, or N,
$Z^{54}$ is CH, $CR^{54}$, or N,
$Z^{55}$ is CH, $CR^{55}$, or N,
$Z^{56}$ is CH, $CR^{56}$, or N,
$Z^{57}$ is CH, $CR^{57}$, or N,
$Z^{58}$ is CH, $CR^{58}$, or N,
$R^{51}$ to $R^{58}$ are each independently a group from (5a) to (5h):
(5a) a cyano group;

(5b) a substituted or unsubstituted alkyl group having 1 to 20 carbon atoms;
(5c) a substituted or unsubstituted alkoxy group having 1 to 20 carbon atoms;
(5d) a substituted or unsubstituted arylamino group having 6 to 20 carbon atoms;
(5e) a substituted or unsubstituted phosphoryl group (a $-POH_2$ group);
(5f) a substituted or unsubstituted silyl group (a $-SiQ^1Q^2Q^3$ group);
(5g) a substituted or unsubstituted aromatic hydrocarbon group;
(5h) a substituted or unsubstituted heterocyclic group; and
$Ar^{51}$ is a group comprising at least one of the aromatic hydrocarbon group and the heterocyclic group, and
m is 1, 2, 3, 4, 5, or 6,
wherein $R^{51}$ and $R^{52}$, $R^{52}$ and $R^{53}$, $R^{53}$ and $R^{54}$, $R^{55}$ and $R^{56}$, $R^{56}$ and $R^{57}$, or $R^{57}$ and $R^{56}$ optionally form an aliphatic hydrocarbon ring, an aromatic hydrocarbon ring, or a hetero ring, each comprising bonded carbon atoms, and
wherein $Q^1$, $Q^2$, and $Q^3$ are independently hydrogen, a substituted or unsubstituted alkyl group having 1 to 20 carbon atoms, a substituted or unsubstituted aromatic hydrocarbon group, or a substituted or unsubstituted heterocyclic group.

**13.** The organic light-emitting device of any of claims 10-12, wherein the host material comprises a compound having a triazine ring structure;

preferably wherein the compound having the triazine ring structure has a structure represented by Formula (6):

Formula (6)

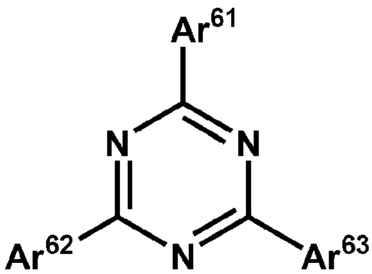

wherein, in Formula (6),
$Ar^{61}$ to $Ar^{63}$ are each independently a substituted or unsubstituted aromatic hydrocarbon group or a substituted or unsubstituted heterocyclic group.

**14.** The organic light-emitting device of any of claims 10-13, wherein the host material comprises a compound having an anthracene ring structure.

## Patentansprüche

**1.** Blau lumineszierende Verbindung, umfassend:

ein stickstoffhaltiges aromatisches heterocyclisches Gerüst; und
mindestens zwei aromatische Substituenten, die an das stickstoffhaltige aromatische heterocyclische Gerüst gebunden sind,
wobei das stickstoffhaltige aromatische heterocyclische Gerüst als Substituenten vier verzweigte oder cyclische Alkylgruppen mit jeweils 3 bis 20 Kohlenstoffatomen umfasst und sich das stickstoffhaltige aromatische heterocyclische Gerüst und die mindestens zwei aromatischen Substituenten nicht auf derselben Ebene befinden; und
wobei die blau lumineszierende Verbindung durch Formel (1) dargestellt ist, wobei $\beta_{Summe}$ wie nachstehend definiert größer als 13 und kleiner als 28 ist:

Formel (1)

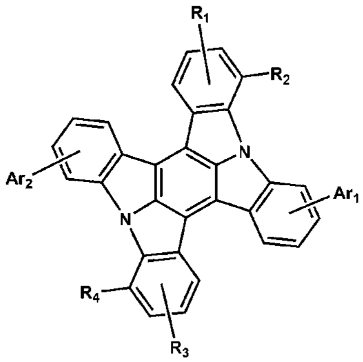

wobei in Formel (1)
$R_1$ bis $R_4$ jeweils unabhängig eine substituierte oder unsubstituierte verzweigte oder cyclische Alkylgruppe mit 3 bis 20 Kohlenstoffatomen oder eine substituierte oder unsubstituierte Trialkylsilylgruppe mit 3 bis 60 Kohlenstoffatomen sind und
$Ar_1$ und $Ar_2$ jeweils unabhängig eine substituierte oder unsubstituierte cyclische aromatische Kohlenwasserstoffgruppe mit 6 bis 14 Ringatomen oder eine substituierte oder unsubstituierte aromatische heterocyclische Gruppe mit 5 bis 14 Ringatomen sind:
$\beta_{Summe}$
wobei, wenn $\beta_1$ eine Größe des Substituenten $Ar_1$ in Formel (1) in einer Richtung senkrecht zu einem durch Formel (1a) dargestellten Kern ist,
$\beta_2$ eine Größe des Substituenten $Ar_2$ in Formel (1) in der Richtung senkrecht zu dem durch Formel (1a) dargestellten Kern ist,
$\beta_3$ eine Größe des Substituenten $R_1$ in Formel (1) in der Richtung senkrecht zu dem durch Formel (1a) dargestellten Kern ist,
$\beta_4$ eine Größe des Substituenten $R_2$ in Formel (1) in der Richtung senkrecht zu dem durch Formel (1a) dargestellten Kern ist,
$\beta_5$ eine Größe des Substituenten $R_3$ in Formel (1) in der Richtung senkrecht zu dem durch Formel (1a) dargestellten Kern ist und
$\beta_6$ eine Größe des Substituenten $R_4$ in Formel (1) in der Richtung senkrecht zu dem durch Formel (1a) dargestellten Kern ist,

$$\beta_{Summe}=\beta_1+\beta_2+\beta_3+\beta_4+\beta_5+\beta_6$$

Formel (1a)

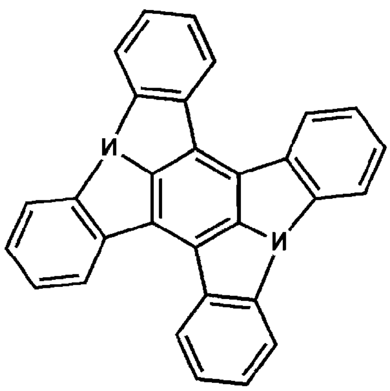

.

2. Blau lumineszierende Verbindung nach Anspruch 1, wobei in Formel (1) $R_1$ bis $R_4$ jeweils unabhängig eine Isopropylgruppe, eine Isobutylgruppe, eine sec-Butylgruppe, eine tert-Butylgruppe, eine Isopentylgruppe, eine Neopentylgruppe, eine tert-Pentylgruppe, eine sec-Isoamylgruppe, eine Isohexylgruppe, eine Neohexylgruppe,

eine 4-Methylhexylgruppe, eine 5-Methylhexylgruppe, eine 1-Ethylhexylgruppe, eine 2-Ethylhexylgruppe, eine 3-Ethylhexylgruppe, eine 4-Ethylhexylgruppe, eine 2-Ethylpentylgruppe, eine Heptan-3-ylgruppe, eine Heptan-4-ylgruppe, eine 4-Methylhexan-2-ylgruppe, eine 3-Methylhexan-3-ylgruppe, eine 2,3-Dimethylpentan-2-ylgruppe, eine 2,4-Dimethylpentan-2-ylgruppe, eine 4,4-Dimethylpentan-2-ylgruppe, eine 6-Methylheptylgruppe, eine 2-Ethylhexylgruppe, eine Octan-2-ylgruppe, eine 6-Methylheptan-2-ylgruppe, eine 6-Methyloctylgruppe, eine 3,5,5-Trimethylhexylgruppe, eine Nonan-4-ylgruppe, eine 2,6-Dimethylheptan-3-ylgruppe, eine 3,6-Dimethylheptan-3-ylgruppe, eine 3-Ethylheptan-3-ylgruppe, eine 3,7-Dimethyloctylgruppe, eine 8-Methylnonylgruppe, eine 3-Methylnonan-3-ylgruppe, eine 4-Ethyloctan-4-ylgruppe, eine 9-Methyldecylgruppe, eine Undecan-5-ylgruppe, eine 3-Ethylnonan-3-ylgruppe, eine 5-Ethylnonan-5-ylgruppe, eine 2,2,4,5,5-Pentamethylhexan-4-ylgruppe, eine 10-Methylundecylgruppe, eine 11-Methyldodecylgruppe, eine Tridecan-6-ylgruppe, eine Tridecan-7-ylgruppe, eine 7-Ethylundecan-2-ylgruppe, eine 3-Ethylundecan-3-ylgruppe, eine 5-Ethylundecan-5-ylgruppe, eine 12-Methyltridecylgruppe, eine 13-Methyltetradecylgruppe, eine Pentadecan-7-ylgruppe, eine Pentadecan-8-ylgruppe, eine 14-Methylpentadecylgruppe, eine 15-Methylhexadecylgruppe, eine Heptadecan-8-ylgruppe, eine Heptadecan-9-ylgruppe, eine 3,13-Dimethylpentadecan-7-ylgruppe, eine 2,2,4,8,10,10-Hexamethylundecan-5-ylgruppe, eine 16-Methylheptadecylgruppe, eine 17-Methyloctadecylgruppe, eine Nonadecan-9-ylgruppe, eine Nonadecan-10-ylgruppe, eine 2,6,10,14-Tetramethylpentadecan-7-ylgruppe, eine 18-Methylnonadecylgruppe, eine Cyclopropylgruppe, eine Cyclobutylgruppe, eine Cyclopentylgruppe, eine Cyclohexylgruppe, eine Cycloheptylgruppe, eine Cyclooctylgruppe, eine Cyclononylgruppe, eine Cyclodecylgruppe, eine Cycloundecylgruppe, eine Cyclododecylgruppe, eine Cyclotridecylgruppe, eine Cyclotetradecylgruppe, eine Cyclopentadecylgruppe, eine Cyclohexadecylgruppe, eine Cycloheptadecylgruppe, eine Cyclooctadecylgruppe, eine Cyclononadecylgruppe, eine Cycloeicosylgruppe, eine Trimethylsilylgruppe, eine Triethylsilylgruppe, eine Tri-isopropylsilylgruppe, eine Tri-tert-butylsilylgruppe, eine Di-tert-butyl(methyl)silylgruppe oder eine tert-Butyldimethylsilylgruppe sind, jeweils substituiert oder unsubstituiert.

**3.** Blau lumineszierende Verbindung nach Anspruch 1 oder 2, wobei in Formel (1) $Ar_1$ und $Ar_2$ jeweils unabhängig eine Phenylgruppe, eine Biphenylgruppe, eine Naphthylgruppe, eine Fluorenylgruppe, eine Anthracenylgruppe, eine Phenanthrenylgruppe, eine Thiophenylgruppe, eine Furanylgruppe, eine Pyranylgruppe, eine Imidazonylgruppe, eine Thiazonylgruppe, eine Oxazonylgruppe, eine Oxadiazonylgruppe, eine Pyridylgruppe, eine Bipyridylgruppe, eine Pyrimidylgruppe, eine Triazinylgruppe, eine Triazolylgruppe, eine Pyridazinylgruppe, eine Pyridinylgruppe, eine Chinolinylgruppe, eine Chinazolinylgruppe, eine Chinoxalinylgruppe, eine Phenoxadinylgruppe, eine Phthalazinylgruppe, eine Pyridpyrimidinylgruppe, eine Pyridpyrazinylgruppe, eine Pyrazinopyrazinylgruppe, eine Isochinolinylgruppe, eine Indolylgruppe, eine Carbazolylgruppe, eine Benzoxazolylgruppe, eine Benzimidazolylgruppe, eine Benzothiazolylgruppe, eine Benzothiophenylgruppe, eine Dibenzothiophenylgruppe, eine Thienothiophenylgruppe, eine Benzofuranylgruppe, eine Phenanthrolinylgruppe, eine Thiazolylgruppe, eine Isoxazolylgruppe, eine Oxadiazolylgruppe, eine Thiadiazolylgruppe, eine Phenothiazinylgruppe, eine Dibenzosilolylgruppe, eine Dibenzofuranylgruppe oder eine Xanthonylgruppe sind, jeweils substituiert oder unsubstituiert.

**4.** Blau lumineszierende Verbindung nach einem der Ansprüche 1-3, wobei die blau lumineszierende Verbindung eine durch Formel (2) dargestellte Verbindung ist:

Formel (2)

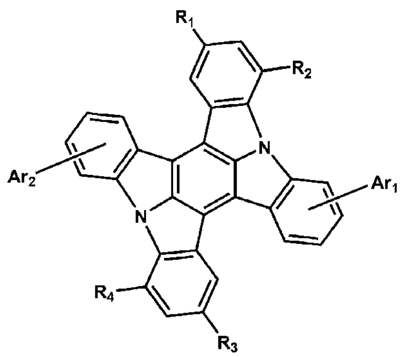

wobei in Formel (2)

$R_1$ bis $R_4$ jeweils unabhängig eine substituierte oder unsubstituierte verzweigte oder cyclische Alkylgruppe mit 3 bis 20 Kohlenstoffatomen oder eine substituierte oder unsubstituierte Trialkylsilylgruppe mit 3 bis 60 Kohlen-

stoffatomen sind und
$Ar_1$ und $Ar_2$ jeweils unabhängig eine substituierte oder unsubstituierte cyclische aromatische Kohlenwasserstoffgruppe mit 6 bis 14 Ringatomen oder eine substituierte oder unsubstituierte aromatische heterocyclische Gruppe mit 5 bis 14 Ringatomen sind;
wobei bevorzugt die blau lumineszierende Verbindung eine durch Formel (3) dargestellte Verbindung ist:

Formel (3)

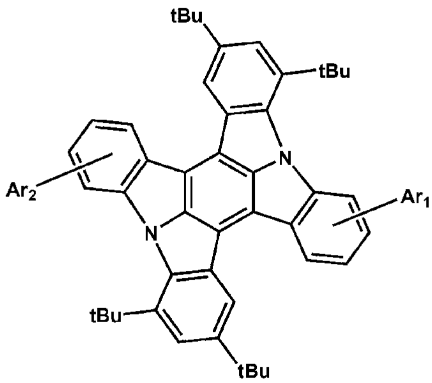

wobei in Formel (3)
$Ar_1$ und $Ar_2$ jeweils unabhängig eine substituierte oder unsubstituierte cyclische aromatische Kohlenwasserstoffgruppe mit 6 bis 14 Ringatomen oder eine substituierte oder unsubstituierte aromatische heterocyclische Gruppe mit 5 bis 14 Ringatomen sind und
tBu eine tert-Butylgruppe darstellt.

**5.** Blau lumineszierende Verbindung nach einem der Ansprüche 1-4, wobei eine Peak-Wellenlänge eines Fluoreszenzspektrums bei Photolumineszenz (PL) der blau lumineszierenden Verbindung 445 nm bis 470 nm beträgt; und/oder wobei eine Halbwertsbreite eines Peaks eines Fluoreszenzspektrums bei Photolumineszenz der blau lumineszierenden Verbindung 20 nm oder weniger beträgt.

**6.** Blau lumineszierende Verbindung nach einem der Ansprüche 1-5, wobei die blau lumineszierende Verbindung eine der Verbindungen 1 bis 48 ist:

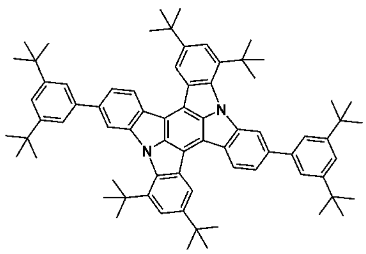

1

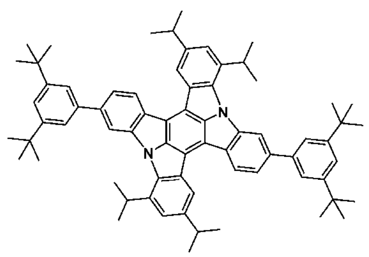

2

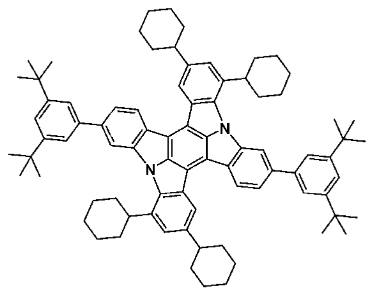

3

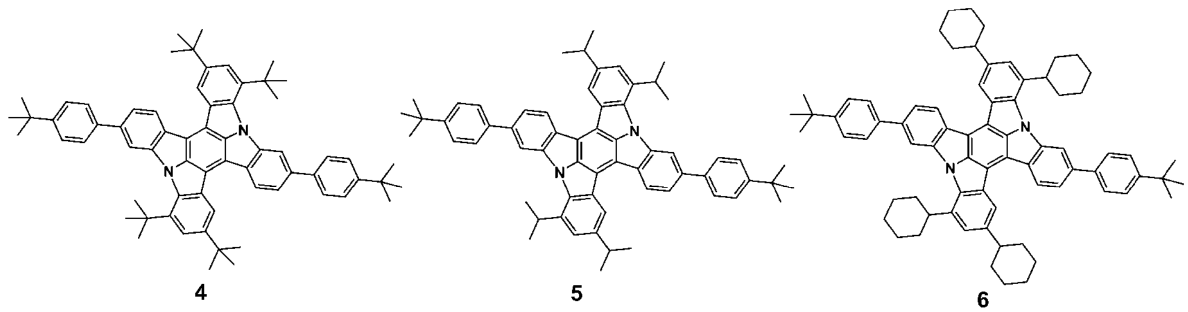

4

5

6

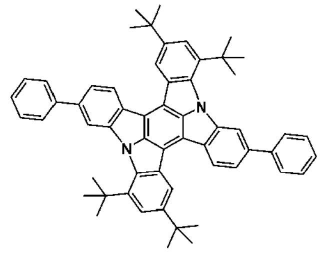
7

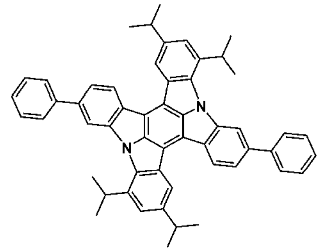
8

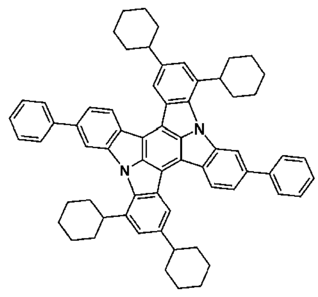
9

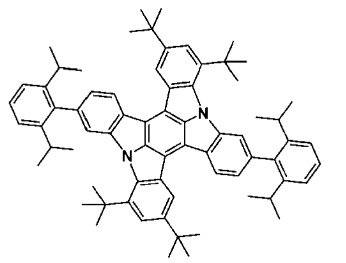
10

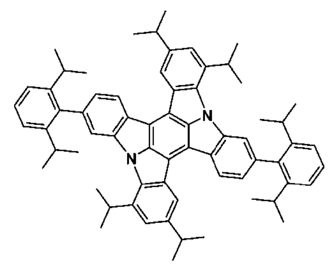
11

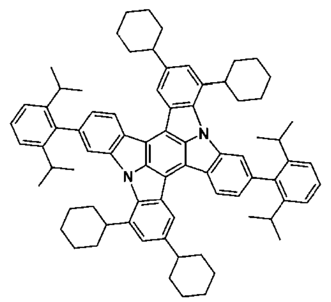
12

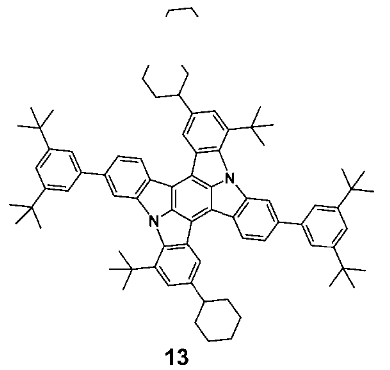
13

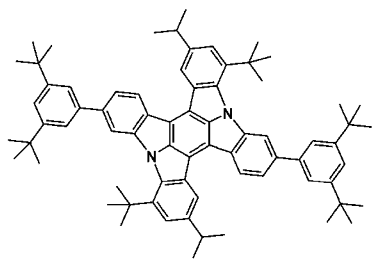
14

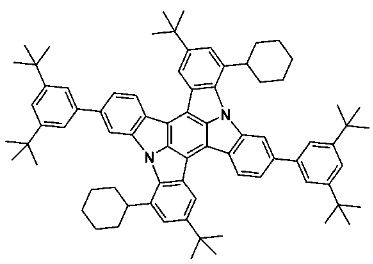
15

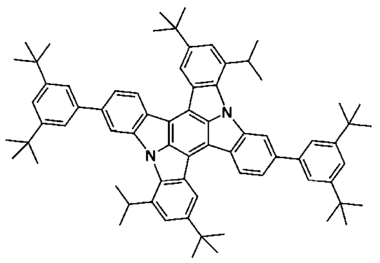
16

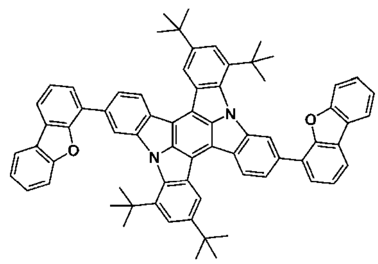
17

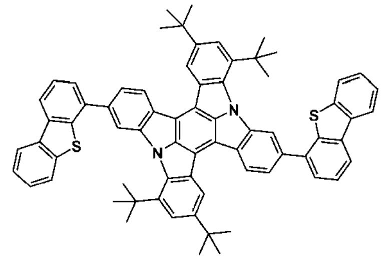
18

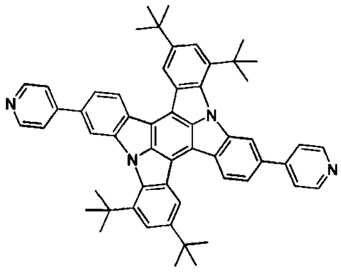
19

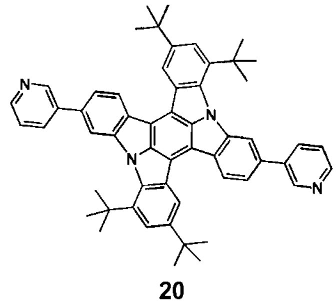
20

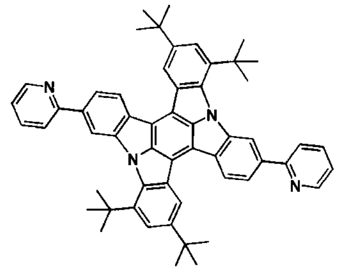
21

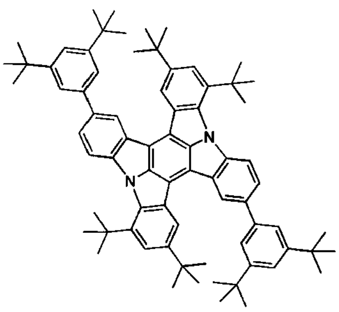

22

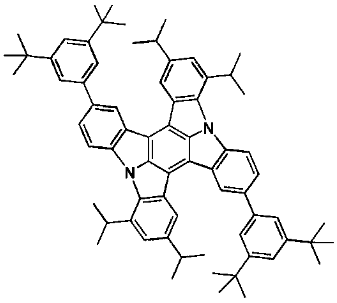

23

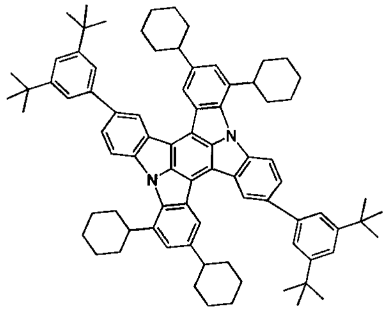

24

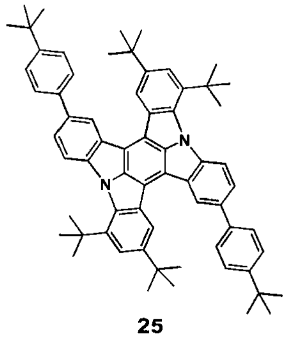

25

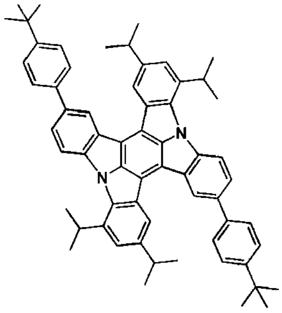

26

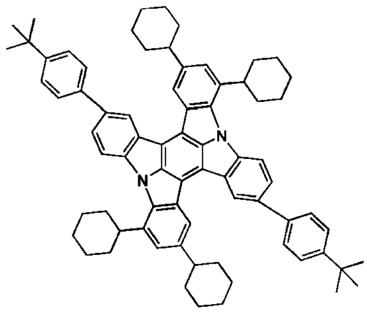

27

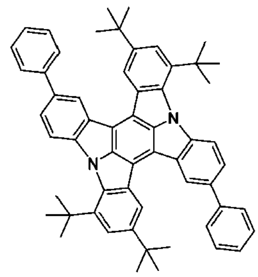

28

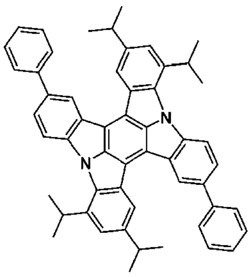

29

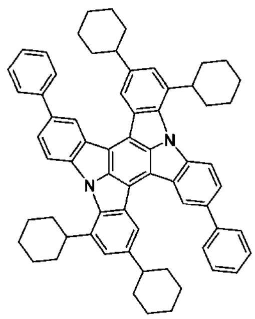

30

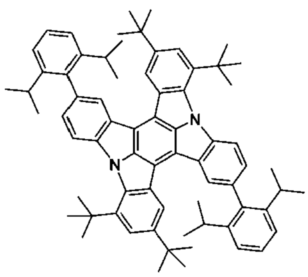

31

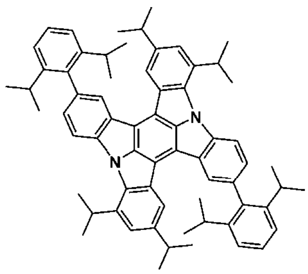

32

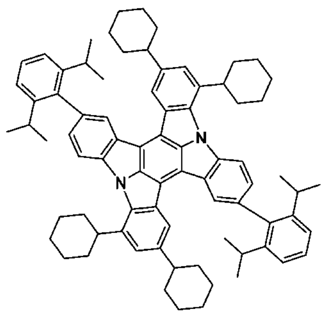

33

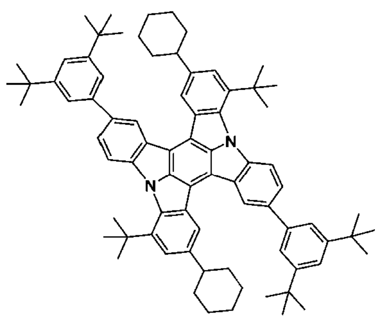

34

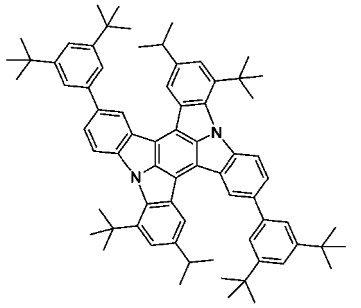

35

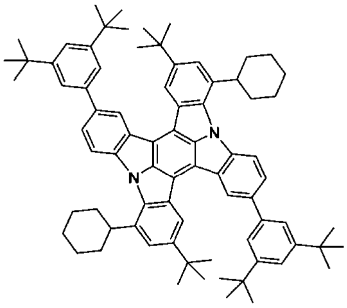

36

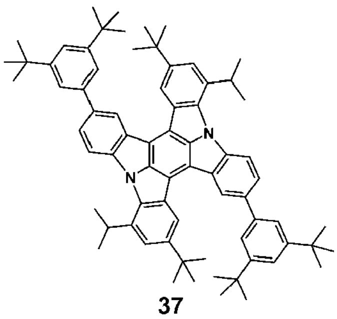

37

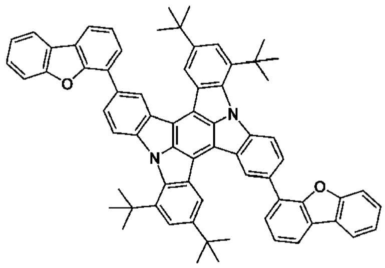

38

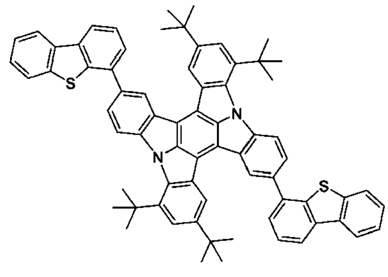

39

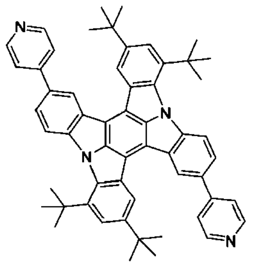

40

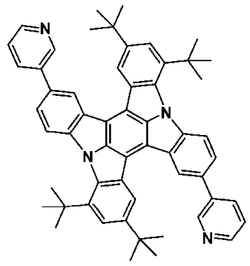

41

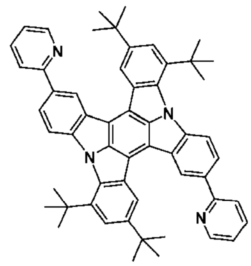

42

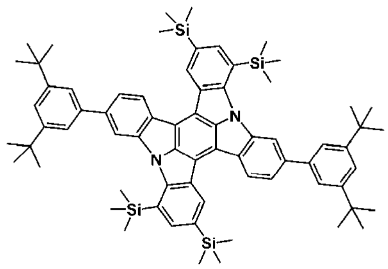

43

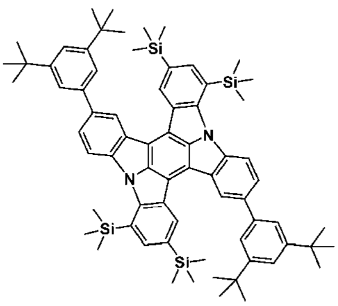

44

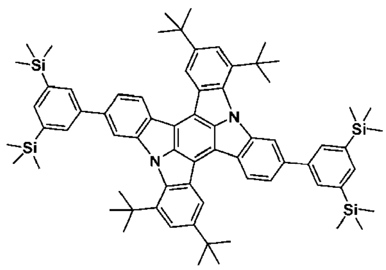

45

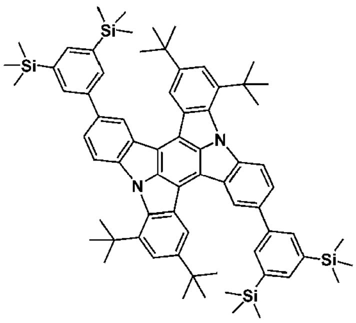

46

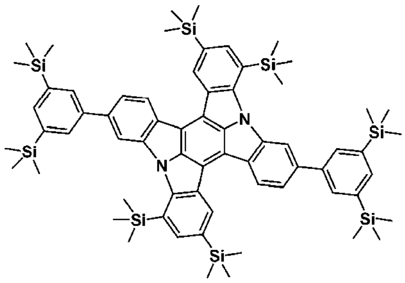

47

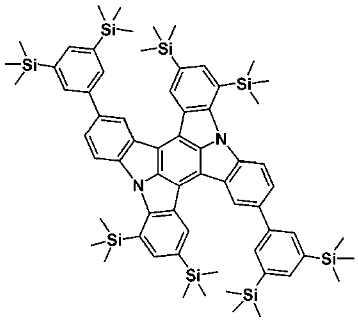

48

.

**7.** Organische lichtemittierende Vorrichtung, umfassend eine Emissionsschicht, umfassend die blau lumineszierende Verbindung nach einem der Ansprüche 1-6.

**8.** Organische lichtemittierende Vorrichtung nach Anspruch 7, wobei die Emissionsschicht ferner einen phosphoreszierenden Komplex umfasst.

**9.** Organische lichtemittierende Vorrichtung nach Anspruch 8, wobei der phosphoreszierende Komplex ein Platinkomplex ist; und/oder

wobei der phosphoreszierende Komplex eine Verbindung mit einer Struktur der Formel (4) ist:

Formel (4)

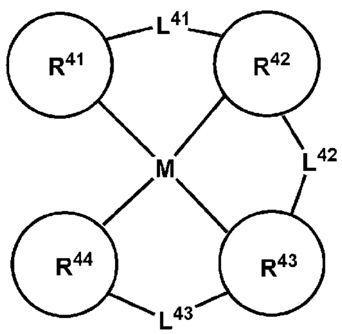

wobei in Formel (4) M ein Metallion mit einer Koordinationszahl von 4 ist,
$R^{41}$, $R^{42}$, $R^{43}$ und $R^{44}$ jeweils unabhängig eine substituierte oder unsubstituierte cyclische Kohlenwasserstoffgruppe oder eine substituierte oder unsubstituierte heterocyclische Gruppe sind,
$L^{41}$ eine Verknüpfungsgruppe ist, die $R^{41}$ und $R^{42}$ verknüpft,
$L^{42}$ eine Verknüpfungsgruppe ist, die $R^{42}$ und $R^{43}$ verknüpft, und
$L^{43}$ eine Verknüpfungsgruppe ist, die $R^{43}$ und $R^{44}$ verknüpft.

**10.** Organische lichtemittierende Vorrichtung nach einem der Ansprüche 7-9, wobei die Emissionsschicht ferner ein Wirtsmaterial umfasst.

**11.** Organische lichtemittierende Vorrichtung nach Anspruch 10, wobei das Wirtsmaterial eine Verbindung mit einer Triphenylsilylgruppe umfasst.

**12.** Organische lichtemittierende Vorrichtung nach Anspruch 10 oder 11, wobei das Wirtsmaterial eine Verbindung mit einer Carbazolringstruktur, eine Verbindung mit einer Ringstruktur, in der mindestens ein ringbildendes Kohlenstoffatom eines Carbazolrings mit einem Stickstoffatom substituiert ist, eine Verbindung mit einer Triazinringstruktur, eine Verbindung mit einer Anthracenringstruktur oder eine beliebige Kombination davon umfasst;

wobei bevorzugt die Verbindung mit der Carbazolringstruktur oder die Verbindung mit der Ringstruktur, in der mindestens ein ringbildendes Kohlenstoffatom eines Carbazolrings mit einem Stickstoffatom substituiert ist, eine Verbindung mit einer durch Formel (5) dargestellten Struktur ist:

Formel (5)

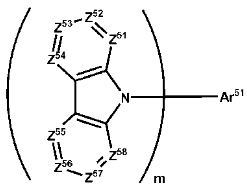

wobei in Formel (5)
$Z^{51}$ CH, $CR^{51}$ oder N ist,
$Z^{52}$ CH, $CR^{52}$ oder N ist,
$Z^{53}$ CH, $CR^{53}$ oder N ist,
$Z^{54}$ CH, $CR^{54}$ oder N ist,
$Z^{55}$ CH, $CR^{55}$ oder N ist,
$Z^{56}$ CH, $CR^{56}$ oder N ist,
$Z^{51}$ CH, $CR^{57}$ oder N ist,
$Z^{51}$ CH, $CR^{58}$ oder N ist,
$R^{51}$ bis $R^{58}$ jeweils unabhängig eine Gruppe aus (5a) bis (5h) sind:
(5a) eine Cyanogruppe;
(5b) eine substituierte oder unsubstituierte Alkylgruppe mit 1 bis 20 Kohlenstoffatomen;
(5c) eine substituierte oder unsubstituierte Alkoxygruppe mit 1 bis 20 Kohlenstoffatomen;
(5d) eine substituierte oder unsubstituierte Arylaminogruppe mit 6 bis 20 Kohlenstoffatomen; (5e) eine substituierte oder unsubstituierte Phosphorylgruppe (eine $-POH_2$-Gruppe);
(5f) eine substituierte oder unsubstituierte Silylgruppe (eine $-SiQ^1Q^2Q^3$-Gruppe);
(5g) eine substituierte oder unsubstituierte aromatische Kohlenwasserstoffgruppe;
(5h) eine substituierte oder unsubstituierte heterocyclische Gruppe; und
$Ar^{51}$ eine Gruppe ist, die mindestens eine der aromatischen Kohlenwasserstoffgruppe und der heterocyclischen Gruppe umfasst, und
m 1,2, 3, 4, 5 oder 6 ist,
wobei $R^{51}$ und $R^{52}$, $R^{52}$ und $R^{53}$, $R^{53}$ und $R^{54}$, $R^{55}$ und $R^{56}$, $R^{51}$ und $R^{57}$ oder $R^{57}$ und $R^{58}$ optional einen aliphatischen Kohlenwasserstoffring, einen aromatischen Kohlenwasserstoffring oder einen Heteroring bilden, die jeweils gebundene Kohlenstoffatome umfassen, und
wobei $Q^1$, $Q^2$ und $Q^3$ unabhängig Wasserstoff, eine substituierte oder unsubstituierte Alkylgruppe mit 1 bis 20 Kohlenstoffatomen, eine substituierte oder unsubstituierte aromatische Kohlenwasserstoffgruppe oder eine substituierte oder unsubstituierte heterocyclische Gruppe sind.

**13.** Organische lichtemittierende Vorrichtung nach einem der Ansprüche 10-12, wobei das Wirtsmaterial eine Verbindung mit einer Triazinringstruktur umfasst;

wobei bevorzugt die Verbindung mit der Triazinringstruktur eine durch Formel (6) dargestellte Struktur aufweist:

Formel (6)

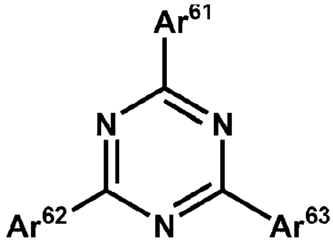

wobei in Formel (6)

$Ar^{61}$ bis $Ar^{63}$ jeweils unabhängig eine substituierte oder unsubstituierte aromatische Kohlenwasserstoffgruppe oder eine substituierte oder unsubstituierte heterocyclische Gruppe sind.

**14.** Organische lichtemittierende Vorrichtung nach einem der Ansprüche 10-13, wobei das Wirtsmaterial eine Verbindung mit einer Anthracenringstruktur umfasst.

**Revendications**

**1.** Composé luminescent bleu comprenant :

un squelette hétérocyclique aromatique contenant de l'azote ; et
au moins deux substituants aromatiques liés au squelette hétérocyclique aromatique contenant de l'azote,
ledit squelette hétérocyclique aromatique contenant de l'azote comprenant, en tant que substituants, quatre groupes alkyle ramifiés ou cycliques comportant chacun 3 à 20 atomes de carbone, et ledit squelette hétérocyclique aromatique contenant de l'azote et lesdits au moins deux substituants aromatiques n'étant pas situés dans le même plan ; et
ledit composé luminescent bleu étant représenté par la formule (1), $\beta_{somme}$ tel que défini ci-dessous étant supérieur à 13 et inférieur à 28 :

Formule (1)

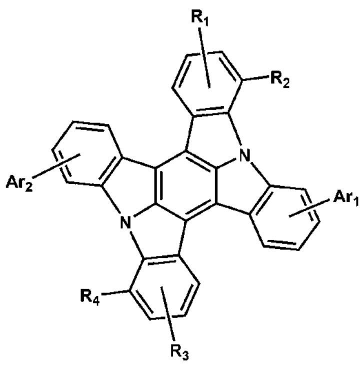

dans lequel, dans la Formule (1),
$R_1$ à $R_4$ représentent chacun indépendamment un groupe alkyle ramifié ou cyclique substitué ou non substitué comportant 3 à 20 atomes de carbone ou un groupe trialkylsilyle substitué ou non substitué comportant 3 à 60 atomes de carbone, et
$Ar_1$ et $Ar_2$ représentent chacun indépendamment un groupe cyclique hydrocarboné aromatique substitué ou non substitué comportant 6 à 14 atomes cycliques ou un groupe hétérocyclique aromatique substitué ou non substitué comportant 5 à 14 atomes cycliques :
$\beta_{somme}$
dans lequel, lorsque $B_1$ représente une taille du substituant $Ar_1$ dans la formule (1) dans une direction perpendiculaire à un noyau représenté par la formule (1a),
$\beta_2$ représente une taille du substituant $Ar_2$ dans la formule (1) dans la direction perpendiculaire au noyau représenté par la formule (1a),
$\beta_3$ représente une taille du substituant $R_1$ dans la formule (1) dans la direction perpendiculaire au noyau représentée par la formule (1a),
$\beta_4$ représente une taille du substituant $R_2$ dans la formule (1) dans la direction perpendiculaire au noyau représenté par la Formule (1a),
$\beta_5$ représente une taille du substituant $R_3$ dans la formule (1) dans la direction perpendiculaire au noyau représenté par la formule (1a), et
$\beta_6$ représente une taille du substituant $R_4$ dans la formule (1) dans la direction perpendiculaire au noyau représenté par la formule (1a),

$$\beta_{somme} = \beta_1 + \beta_2 + \beta_3 + \beta_4 + \beta_5 + \beta_6$$

Formule (1A)

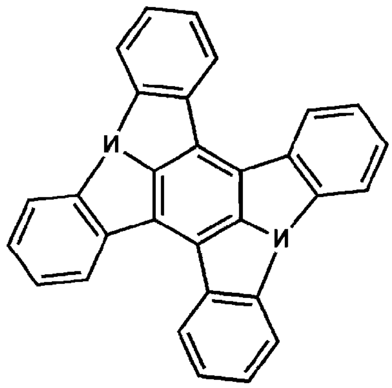

.

**2.** Composé luminescent bleu selon la revendication 1, dans la formule (1), $R_1$ à $R_4$ représentant chacun indépendamment un groupe isopropyle, un groupe isobutyle, un groupe sec-butyle, un groupe tert-butyle, un groupe isopentyle, un groupe néopentyle, un groupe tert-pentyle, un groupe sec-isoamyle, un groupe isohexyle, un groupe néohexyle, un groupe 4-méthylhexyle, un groupe 5-méthylhexyle, un groupe 1-éthylhexyle, un groupe 2-éthylhexyle, un groupe 3-éthylhexyle, un groupe 4-éthylhexyle, un groupe 2-éthylpentyle, un groupe heptan-3-yle, un groupe heptan-4-yle, un groupe 4-méthylhexan-2-yle, un groupe 3-méthylhexan-3-yle, un groupe 2,3-diméthylpentan-2-yle, un groupe 2,4-diméthylpentan-2-yle, un groupe 4,4-diméthylpentan-2-yle, un groupe 6- méthylheptyle, un groupe 2-éthylhexyle, un groupe octan-2-yle, un groupe 6-méthylheptan-2-yle, un groupe 6-méthyloctyle, un groupe 3,5,5-triméthylhexyle, un groupe nonan-4-yle, un groupe 2,6-diméthylheptan-3-yle, un groupe 3,6-diméthylheptan-3-yle, un groupe 3-éthylheptan-3-yle, un groupe 3,7-diméthyloctyle, un groupe 8-méthylnonyle, un groupe 3-méthylnonan-3-yle, un groupe 4-éthyloctan-4-yle, un groupe 9-méthyldécyle, un groupe undécan-5-yle, un groupe 3-éthylnonan-3-yle, un groupe 5-éthylnonan-5-yle, un groupe 2,2,4,5,5-pentaméthylhexan-4-yle, un groupe 10-méthylundécyle, un groupe 11-méthyldodécyle, un groupe tridécan-6-yle, un groupe tridécan-7-yle, un groupe 7-éthylundécan-2-yle, un groupe 3-éthylundécan-3-yle, un groupe 5-éthylundécan-5-yle, un groupe 12-méthyltridécyle, un groupe 13-méthyltétradécyle, un groupe pentadécan-7-yle, un groupe pentadécan-8-yle, un groupe 14-méthylpentadécyle, un groupe 15-méthylhexadécyle, un groupe heptadécan-8-yle, un groupe heptadécane-9-yle, un groupe 3,13-diméthylpentadécan-7-yle, un groupe 2,2,4,8,10,10-hexaméthylundécan-5-yle, un groupe 16-méthylheptadécyle, un groupe 17-méthyloctadécyle, un groupe nonadécan-9-yle, un groupe nonadécan-10-yle, un groupe 2,6,10,14-tétraméthylpentadécan-7-yle, un groupe 18-méthylnonadécyle, un groupe cyclopropyle, un groupe cyclobutyle, un groupe cyclopentyle, un groupe cyclohexyle, un groupe cycloheptyle, un groupe cyclooctyle, un groupe cyclononyle, un groupe cyclodécyle, un groupe cycloundécyle, un groupe cyclodécyle, un groupe cyclotridécyle, un groupe cyclotétradécyle, un groupe cyclopentadécyle, un groupe cyclohexadécyle, un groupe cycloheptadécyle, un groupe cyclooctadécyle, un groupe cyclononadécyle, un groupe cycloéicosyle, un groupe triméthylsilyle, un groupe triéthylsilyle, un groupe tri-iso-propylsilyle, un groupe tri-tert-butylsilyle, un groupe di-tert-butyl(méthyl)silyle, ou un groupe tert-butyldiméthylsilyle, chacun substitué ou non substitué.

**3.** Composé luminescent bleu selon la revendication 1 ou 2, dans la formule (1), $Ar_1$ et $Ar_2$ représentant chacun indépendamment un groupe phényle, un groupe biphényle, un groupe naphtyle, un groupe fluorényle, un groupe anthracényle, un groupe phénanthrényle, un groupe thiophényle, un groupe furanyle, un groupe pyranyle, un groupe imidazonyle, un groupe thiazonyle, un groupe oxazonyle, un groupe oxadiazonyle, un groupe pyridyle, un groupe bipyridyle, un groupe pyrimidyle, un groupe triazinyle, un groupe triazolyle, un groupe pyridazinyle, un groupe pyridinyle, un groupe quinolinyle, un groupe quinazolinyle, un groupe quinoxalinyle, un groupe phénoxadinyle, un groupe phtalazinyle, un groupe pyridopyrimidinyle, un groupe pyridopyrazinyle, un groupe pyrazinopyrazinyle, un groupe isoquinolinyle, un groupe indolyle, un groupe carbazolyle, un groupe benzoxazolyle, un groupe benzimidazolyle, un groupe benzothiazolyle, un groupe benzothiophényle, un groupe dibenzothiophényle, un groupe thiénothiophényle, un groupe benzofuranyle, un groupe phénanthrolinyle, un groupe thiazolyle, un groupe isoxazolyle, un groupe oxadiazolyle, un groupe thiadiazolyle, un groupe phénothiazinyle, un groupe dibenzosilolyle, un groupe dibenzofuranyle ou un groupe xanthonyle, chacun substitué ou non substitué.

**4.** Composé luminescent bleu selon l'une quelconque des revendications 1 à 3, ledit composé luminescent bleu étant un composé représenté par la formule (2) :

Formule (2)

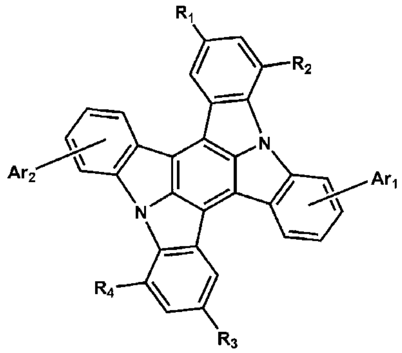

dans lequel, dans la Formule (2),

$R_1$ à $R_4$ représentent chacun indépendamment un groupe alkyle ramifié ou cyclique substitué ou non substitué comportant 3 à 20 atomes de carbone ou un groupe trialkylsilyle substitué ou non substitué comportant 3 à 60 atomes de carbone, et
$Ar_1$ et $Ar_2$ représentent chacun indépendamment un groupe cyclique hydrocarboné aromatique substitué ou non substitué comportant 6 à 14 atomes cycliques ou un groupe hétérocyclique aromatique substitué ou non substitué comportant 5 à 14 atomes cycliques ;
de préférence, ledit composé luminescent bleu étant un composé représenté par la Formule (3) :

Formule (3)

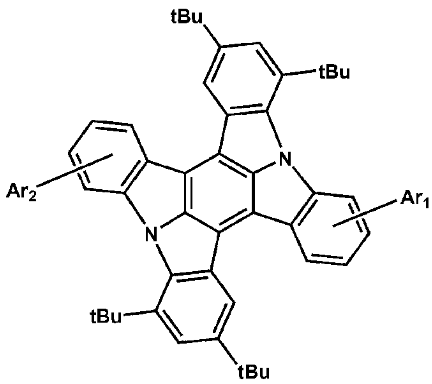

dans lequel, dans la Formule (3),
$Ar_1$ et $Ar_2$ représentent chacun indépendamment un groupe cyclique hydrocarboné aromatique substitué ou non substitué comportant 6 à 14 atomes cycliques ou un groupe hétérocyclique aromatique substitué ou non substitué comportant 5 à 14 atomes cycliques, et
tBu représente un groupe tert-butyle.

5. Composé luminescent bleu selon l'une quelconque des revendications 1 à 4, une longueur d'onde de crête d'un spectre de fluorescence en photoluminescence (PL) du composé luminescent bleu étant de 445 nm à 470 nm ; et/ou une largeur totale à mi-hauteur d'un pic d'un spectre de fluorescence en photoluminescence du composé luminescent bleu étant inférieure ou égale à 20 nm.

6. Composé luminescent bleu selon l'une quelconque des revendications 1 à 5, ledit composé luminescent bleu étant l'un des composés 1 à 48 :

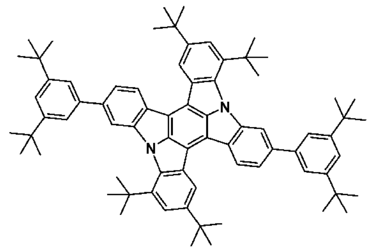

1

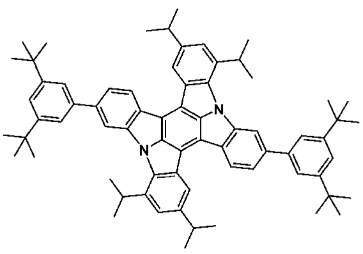

2

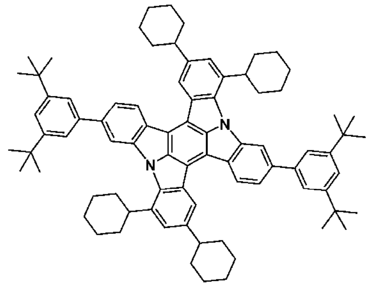

3

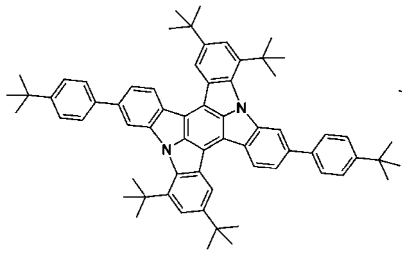

4

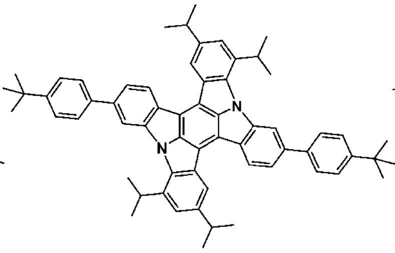

5

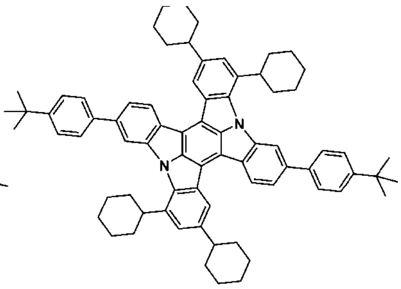

6

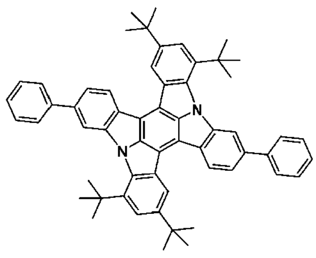

7

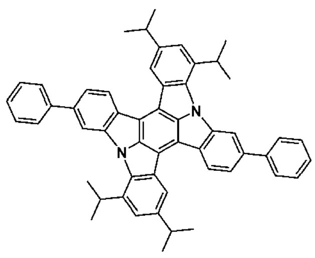

8

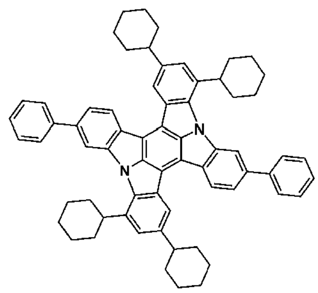

9

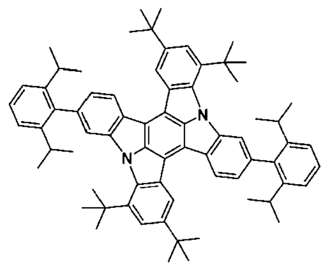

10

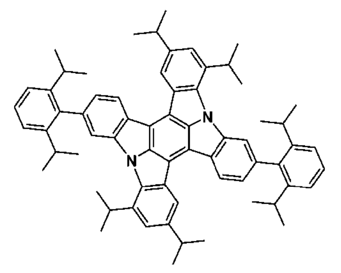

11

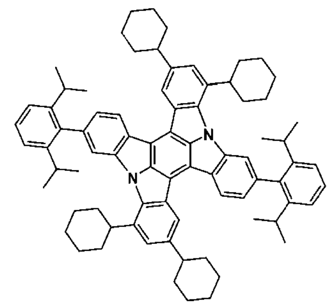

12

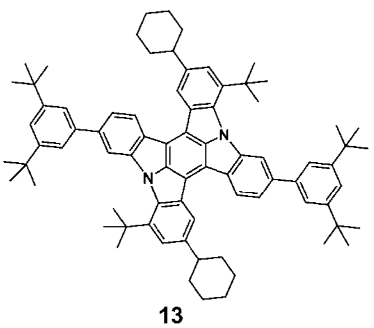

13

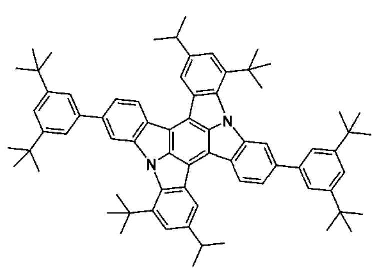

14

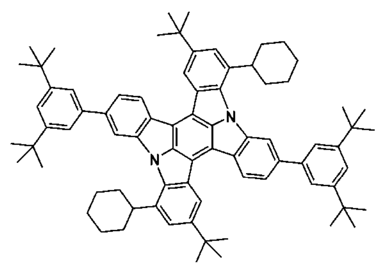

15

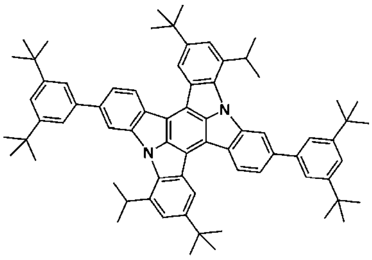

16

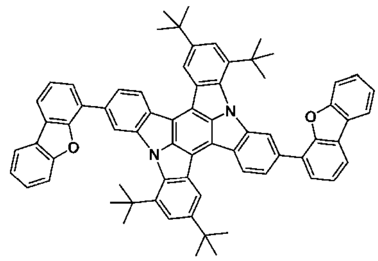

17

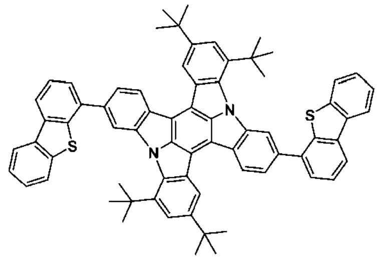

18

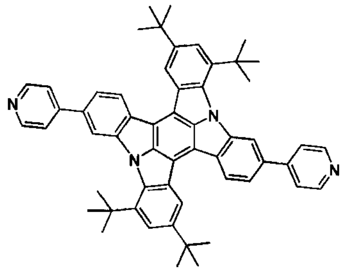

19

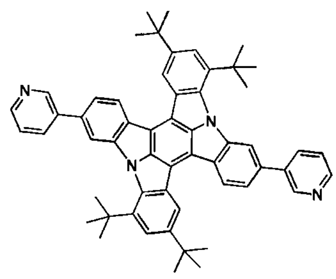

20

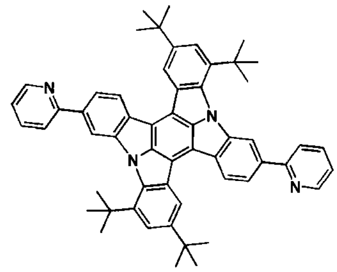

21

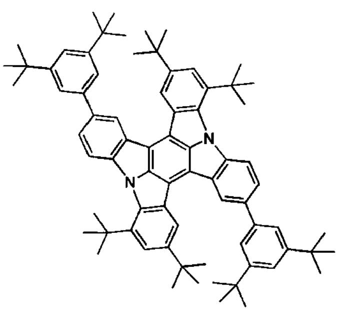

22

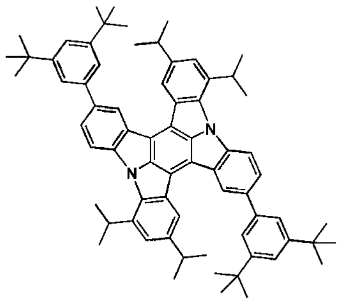

23

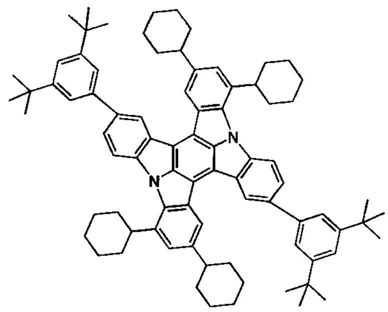

24

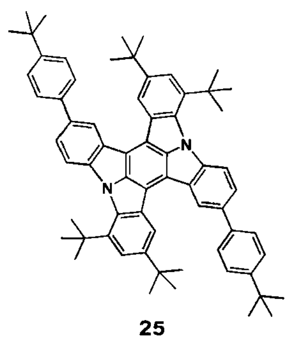

25

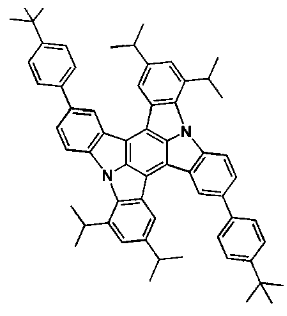

26

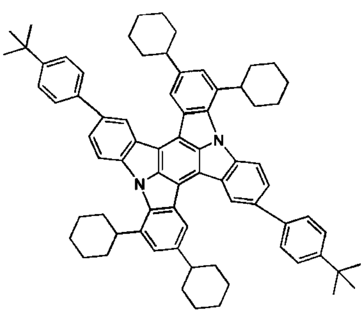

27

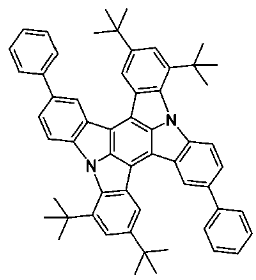

28

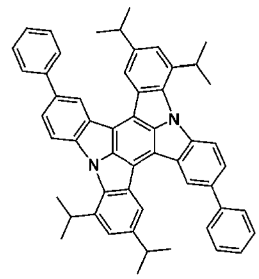

29

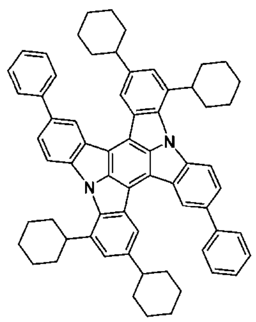

30

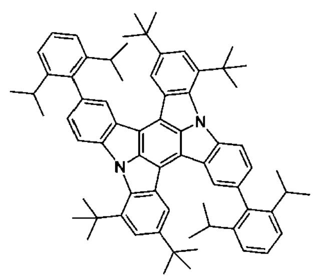

31

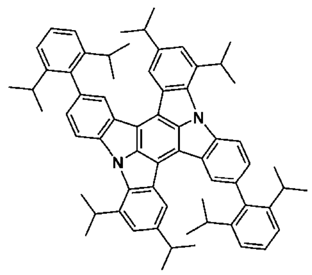

32

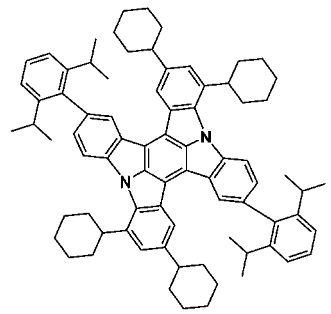

33

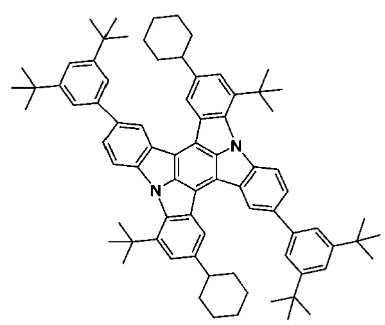

34

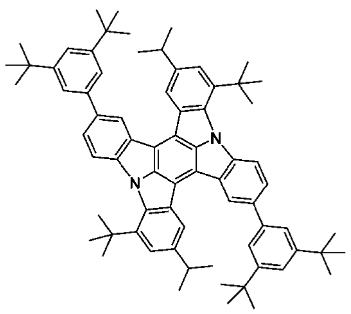

35

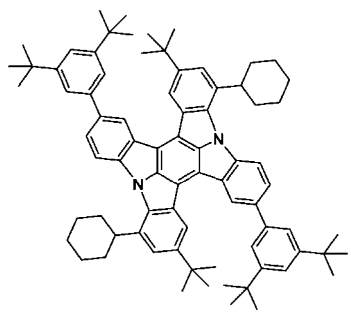

36

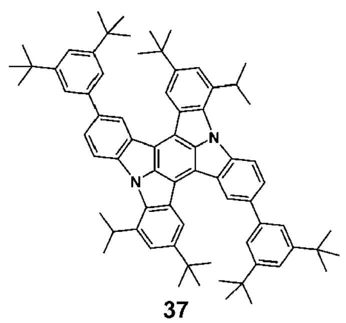

37

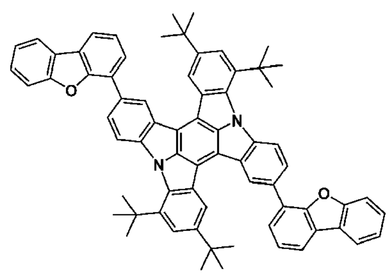

38

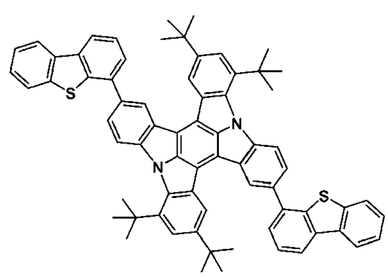

39

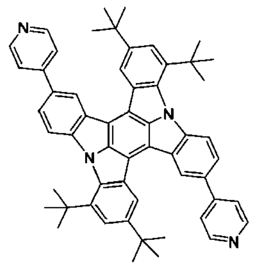

40

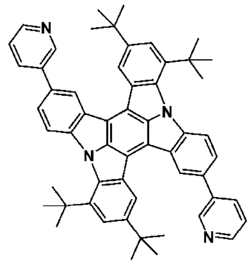

41

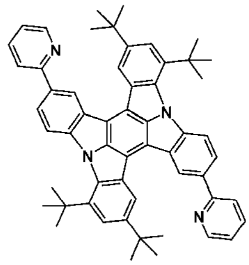

42

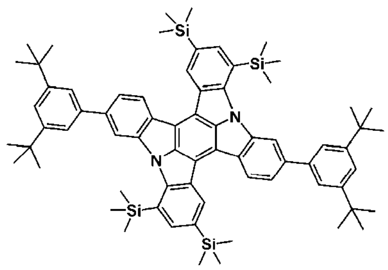

43

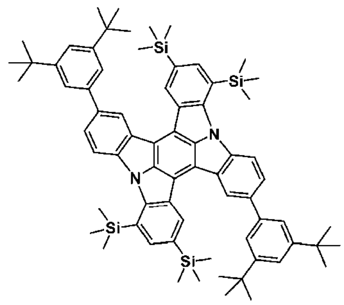

44

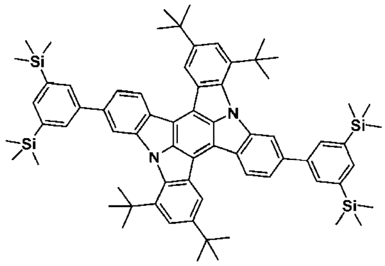

45

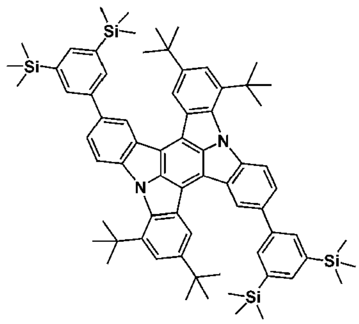

46

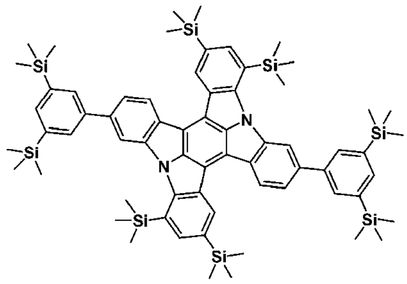

47

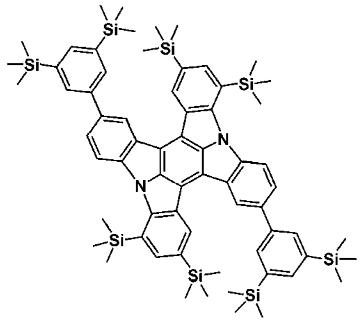

48

.

**7.** Dispositif électroluminescent organique comprenant une couche d’émission comprenant le composé luminescent bleu selon l’une quelconque des revendications 1 à 6.

**8.** Dispositif électroluminescent organique selon la revendication 7, ladite couche d’émission comprenant en outre un complexe phosphorescent.

**9.** Dispositif électroluminescent organique selon la revendication 8, ledit complexe phosphorescent étant un complexe de platine ; et/ou

ledit complexe phosphorescent étant un composé comportant une structure de formule (4) :

Formule (4)

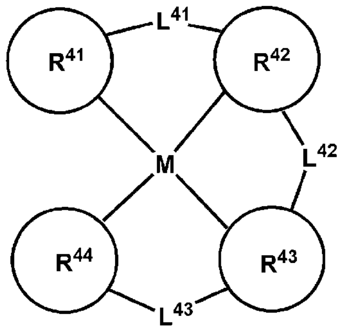

dans lequel, dans la Formule (4), M représente un ion métallique comportant un nombre de coordination de 4, $R^{41}$, $R^{42}$, $R^{43}$ et $R^{44}$ représentent chacun indépendamment un groupe cyclique hydrocarboné substitué ou non substitué ou un groupe hétérocyclique substitué ou non substitué,
$L^{41}$ représente un groupe de liaison liant $R^{41}$ et $R^{42}$,
$L^{42}$ représente un groupe de liaison liant $R^{42}$ et $R^{43}$, et
$L^{43}$ représente un groupe de liaison liant $R^{43}$ et $R^{44}$.

**10.** Dispositif électroluminescent organique selon l'une quelconque des revendications 7 à 9, ladite couche d'émission comprenant en outre un matériau hôte.

**11.** Dispositif électroluminescent organique selon la revendication 10, ledit matériau hôte comprenant un composé comportant un groupe triphénylsilyle.

**12.** Dispositif électroluminescent organique selon la revendication 10 ou 11, ledit matériau hôte comprenant un composé comportant une structure cyclique carbazole, un composé comportant une structure cyclique dans laquelle au moins un atome de carbone formant un cycle d'un cycle carbazole est substitué par un atome d'azote, un composé comportant une structure cyclique triazine, un composé comportant une structure cyclique anthracène, ou toute combinaison de ceux-ci ;

de préférence, ledit composé comportant la structure cyclique carbazole ou ledit composé comportant la structure cyclique dans laquelle au moins un atome de carbone formant un cycle d'un cycle carbazole est substitué par un atome d'azote étant un composé comportant une structure représentée par la formule (5) :

Formule (5)

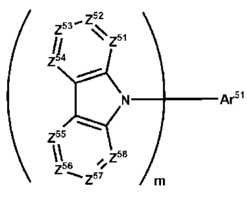

dans lequel, dans la Formule (5),
$Z^{51}$ représente un groupe CH, $CR^{51}$, ou un atome N,
$Z^{52}$ représente un groupe CH, $CR^{52}$ ou un atome N,
$Z^{53}$ représente un groupe CH, $CR^{53}$ ou un atome N,
$Z^{54}$ représente un groupe CH, $CR^{54}$, ou un atome N,
$Z^{55}$ représente un groupe CH, $CR^{55}$, ou un atome N,
$Z^{56}$ représente un groupe CH, $CR^{56}$, ou un atome N,
$Z^{57}$ représente un groupe CH, $CR^{57}$, ou un atome N,
$Z^{58}$ représente un groupe CH, $CR^{58}$ ou un atome N,
$R^{51}$ à $R^{58}$ représentent chacun indépendamment un groupe parmi (5a) à (5h) :
(5a) un groupe cyano ;

(5b) un groupe alkyle substitué ou non substitué comportant 1 à 20 atomes de carbone ;
(5c) un groupe alcoxy substitué ou non substitué comportant 1 à 20 atomes de carbone ;
(5d) un groupe arylamino substitué ou non substitué comportant 6 à 20 atomes de carbone ;
(5e) un groupe phosphoryle (un groupe $-POH_2$) substitué ou non substitué ;
(5f) un groupe silyle (un groupe $-SiQ^1Q^2Q^3$) substitué ou non substitué ;
(5g) un groupe hydrocarboné aromatique substitué ou non substitué ;
(5h) un groupe hétérocyclique substitué ou non substitué ; et
$Ar^{51}$ représente un groupe comprenant au moins un groupe parmi le groupe hydrocarboné aromatique et le groupe hétérocyclique, et
m vaut 1, 2, 3, 4, 5 ou 6,
dans laquelle $R^{51}$ et $R^{52}$, $R^{52}$ et $R^{53}$, $R^{53}$ et $R^{54}$, $R^{55}$ et $R^{56}$, $R^{56}$ et $R^{57}$, ou $R^{57}$ et $R^{58}$ forment éventuellement un cycle hydrocarboné aliphatique, un cycle hydrocarboné aromatique ou un hétérocycle, comprenant chacun des atomes de carbone liés, et
dans laquelle $Q^1$, $Q^2$ et $Q^3$ représentent indépendamment un atome d'hydrogène, un groupe alkyle substitué ou non substitué comportant 1 à 20 atomes de carbone, un groupe hydrocarboné aromatique substitué ou non substitué, ou un groupe hétérocyclique substitué ou non substitué.

**13.** Dispositif électroluminescent organique selon l'une quelconque des revendications 10 à 12, ledit matériau hôte comprenant un composé comportant une structure cyclique triazine ;

de préférence ledit composé comportant la structure cyclique triazine comportant une structure représentée par la formule (6) :

Formule (6)

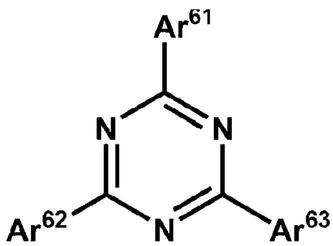

dans lequel, dans la Formule (6),
$Ar^{61}$ à $Ar^{63}$ représentent chacun indépendamment un groupe hydrocarboné aromatique substitué ou non substitué ou un groupe hétérocyclique substitué ou non substitué.

**14.** Dispositif électroluminescent organique selon l'une quelconque des revendications 10 à 13, ledit matériau hôte comprenant un composé comportant une structure cyclique anthracène.

FIG. 1

(a)

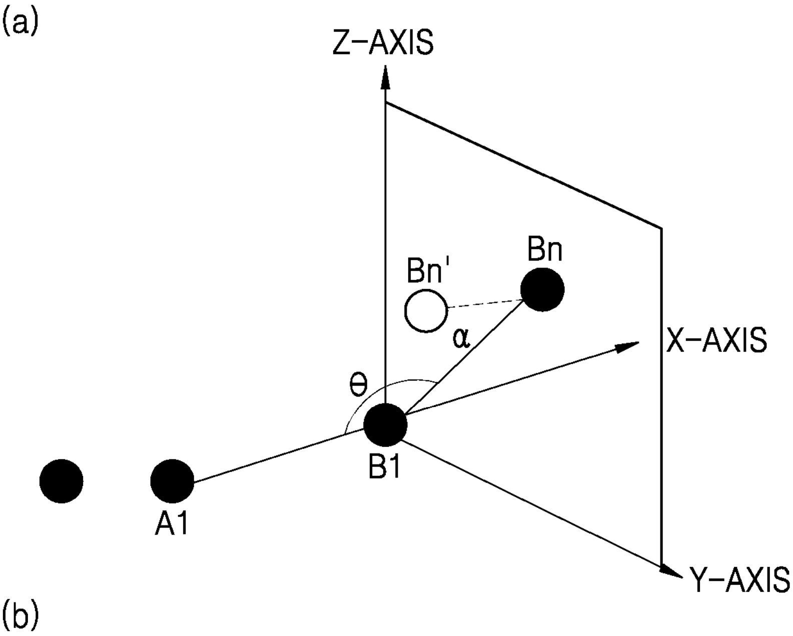


(b)

VIEW FROM X-AXIS

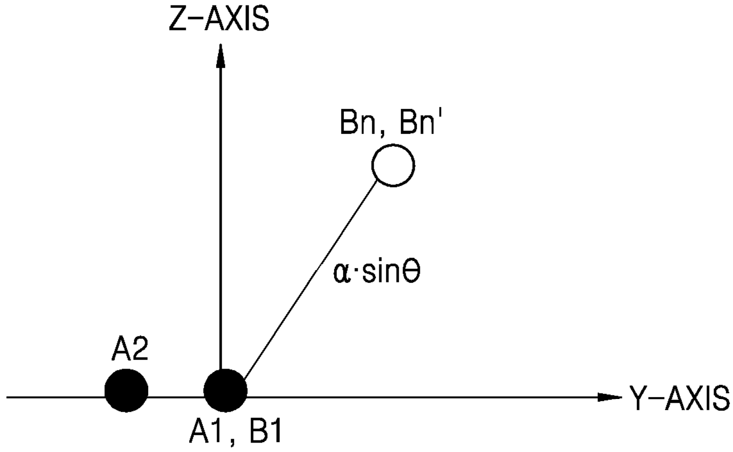


(c)

VIEW FROM X-AXIS

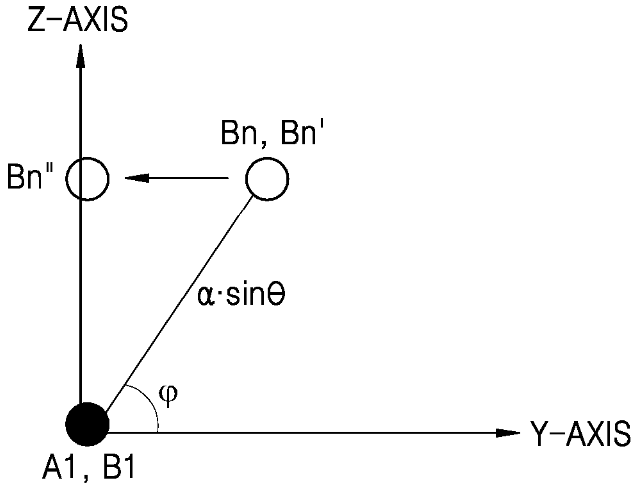

FIG. 2

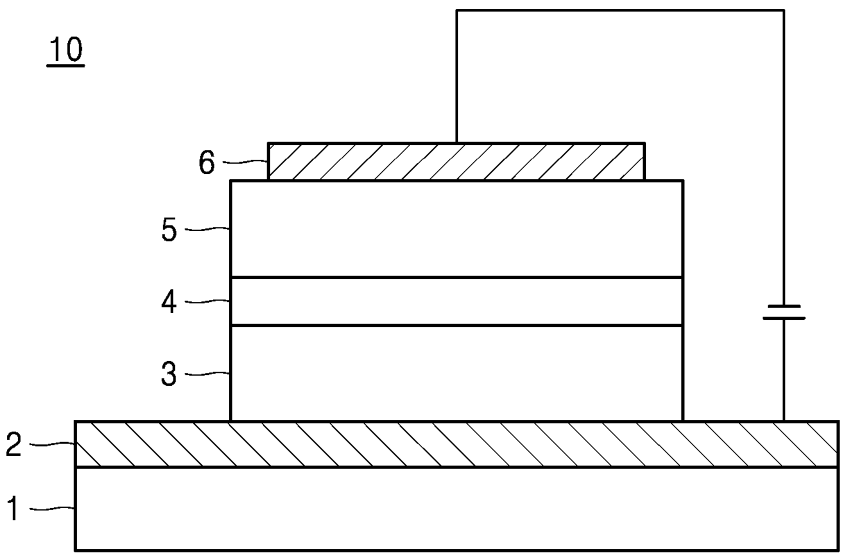

FIG. 3

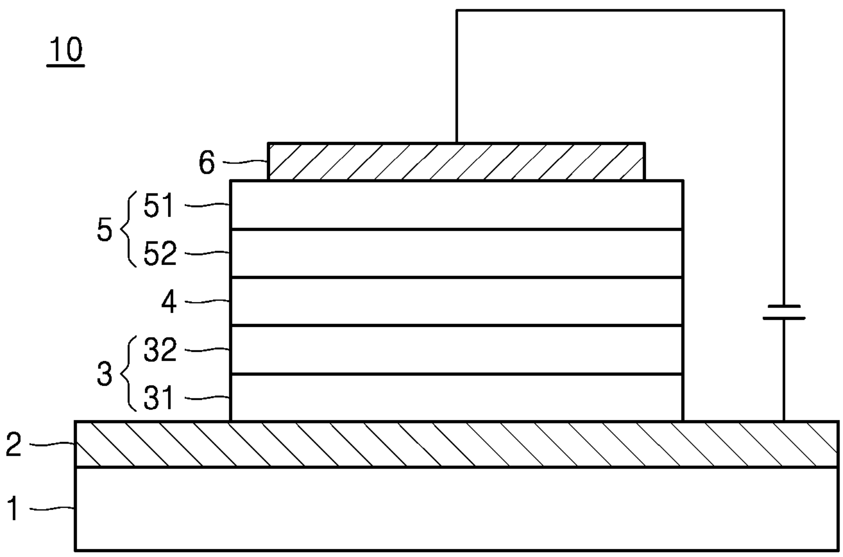

FIG. 4

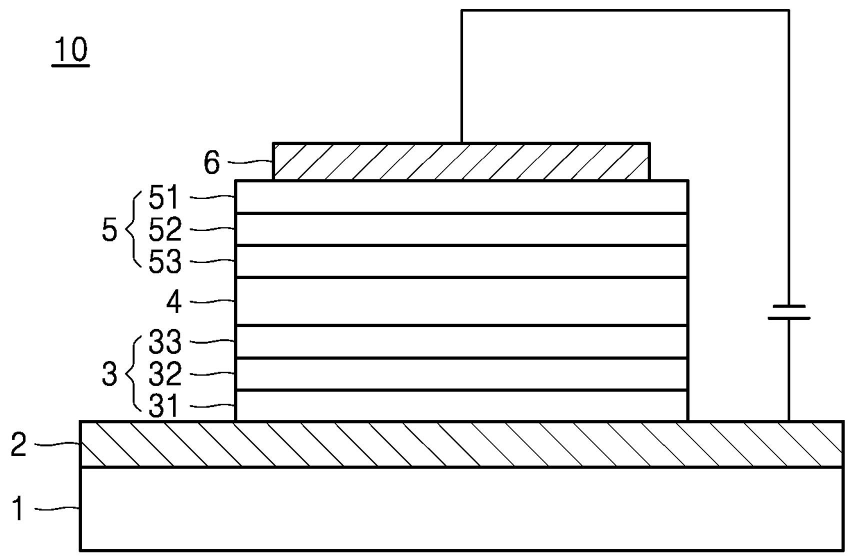

FIG. 5

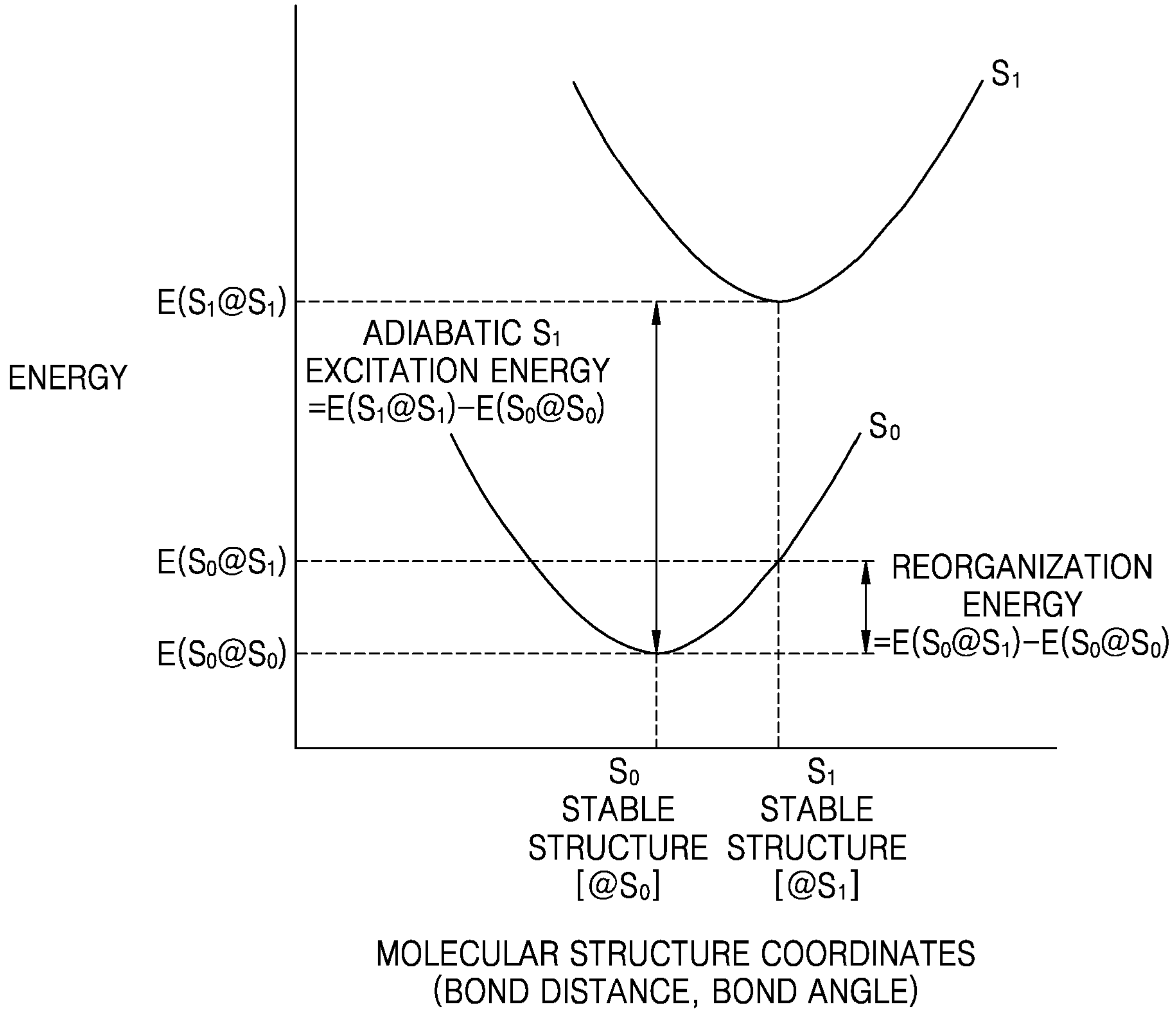
S1
E(S1@S1)
ADIABATIC S1
EXCITATION ENERGY
=E(S1@S1)−E(S0@S0)
ENERGY
S0
E(S0@S1)
REORGANIZATION
ENERGY
=E(S0@S1)−E(S0@S0)
E(S0@S0)
S0
STABLE
STRUCTURE
[@S0]
S1
STABLE
STRUCTURE
[@S1]
MOLECULAR STRUCTURE COORDINATES
(BOND DISTANCE, BOND ANGLE)

FIG. 6

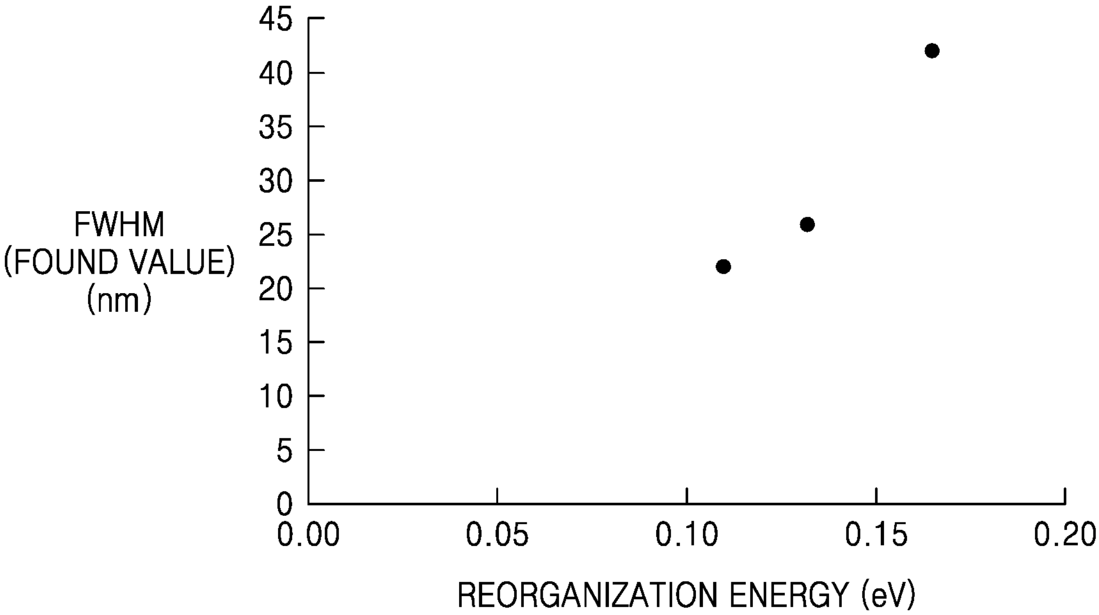
FWHM
(FOUND VALUE)
(nm)
45
40
35
30
25
20
15
10
5
0
0.00
0.05
0.10
0.15
0.20
REORGANIZATION ENERGY (eV)

FIG. 7

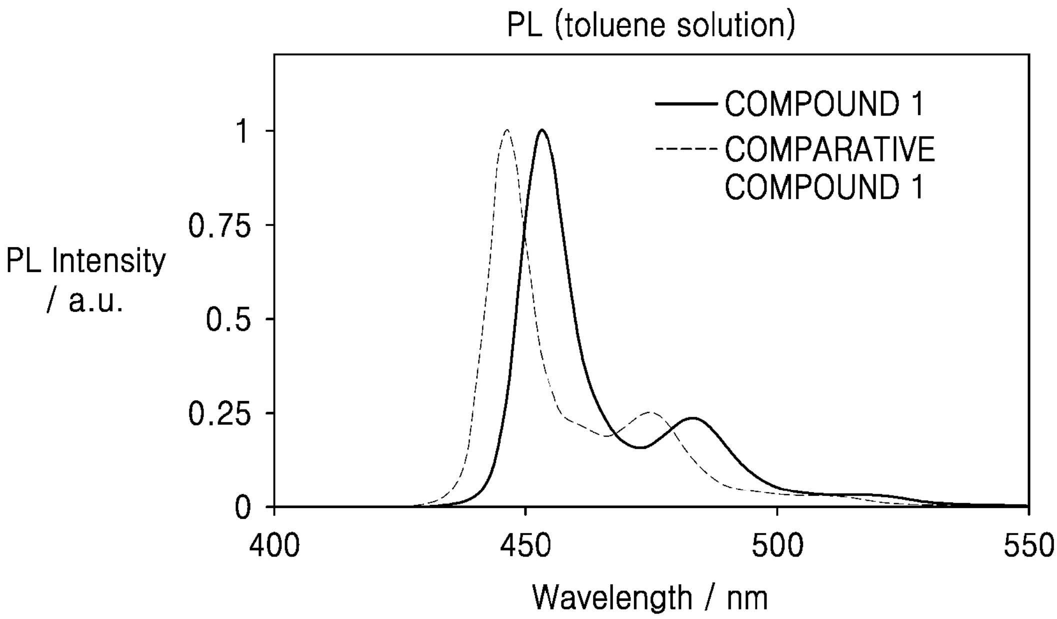
PL (toluene solution)
COMPOUND 1
COMPARATIVE COMPOUND 1
PL Intensity / a.u.
1
0.75
0.5
0.25
0
400
450
500
550
Wavelength / nm

FIG. 8

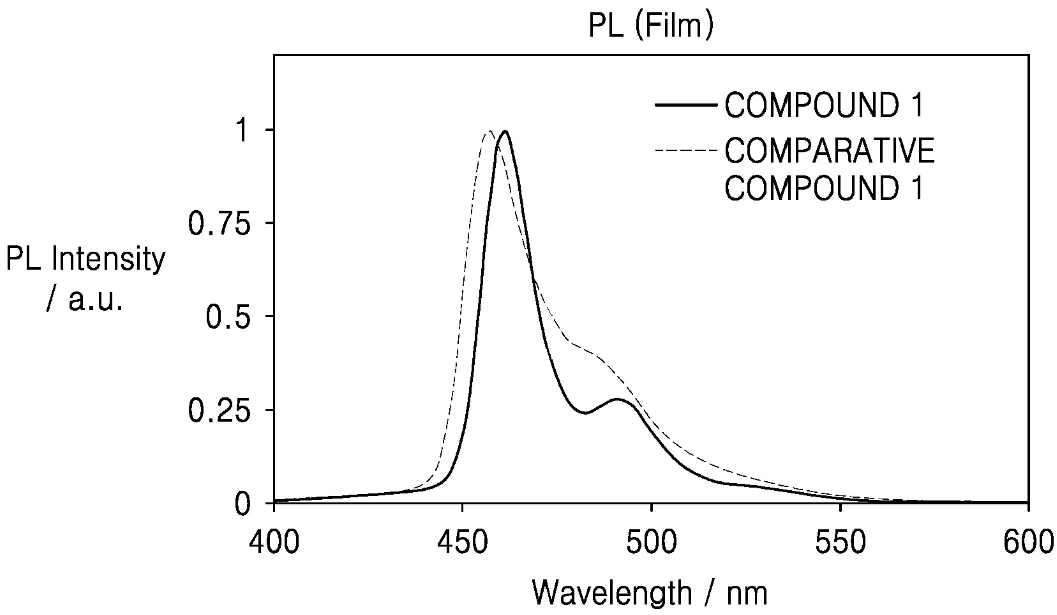
PL (Film)
COMPOUND 1
COMPARATIVE COMPOUND 1
PL Intensity / a.u.
1
0.75
0.5
0.25
0
400
450
500
550
600
Wavelength / nm

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2023008865 A
- WO 2013084805 A
- JP 2020107742 A
- JP 2022103061 A
- EP 3670520 A
- JP 2019029500 A
- US 20150162552 A


### Non-patent literature cited in the description


- **HAJIME NAKANOTANI et al.** High-efficiency organic light-emitting diodes with fluorescent emitters. *Nature Communications*, 2014, vol. 5, 4016
- **MORGANE RIVOAL et al.** Substituted dibenzo [2,3:5,6]-pyrrolizino[1,7-bc]indolo[1,2,3-lm]carbazoles: a series of new electron donors. *Tetrahedron*, 2013, vol. 69, 3302-3307
- **CLAUDE NIEBEL et al.** Dibenzo[2,3:5,6]pyrrolizino [1,7-bc]indolo[1,2,3-lm]carbazole: a new electron donor. *New Journal of Chemistry*, 2010, vol. 34, 1243-1246
- **TYLER FLEETHAM et al.** Efficient ''Pure'' Blue OLEDs Employing Tetradentate Pt Complexes with a Narrow Spectral Bandwidth. *Advanced Materials*, 2014, vol. 26, 7116-7121
- **SEOB PARK** ; **KYOHEI MATSUO** ; **NAOYA AIZAWA** ; **TAKUMA YASUDA**. High-Performance Dibenzoheteraborin-Based Thermally Activated Delayed Fluorescence Emitters: Molecular Architectonics for Concurrently Achieving Narrowband Emission and Efficient Triplet-Singlet Spin Conversion. *Advanced Functional Materials*, 2018, vol. 28, 1802031